# EUROPEAN PATENT SPECIFICATION

(11) **EP 4 603 488 B1**
(45) Date of publication and mention of the grant of the patent: **09.12.2026**
(21) Application number: 23877703.1
(22) Date of filing: 12.10.2023
(51) Int. Cl.: C07D 401/06, C07D 401/14, C07D 405/12, C07D 405/14, A61P 35/00, A61K 31/416, A61K 31/454, A61K 31/496, A61K 31/5377, A61K 31/337, A61K 45/06

(54) **INDAZOLE DERIVATIVES AS HSP90 INHIBITORS FOR THE TREATMENT OF CANCER**
INDAZOLDERIVATE ALS HSP90-INHIBITOREN ZUR BEHANDLUNG VON KREBS
DÉRIVÉS D'INDAZOLE EN TANT QU'INHIBITEURS DE HSP90 POUR LE TRAITEMENT DU CANCER

(30) Priority: 12.10.2022 KR 20220130925
(43) Date of publication of application: 20.08.2025
(73) Proprietor: Korea University Research and Business Foundation, Seoul 02841 (KR); Seoul National University R&DB Foundation, Seoul 08826 (KR)
(72) Inventor: SEO, Jae Hong, Gwacheon-si Gyeonggi-do 13803 (KR); KIM, Ji Young, Seoul 08226 (KR); KIM, Yoon Jae, Paju-si Gyeonggi-do 10887 (KR); PARK, Minsu, Seoul 08298 (KR); SEO, Juyeon, Seoul 08234 (KR); KO, Dongmi, Seoul 08302 (KR); KIM, Seongjae, Seoul 08307 (KR); PARK, Soeun, Seoul 08301 (KR); LEE, Jeewoo, Seoul 08826 (KR); ANN, Ji Hyae, Seoul 08826 (KR); HOANG, Van-Hai, Seoul 08826 (KR); LA, Minh Thanh, Seoul 08826 (KR)
(74) Representative: Weickmann & Weickmann PartmbB
(86) International application number: PCT/KR2023/015723
(87) International publication number: WO 2024/080780

(56) References cited:
- WO-A1-2008/003396
- WO-A1-2018/171575
- WO-A1-2020/040582
- KR-A- 20090 025 371
- KR-A- 20120 109 667
- KR-A- 20190 141 133
- NGUYEN CONG-TRUONG ET AL: "Discovery of novel anti-breast cancer agents derived from deguelin as inhibitors of heat shock protein 90 (HSP90)", BIOORGANIC & MEDICINAL CHEMISTRY LETTERS, ELSEVIER, AMSTERDAM NL, vol. 30, no. 17, 2 July 2020 (2020-07-02), XP086232483, ISSN: 0960-894X, DOI: 10.1016/J.BMCL.2020.127374
- HO SHIN KIM, VAN-HAI HOANG, MANNKYU HONG, KYUNG CHUL KIM, JIHYAE ANN, CONG-TRUONG NGUYEN, JI HAE SEO, HOON CHOI, JUN YONG KIM, KYU: "Investigation of B,C-ring truncated deguelin derivatives as heat shock protein 90 (HSP90) inhibitors for use as anti-breast cancer agents", BIOORGANIC & MEDICINAL CHEMISTRY, ELSEVIER, AMSTERDAM, NL, vol. 27, no. 7, 1 April 2019 (2019-04-01), AMSTERDAM, NL, pages 1370 - 1381, XP055687066, ISSN: 0968-0896, DOI: 10.1016/j.bmc.2019.02.040
- NGUYEN CONG-TRUONG; ANN JIHYAE; SAHU RAGHABA; BYUN WOONG SUB; LEE SANGKOOK; NAM GIBEOM; PARK HYUN-JU; PARK SOEUN; KIM YOON-JAE; KI: "Discovery of novel anti-breast cancer agents derived from deguelin as inhibitors of heat shock protein 90 (HSP90)", BIOORGANIC & MEDICINAL CHEMISTRY LETTERS, ELSEVIER, AMSTERDAM NL, vol. 30, no. 17, 2 July 2020 (2020-07-02), Amsterdam NL , XP086232483, ISSN: 0960-894X, DOI: 10.1016/j.bmcl.2020.127374

## Description

### TECHNICAL FIELD

The present disclosure relates to a novel indazole derivative or a pharmaceutically acceptable salt thereof, a method for preparing the same, a composition for use in a method for preventing, treating, or improving cancer including the same as an effective ingredient.

### BACKGROUND ART

The cases of breast cancer have continued to increase domestically and internationally since the 1990s, and in the 2000s, breast cancer ranked first and second among all cancers in women, showing high incidence and mortality rates. HER2-positive breast cancer is a cancer in which HER2 (human epidermal growth factor receptor 2), a membrane protein receptor present in the cancer cell membrane, is overexpressed. It accounts for 20-30% of all breast cancer patients, and has a worse prognosis than HER2-negative patients, with a significantly lower survival rate and very low responsiveness to commonly used cytotoxic anticancer drugs.

Monoclonal antibodies trastuzumab (trade name: Herceptin^{®}), pertuzumab (trade name: Perjeta ^{®}), and T-DM1 (trade name: Kadcyla ^{®}) have been developed as targeted therapies for HER2-positive breast cancer, but they are expensive and impose a significant financial burden on patients. In particular, trastuzumab is mainly used as a first-line treatment for patients with HER2-positive breast cancer, but approximately 40-50% of patients develop primary resistance in the initial treatment, and even among patients with a good initial response, approximately 90% develop secondary resistance within one year. This resistance is a major cause of recurrence and metastasis and plays a critical role in decreasing the patient's survival rate.

On the other hand, patients with triple-negative breast cancer (TNBC; ER-, PR-, HER2-), who account for 10-15% of all breast cancer patients, lack hormone receptors (ER (estrogen receptors), PR (progesterone receptors)) and HER2 proteins, and thus they do not benefit from hormone therapy or HER2-targeted therapy. Currently, the standard of care for triple-negative breast cancer relies entirely on general cytotoxic anti-cancer drugs (Taxane- or anthracene-based agents). Since there are no established targeted therapeutic agents, treatment strategies for other subtypes are less diverse than those for breast cancer. More seriously, after surgery or anticancer treatment, recurrence occurs within 2-3 years in most patients, and metastasis to other organs such as the lungs, liver, brain and bones is easily triggered, affecting the survival rate of the patients.

The 5-year overall survival rate for patients diagnosed with stage III cancer is less than 55%, and for patients whose cancer has already metastasized (advanced-stage), the 5-year overall survival rate is even lower, at 30% or less. This is a very serious disease that will ultimately kill most of these patients within a few years.

Hsp90 inhibitors are potential chemotherapeutic agents for angiogenesis-related diseases, especially for HER2, one of the major client proteins of Hsp90, whose kinase activity is directly regulated by Hsp90. Therefore, inhibition of Hsp90 may directly suppress the activation of p95HER2 and HER2/HER3 dimerization, which are ultimately the main causes of resistance, hence Hsp90 inhibitors may be a top targeted therapeutic agent for HER2. Therefore, Hsp90 inhibitors have high value as drugs to overcome drug resistance.

However, most of the Hsp90 inhibitors in clinical trials to date inhibit the N-terminal of HSP90, and they have failed or have been discontinued in clinical trials due to serious side effects such as lack of pharmacological activity, toxicity, and induction of heat shock response.

The paper Nguyen Cong-Truong et al., Bioorganic & Medicinal Chemistry Letters, Elsevier, Amsterdam NL, vol. 30, no. 17, 2 July 2020 discloses anti-breast cancer agents derived from deguelin as inhibitors of heat shock protein 90 (HSP90).

WO 2008/003396 A1 relates to indazole derivatives of formula 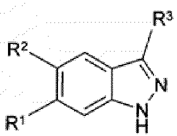 which are inhibitors of HSP90 and are used to produce a medicament for treating diseases, in which the inhibition, regulation and/or modulation of HSP90 plays a role.
WO 2018/171575 discloses substituted aromatic compounds that effectively inhibit HSP90 with good safety profiles.

Accordingly, the present inventors have found that a novel indazole derivative of the present disclosure or a pharmaceutically acceptable salt thereof imparts strong binding affinity to the C-terminal of Hsp90, thereby inhibiting the activity of Hsp90, inhibiting the growth and proliferation of cancer cells, and inducing effective cell death, which lead them to create the present disclosure.

### DISCLOSURE OF THE INVENTION

### TECHNICAL GOALS

A technical goal to be achieved by the present disclosure is to provide an indazole derivative or a pharmaceutically acceptable salt thereof.

Another goal of the present disclosure is to provide a composition for use in a method for preventing, treating, or improving cancer, including the indazole derivative or a pharmaceutically acceptable salt thereof as an active ingredient.

Another goal of the present disclosure is to provide a method for preparing the indazole derivative or a pharmaceutically acceptable salt thereof.

References in the present description to methods of medical treatment or medical uses have to be understood as references to the compounds of the present invention for use in methods of medical treatment. Methods of medical treatment or medical uses are not encompassed by the claimed invention.

### TECHNICAL SOLUTIONS

In order to achieve the above mentioned goals, the present disclosure may provide an indazole derivative represented by the following [Formula 1], or a pharmaceutically acceptable salt thereof. 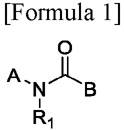 wherein, in Formula 1,
A is 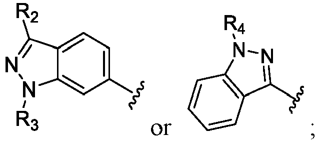
B is 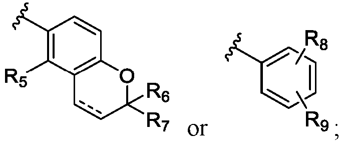
R₁ and R₂ are identical or different from each other, and each represents hydrogen or a C₁-C₆ chained alkyl group;
R₃ and R₄ are identical or different from each other, and each represents hydrogen or a C₁-C₆ chained alkyl group, wherein at least one hydrogen of the alkyl group is unsubstituted or substituted with a C₁-C₆ alkoxy group, a C₅-C₁₀ heterocycloalkyl group, or a C₅-C₁₀ heteroaryl group, wherein the alkoxy group is unsubstituted or substituted with a C₁-C₆ alkoxy group, and the heterocycloalkyl group is unsubstituted or substituted with a C₁-C₆ chained alkyl group, 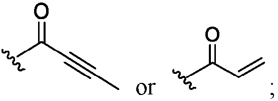
R₅ is a halogen group or a C₁-C₆ alkoxy group;
R₆ and R₇ are identical or different from each other, and each represents a C₁-C₆ chained alkyl group;
R₈ is NR₁₀C(O)R₁₁, OR₁₂, a C₅-C₁₀ heteroaryl group, or a tetrahydropyranyl amine group;
R₉ is hydrogen, a C₁-C₆ alkoxy group, or a C₅-C₁₀ heterocycloalkyl group, wherein the heterocycloalkyl group is unsubstituted or substituted with a C₁-C₆ chained alkyl group;
R₁₀ and R₁₁ are identical or different from each other, and each represents a C₁-C₆ chained alkyl group or C₅-C₁₀ aryl group, wherein the alkyl group is unsubstituted or substituted with a methylpiperazinyl group;
R₁₂ is a C₅-C₁₀ aryl group or a C₅-C₁₀ benzyl group, wherein the benzyl group is unsubstituted or substituted with a halogen group;
- - - may be a single bond or a double bond.

In an embodiment of the present disclosure, the heterocycloalkyl group may be a morpholino group, a piperidinyl group, a piperazinyl group, a pyrrolidinyl group, etc.. The heteroaryl group may be a pyridinyl group, a pyrimidinyl group, a pyrazinyl group, a furyl group, a thiophenyl group, an oxazolyl group, a benzimidazolyl group, an indolyl group, etc., preferably a pyridinyl group.

In an embodiment of the present disclosure, R₃ and R₄ are identical or different from each other, and may be any one selected from the group consisting of hydrogen, a methyl group, 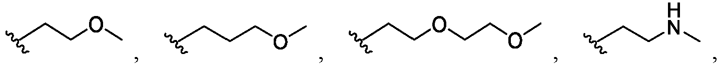 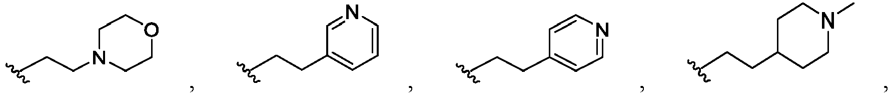 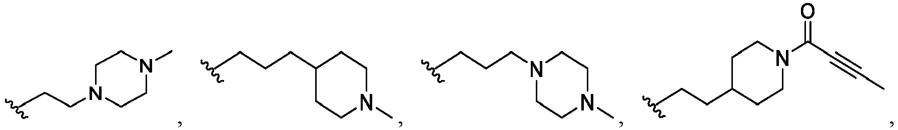 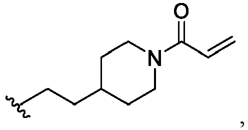 and combinations thereof.

In another embodiment of the present disclosure, the indazole derivative represented by the above-mentioned [Formula 1] may be any one or more selected from the group consisting of the compounds represented by the following formulas.
(1) 5-Methoxy-2,2-dimethyl-N-(3-methyl-1H-indazol-6-yl)-2H-chromene-6-carboxamide (formula 1-1, 6a);
(2) N-(1,3-dimethyl-1H-indazol-6-yl)-5-methoxy-2,2-dimethyl-2H-chromene-6-carboxamide (formula 1-2, 6b);
(3) 5-Methoxy-N-(1-(2-methoxyethyl)-3-methyl-1H-indazol-6-yl)-2,2-dimethyl-2H-chromene-6-carboxamide (formula 1-3, 6c);
(4) 5-Methoxy-2,2-dimethyl-N-(3-methyl-1-(2-morpholinoethyl)-1H-indazol-6-yl)-2H-chromene-6-carboxamide (formula 1-4, 6d);
(5) 5-Methoxy-2,2-dimethyl-N-(3-methyl-1-(2-(pyridin-3-yl)ethyl)-1H-indazol-6-yl)-2H-chromene-6-carboxamide (formula 1-5, 6e);
(6) 5-Methoxy-2,2-dimethyl-N-(3-methyl-1-(2-(pyridin-4-yl)ethyl)-1H-indazol-6-yl)-2H-chromene-6-carboxamide (formula 1-6, 6f);
(7) 5-Methoxy-2,2-dimethyl-N-(3-methyl-1-(2-(1-methylpiperidin-4-yl)ethyl)-1H-indazol-6-yl)-2H-chromene-6-carboxamide (formula 1-7, 11k);
(8) 5-Methoxy-2,2-dimethyl-N-(3-methyl-1-(2-(4-methylpiperazin-1-yl)ethyl)-1H-indazol-6-yl)-2H-chromene-6-carboxamide (formula 1-8, 11l);
(9) 5-methoxy-2,2-dimethyl-N-(3-methyl-1-(3-(1-methylpiperidin-4-yl)propyl)-1H-isoindol-6-yl)-2H-chromene-6-carboxamide (formula 1-9, 11m);
(10) 5-methoxy-2,2-dimethyl-N-(3-methyl-1-(3-(4-methylpiperazin-1-yl)propyl)-1H-indazol-6-yl)-2H-chromene-6-carboxamide (formula 1-10, 11n);
(11) N-(1-(2-(1-(but-2-ynoyl)piperidin-4-yl)ethyl)-3-methyl-1H-indazol-6-yl)-5-methoxy-2,2-dimethyl-2H-chromene-6-carboxamide (formula 1-11, 11o);
(12) N-(1-(2-(1-acryloylpiperidin-4-yl)ethyl)-3-methyl-1H-indazol-6-yl)-5-methoxy-2,2-dimethyl-2H-chromene-6-carboxamide (formula 1-12, 11p);
(13) 5-methoxy-2,2-dimethyl-N-(1-(2-(1-methylpiperidin-4-yl)ethyl)-1H-indazol-6-yl)-2H-chromene-6-carboxamide (formula 1-13, 12k);
(14) N-(1H-indazol-3-yl)-5-methoxy-2,2-dimethyl-2H-chromene-6-carboxamide (formula 1-14, 18a);
(15) 5-Methoxy-N,2,2-trimethyl-N-(1-methyl-1H-indazol-3-yl)-2H-chromene-6-carboxamide (formula 1-15, 18b);
(16) 5-Methoxy-N-(1-(2-methoxyethyl)-1H-indazol-3-yl)-2,2-dimethyl-2H-chromene-6-carboxamide (formula 1-16, 18c);
(17) 5-Methoxy-N-(1-(3-methoxypropyl)-1H-indazol-3-yl)-2,2-dimethyl-2H-chromene-6-carboxamide (formula 1-17, 18d);
(18) 5-Methoxy-N-(1-(2-(2-methoxyethoxy)ethyl)-1H-indazol-3-yl)-2,2-dimethyl-2H-chromene-6-carboxamide (formula 1-18, 18e);
(19) 5-Methoxy-2,2-dimethyl-N-(1-(2-(methylamino)ethyl)-1H-indazol-3-yl)-2H-chromene-6-carboxamide (formula 1-19, 19l);
(20) 5-Methoxy-2,2-dimethyl-N-(1-(2-(1-methylpiperidin-4-yl)ethyl)-1H-indazol-3-yl)-2H-chromene-6-carboxamide (formula 1-20, 18g);
(21) 5-Methoxy-2,2-dimethyl-N-(1-(2-(4-methylpiperazin-1-yl)ethyl)-1H-indazol-3-yl)-2H-chromene-6-carboxamide (formula 1-21, 18h);
(22) 5-Methoxy-2,2-dimethyl-N-(1-(2-morpholinoethyl)-1H-indazol-3-yl)-2H-chromene-6-carboxamide (formula 1-22, 18i);
(23) 5-Methoxy-2,2-dimethyl-N-(1-(2-(pyridin-3-yl)ethyl)-1H-indazol-3-yl)-2H-chromene-6-carboxamide (formula 1-23, 18j);
(24) 5-Methoxy-2,2-dimethyl-N-(1-(2-(pyridin-4-yl)ethyl)-1H-indazol-3-yl)-2H-chromene-6-carboxamide (formula 1-24, 18k);
(25) 5-Methoxy-N-(1-(2-methoxyethyl)-1H-indazol-3-yl)-N,2,2-trimethyl-2H-chromene-6-carboxamide (formula 1-25, 20c);
(26) 5-methoxy-2,2-dimethyl-N-(3-methyl-1-(2-(1-methylpiperidin-4-yl)ethyl)-1H-indazol-6-yl)chroman-6-carboxamide (formula 1-26, 34);
(27) 5-ethoxy-2,2-dimethyl-N-(3-methyl-1-(2-(1-methylpiperidin-4-yl)ethyl)-1H-indazol-6-yl)-2H-chromene-6-carboxamide (formula 1-27, 35);
(28) 2,2-diethyl-5-methoxy-N-(3-methyl-1-(2-(1-methylpiperidin-4-yl)ethyl)-1H-indazol-6-yl)-2H-chromene-6-carboxamide (formula 1-28, 36);
(29) 5-fluoro-2,2-dimethyl-N-(3-methyl-1-(2-(1-methylpiperidin-4-yl)ethyl)-1H-indazol-6-yl)-2H-chromene-6-carboxamide (formula 1-29, 37);
(30) 5-chloro-2,2-dimethyl-N-(3-methyl-1-(2-(1-methylpiperidin-4-yl)ethyl)-1H-indazol-6-yl)-2H-chromene-6-carboxamide (formula 1-30, 38);
(31) N-methyl-N-(4-((3-methyl-1-(2-(1-methylpiperidin-4-yl)ethyl)-1H-indazol-6-yl)carbamoyl)phenyl)benzamide (formula 1-31, 42a);
(32) N-(3-methyl-1-(2-(1-methylpiperidin-4-yl)ethyl)-1H-indazol-6-yl)-4-(N-methyl-2-(4-methylpiperazin-1-yl)acetamido)benzamide (formula 1-32, 42b);
(33) N-(3-methyl-1-(2-(1-methylpiperidin-4-yl)ethyl)-1H-indazol-6-yl)-4-(4-methylpiperazin-1-yl)-2-((tetrahydro-2H-pyran-4-yl)amino)benzamide (formula 1-33, 46);
(34) N-(3-methyl-1-(2-(1-methylpiperidin-4-yl)ethyl)-1H-indazol-6-yl)-3-(pyridin-4-yl)benzamide (formula 1-34, 50a);
(35) N-(3-methyl-1-(2-(1-methylpiperidin-4-yl)ethyl)-1H-indazol-6-yl)-3-(pyridin-3-yl)benzamide (formula 1-35, 50b);
(36) N-(3-methyl-1-(2-(1-methylpiperidin-4-yl)ethyl)-1H-indazol-6-yl)-4-(pyridin-4-yl)benzamide (formula 1-36, 50c);
(37) N-(3-methyl-1-(2-(1-methylpiperidin-4-yl)ethyl)-1H-indazol-6-yl)-4-(pyridin-3-yl)benzamide (formula 1-37, 50d);
(38) 2-methoxy-N-(3-methyl-1-(2-(1-methylpiperidin-4-yl)ethyl)-1H-indazol-6-yl)-3-(pyridin-4-yl)benzamide (formula 1-38, 50e);
(39) 2-methoxy-N-(3-methyl-1-(2-(1-methylpiperidin-4-yl)ethyl)-1H-indazol-6-yl)-3-(pyridin-3-yl)benzamide (formula 1-39, 50f);
(40) 2-methoxy-N-(3-methyl-1-(2-(1-methylpiperidin-4-yl)ethyl)-1H-indazol-6-yl)-4-(pyridin-4-yl)benzamide (formula 1-40, 50g);
(41) 2-methoxy-N-(3-methyl-1-(2-(1-methylpiperidin-4-yl)ethyl)-1H-indazol-6-yl)-4-(pyridin-3-yl)benzamide (formula 1-41, 50h);
(42) N-(3-methyl-1-(2-(1-methylpiperidin-4-yl)ethyl)-1H-indazol-6-yl)-4-phenoxybenzamide (formula 1-42, 57a);
(43) 4-(benzyloxy)-N-(3-methyl-1-(2-(1-methylpiperidin-4-yl)ethyl)-1H-indazol-6-yl)benzamide (formula 1-43, 57b);
(44) 4-((4-fluorobenzyl)oxy)-N-(3-methyl-1-(2-(1-methylpiperidin-4-yl)ethyl)-1H-indazol-6-yl)benzamide (formula 1-44, 57c);
(45) 4-((3-fluorobenzyl)oxy)-N-(3-methyl-1-(2-(1-methylpiperidin-4-yl)ethyl)-1H-indazol-6-yl)benzamide (formula 1-45, 57d);
(46) 4-((4-chlorobenzyl)oxy)-N-(3-methyl-1-(2-(1-methylpiperidin-4-yl)ethyl)-1H-indazol-6-yl)benzamide (formula 1-46, 57e);
(47) 4-((3-chlorobenzyl)oxy)-N-(3-methyl-1-(2-(1-methylpiperidin-4-yl)ethyl)-1H-indazol-6-yl)benzamide (formula 1-47, 57f);
(48) 2-methoxy-N-(3-methyl-1-(2-(1-methylpiperidin-4-yl)ethyl)-1H-indazol-6-yl)-4-phenoxybenzamide (formula 1-48, 57g);
(49) 4-(benzyloxy)-2-methoxy-N-(3-methyl-1-(2-(1-methylpiperidin-4-yl)ethyl)-1H-indazol-6-yl)benzamide (formula 1-49, 57h);
(50) 4-((3-fluorobenzyl)oxy)-2-methoxy-N-(3-methyl-1-(2-(1-methylpiperidin-4-yl)ethyl)-1H-indazol-6-yl)benzamide (formula 1-50, 57i);
(51) 4-((4-fluorobenzyl)oxy)-2-methoxy-N-(3-methyl-1-(2-(1-methylpiperidin-4-yl)ethyl)-1H-indazol-6-yl)benzamide (formula 1-51, 57j);
(52) 4-((3-Chlorobenzyl)oxy)-2-methoxy-N-(3-methyl-1-(2-(1-methylpiperidin-4-yl)ethyl)-1H-indazol-6-yl)benzamide (formula 1-52, 57k); and
(53) 4-((4-Chlorobenzyl)oxy)-2-methoxy-N-(3-methyl-1-(2-(1-methylpiperidin-4-yl)ethyl)-1H-indazol-6-yl)benzamide(1-53, 57l).

In another embodiment of the present disclosure, the pharmaceutically acceptable salt of the indazole derivative may be any one or more selected from the group consisting of hydrochloride salt, bromate, sulfate, phosphate, nitrate, citrate, acetate, lactate, tartrate, maleate, gluconate, succinate, formate, trifluoroacetate, oxalate, fumarate, glutarate, adipate, methanesulfonate, benzenesulfonate, paratoluenesulfonate, camphorsulfonate, sodium salt, potassium salt, lithium salt, calcium salt, magnesium salt, and combinations thereof.

In addition, the present disclosure provides a pharmaceutical composition for preventing or treating cancer, including the indazole derivative or a pharmaceutically acceptable salt thereof as an active ingredient.

In an embodiment of the present disclosure, the indazole derivative may act as an Hsp90 inhibitor to suppress the growth and proliferation of cancer cells and to induce cell death (apoptosis).

In another embodiment of the present disclosure, the cancer may be any one or more selected from the group consisting of skin cancer, breast cancer, uterine cancer, esophageal cancer, stomach cancer, a brain tumor, colon cancer, rectal cancer, colorectal cancer, lung cancer, ovarian cancer, cervical cancer, endometrial cancer, vulvar cancer, kidney cancer, blood cancer, pancreatic cancer, prostate cancer, testicular cancer, laryngeal cancer, head and neck cancer, thyroid cancer, liver cancer, bladder cancer, osteosarcoma, lymphoma, leukemia, thymic cancer, urethral cancer, bronchial cancer, and combinations thereof, preferably breast cancer, more preferably HER-2 positive breast cancer or triple-negative breast cancer.

In another embodiment of the present disclosure, the pharmaceutical composition may further include any one or more additional component selected from the group consisting of a pharmaceutically acceptable carrier, excipient, diluent, stabilizer, and preservative, in addition to the indazole derivative or a pharmaceutically acceptable salt thereof..

In another embodiment of the present disclosure, the pharmaceutical composition may further include an anticancer agent, in addition to the indazole derivative or a pharmaceutically acceptable salt thereof.

In another embodiment of the present disclosure, the anticancer agent may be paclitaxel, docetaxel, doxorubicin, sorafenib, vemurafenib, irinotecan, cisplatin, alpharadin, mitoxantrone, cyclophosphamide, vinblastine, carboplatin, actinomycin-D, etoposide, teniposide, bisantrene, homoharringtonine, Gleevec (STI-571), 5-fluorouracil, busulfan, chlorambucil, melphalan, nitrogen mustard, nitrosoureas, and combinations thereof, and the like.

In another embodiment of the present disclosure, the pharmaceutical composition may have any one or more dosage form selected from the group consisting of powders, granules, tablets, capsules, and injections.

In addition, the present disclosure provides the indazole derivative or a pharmaceutically acceptable salt thereof for use in a method for preventing or treating cancer.

In addition, the present disclosure provides a cosmetic composition for preventing or ameliorating cancer, including the indazole derivative or a cosmetically acceptable salt thereof as an active ingredient.

Furthermore, the present disclosure provides a food composition for preventing or ameliorating cancer, including the indazole derivative or a food-chemically acceptable salt thereof as an active ingredient.

In addition, the present disclosure provides a feed composition for preventing or treating cancer, including the indazole derivative or a feed-chemically acceptable salt thereof as an active ingredient.

The present disclosure also provides a method for producing an indazole derivative represented by [Formula 1] or a pharmaceutically acceptable salt thereof, including reacting a compound represented by [Formula 2] or [Formula 3] with a compound represented by [Formula 4] or [Formula 5]. 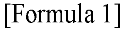 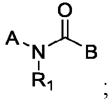 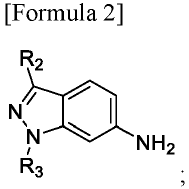 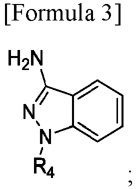 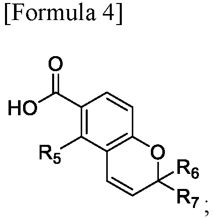 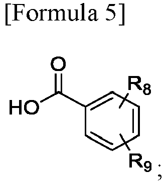 wherein, in Formula 1,
A is 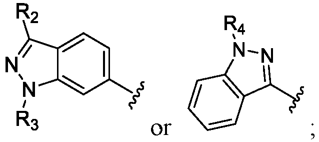
B is 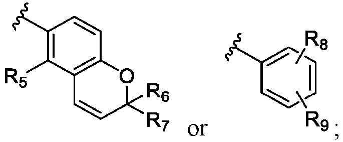
R₁ is hydrogen or a C₁-C₆ chained alkyl group;
wherein, in Formulas 1 and 2,
R₂ is hydrogen or a C₁-C₆ chained alkyl group;
R₃ is hydrogen or a C₁-C₆ chained alkyl group, wherein at least one hydrogen of the alkyl group is unsubstituted or substituted with a C₁-C₆ alkoxy group, a C₅-C₁₀ heterocycloalkyl group, or a C₅-C₁₀ heteroaryl group, wherein the alkoxy group is unsubstituted or substituted with a C₁-C₆ alkoxy group, and the heterocycloalkyl group is unsubstituted or substituted with a C₁-C₆ chained alkyl group, 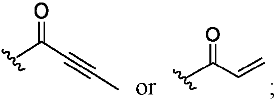
wherein, in Formulas 1 and 3,
R₄ is hydrogen or a chained C₁-C₆ alkyl group,
wherein at least one hydrogen of the alkyl group is unsubstituted or substituted with a C₁-C₆ alkoxy group, a C₅-C₁₀ heterocycloalkyl group, or a C₅-C₁₀ heteroaryl group, wherein the alkoxy group is unsubstituted or substituted with a C₁-C₆ alkoxy group, and the heterocycloalkyl group is unsubstituted or substituted with a C₁-C₆ chained alkyl group, 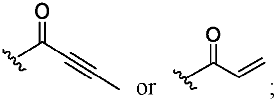
wherein, in Formulas 1 and 4,
R₅ is a halogen group or a C₁-C₆ alkoxy group;
R₆ and R₇ are identical or different from each other, and each represents a C₁-C₆ chained alkyl group;
wherein, in Formulas 1 and 5,
R₈ is NR₁₀C(O)R₁₁, OR₁₂, a C₅-C₁₀ heteroaryl group, or a tetrahydropyranyl amine group;
R₉ is hydrogen, a C₁-C₆ alkoxy group, or a C₅-C₁₀ heterocycloalkyl group, wherein the heterocycloalkyl group is unsubstituted or substituted with a C₁-C₆ chained alkyl group;
R₁₀ and R₁₁ are identical or different from each other, and each represents a C₁-C₆ chained alkyl group or C₅-C₁₀ aryl group, wherein the alkyl group is unsubstituted or substituted with a methylpiperazinyl group;
R₁₂ may be a C₅-C₁₀ aryl group or a C₅-C₁₀ benzyl group, wherein the benzyl group is unsubstituted or substituted with a halogen group.

In an embodiment of the present disclosure, the compound represented by the above-mentioned [Formula 2] may be any one or more selected from the group consisting of the compounds represented by the following formulas.
(1) 3-Methyl-1H-indazol-6-amine (formula 2-1, 5a);
(2) 1,3-Dimethyl-1H-indazol-6-amine (formula 2-2, 5b);
(3) 1-(2-Methoxyethyl)-3-methyl-1H-indazol-6-amine (formula 2-3, 5c);
(4) 3-Methyl-1-(2-morpholinoethyl)-1H-indazol-6-amine (formula 2-4, 5d);
(5) 3-Methyl-1-(2-(pyridin-3-yl)ethyl)-1H-indazol-6-amine (formula 2-5, 5e);
(6) 3-Methyl-1-(2-(pyridin-4-yl)ethyl)-1H-indazol-6-amine (formula 2-6, 5f);
(7) 3-Methyl-1-(2-(1-methylpiperidin-4-yl)ethyl)-1H-indazol-6-amine (formula 2-7, 9k);
(8) 3-Methyl-1-(2-(4-methylpiperazin-1-yl)ethyl)-1H-indazol-6-amine (formula 2-8, 9l);
(9) 3-methyl-1-(3-(1-methylpiperidin-4-yl)propyl)-1H-indazol-6-amine (formula 2-9, 9m);
(10) 3-methyl-1-(3-(4-methylpiperazin-1-yl)propyl)-1H-indazol-6-amine (formula 2-10, 9n); and
(11) 1-(2-(1-methylpiperidin-4-yl)ethyl)-1H-indazol-6-amine (formula 2-11, 10k).

In another embodiment of the present disclosure, the compound represented by the above-mentioned [Formula 3] may be any one or more selected from the group consisting of the compounds represented by the following formulas.
(1) 1-Methyl-1H-indazol-3-amine (formula 3-1, 17b);
(2) 1-(2-Methoxyethyl)-1H-indazol-3-amine (formula 3-2, 17c);
(3) 1-(3-Methoxypropyl)-1H-indazol-3-amine (formula 3-3, 17d);
(4) 1-(2-(2-Methoxyethoxy)ethyl)-1H-indazol-3-amine (formula 3-4, 17e);
(5) 1-(2-(1-methylpiperidin-4-yl)ethyl)-1H-indazol-3-amine (formula 3-5, 17g);
(6) 1-(2-(4-methylpiperazin-1-yl)ethyl)-1H-indazol-3-amine (formula 3-6, 17h);
(7) 1-(2-Morpholinoethyl)-1H-indazol-3-amine (formula 3-7, 17i);
(8) 1-(2-(Pyridin-3-yl)ethyl)-1H-indazol-3-amine (formula 3-8, 17j); and
(9) 1-(2-(Pyridin-4-yl)ethyl)-1H-indazol-3-amine (formula 3-9, 17k).

In another embodiment of the present disclosure, the compound represented by the above-mentioned [Formula 4] may be any one or more selected from the group consisting of the compounds represented by the following formulas.
(1) 5-methoxy-2,2-dimethyl-2H-chromene-6-carboxylic acid (formula 4-1, 29);
(2) 5-ethoxy-2,2-dimethyl-2H-chromene-6-carboxylic acid (formula 4-2, 30);
(3) 2,2-diethyl-5-methoxy-2H-chromene-6-carboxylic acid (formula 4-3, 31);
(4) 5-fluoro-2,2-dimethyl-2H-chromene-6-carboxylic acid (formula 4-4, 32); and
(5) 5-chloro-2,2-dimethyl-2H-chromene-6-carboxylic acid (formula 4-5, 33).

In another embodiment of the present disclosure, the compound represented by the above-mentioned [Formula 5] may be any one or more selected from the group consisting of the compounds represented by the following formulas.
(1) 4-(N-methylbenzamido)benzoic acid (formula 5-1, 41a);
(2) 4-(N-methyl-2-(4-methylpiperazin-1-yl)acetamido)benzoic acid (formula 5-2, 41b);
(3) 3-(pyridin-4-yl)benzoic acid (formula 5-3, 49a);
(4) 3-(pyridin-3-yl)benzoic acid (formula 5-4, 49b);
(5) 4-(pyridin-4-yl)benzoic acid (formula 5-5, 49c);
(6) 4-(pyridin-3-yl)benzoic acid (formula 5-6, 49d);
(7) 2-methoxy-3-(pyridin-4-yl)benzoic acid (formula 5-7, 49e);
(8) 2-methoxy-3-(pyridin-3-yl)benzoic acid (formula 5-8, 49f);
(9) 2-methoxy-4-(pyridin-4-yl)benzoic acid (formula 5-9, 49g);
(10) 2-methoxy-4-(pyridin-3-yl)benzoic acid (formula 5-10, 49h);
(11) 4-phenoxybenzoic acid (formula 5-11, 56a);
(12) 4-(benzyloxy)benzoic acid (formula 5-12, 56b);
(13) 4-((4-fluorobenzyl)oxy)benzoic acid (formula 5-13, 56c);
(14) 4-((3-fluorobenzyl)oxy)benzoic acid (formula 5-14, 56d);
(15) 4-((4-chlorobenzyl)oxy)benzoic acid (formula 5-15, 56e);
(16) 4-((3-chlorobenzyl)oxy)benzoic acid (formula 5-16, 56f);
(17) 2-methoxy-4-phenoxybenzoic acid (formula 5-17, 56g);
(18) 4-(benzyloxy)-2-methoxybenzoic acid (formula 5-11, 56h);
(19) 4-((3-fluorobenzyl)oxy)-2-methoxybenzoic acid (formula 5-11, 56i);
(20) 4-((4-fluorobenzyl)oxy)-2-methoxybenzoic acid (formula 5-11, 56j);
(21) 4-((3-Chlorobenzyl)oxy)-2-methoxybenzoic acid (formula 5-11, 56k); and
(22) 4-((4-Chlorobenzyl)oxy)-2-methoxybenzoic acid (formula 5-11, 561).

In another embodiment of the present disclosure, the compound represented by the above-mentioned [Formula 2] or [Formula 3] may be reacted by adding it to a mixture of the compound represented by the above-mentioned [Formula 4] or [Formula 5], hydroxybenzotriazole (HOBt), triethylamine (Et₃N), and EDC·HCl. In this case, the solvent may be dichloromethane (DCM).

In another embodiment of the present disclosure, the steps of extracting the organic compound after the reaction, washing, drying, and concentrating it in vacuo may be further included.

### EFFECTS OF THE INVENTION

The present disclosure relates to an indazole derivative or a pharmaceutically acceptable salt thereof, and a composition for preventing, treating, or ameliorating cancer, including, as an active ingredient, the derivative or a pharmaceutically acceptable salt thereof. The indazole derivative of the present disclosure does not affect the composite structure of Hsp90 and HSF-1, thus does not induce the activation of HSF-1 or the expression of Hsp genes, and hence effectively promotes apoptosis of cancer cells. Thus, the indazole derivative of the present disclosure may be used in the prevention, treatment, or amelioration of cancer.

### BRIEF DESCRIPTION OF THE DRAWINGS

FIG. 1 shows the cell viability after treatment with the indazole derivatives of the present disclosure corresponding to formulas 1-1 to 1-13 in JIMT-1 and MDA-MB-231 cell lines.
FIG. 2 shows the cell viability after treatment with the indazole derivatives of the present disclosure corresponding to formulas 1-14 to 1-25 in JIMT-1 and MDA-MB-231 cell lines.
FIG. 3 shows the cell viability after treatment with the indazole derivatives of the present disclosure corresponding to formulas 1-26 to 1-41 in JIMT-1 and MDA-MB-231 cell lines.
FIG. 4 shows the cell viability after treatment with the indazole derivatives of the present disclosure corresponding to formulas 1-42 to 1-53 in JIMT-1 and MDA-MB-231 cell lines.
FIG. 5 shows the anticancer effect of compound 18c (formula 1-16) in breast cancer cell lines. FIG. 5A shows the concentration-dependent decrease in cell viability in BT474, JIMT-1, and MDA-MB-231; FIG. 5B shows the degree of cancer cell death confirmed through flow cytometry; and FIG. 5C shows the expression of cell death (apoptosis)-related proteins confirmed through western blotting.
FIG. 6 shows the anticancer effect of compound 11k (formula 1-7) in breast cancer cell lines. FIG. 6A shows the concentration-dependent decrease in cell viability in BT474, JIMT-1, and MDA-MB-231; FIG. 6B shows the degree of cancer cell death confirmed through flow cytometry; and FIG. 6C shows the expression of cell death (apoptosis)-related proteins confirmed through western blotting.
FIG. 7 shows the concentration-dependent decrease in cell viability of compound 11m (formula 1-9), compound 11n (formula 1-10), and compound 12k (formula 1-13) in JIMT-1 and MDA-MB-231 cell lines.
FIG. 8 shows the anticancer effect of compound 38 (formula 1-30) in breast cancer cell lines. FIG. 8A shows the concentration-dependent decrease in cell viability in MDA-MB-231; FIG. 8B shows the concentration-dependent decrease in cell viability in BT549; FIG. 8C shows the concentration-dependent decrease in cell viability in 4T1; FIG. 8D shows the change of cell morphology; and FIG. 8E shows apoptosis.
FIG. 9 shows the results of measuring the expression of Hsp90 clients of compound 18c (formula 1-16) (FIG. 9A) and compound 11k (formula 1-7) (FIG. 9B).

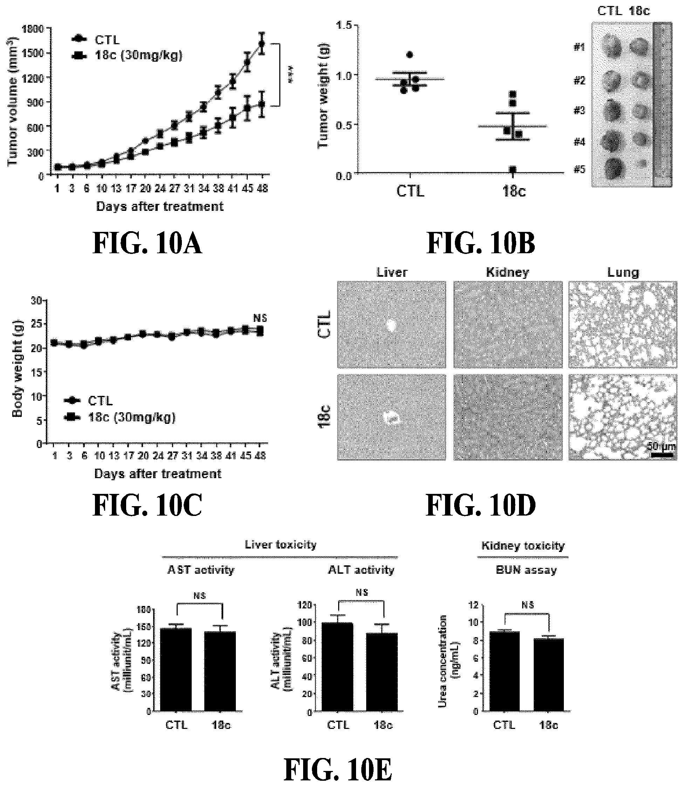

FIG. 10 shows the in vivo anticancer effect of compound 18c (formula 1-16). FIG. 10A shows the change in tumor volume after treatment with compound 18c; FIG. 10B shows the change in tumor weight and size after treatment with compound 18c; FIG. 10C shows the change in body weight after treatment with compound 18c; FIG. 10D shows the results of examining the change in histological morphology of other organs (liver, kidney, and lung) after treatment with compound 18c; and FIG. 10E shows the results of examining the presence or absence of liver and kidney function abnormalities after treatment with compound 18c.

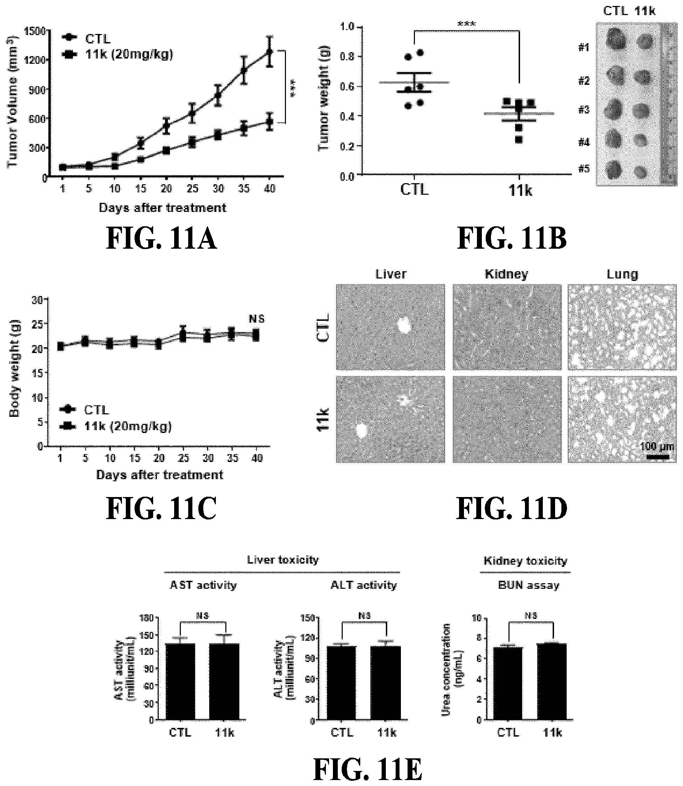

FIG. 11 shows the in vivo anticancer effect of compound 11k (formula 1-7). FIG. 11A shows the change in tumor volume after treatment with compound 11k; FIG. 11B shows the change in tumor weight and size after treatment with compound 11k; FIG. 11C shows the change in body weight after treatment with compound 11k; FIG. 11D shows the results of examining the change in histological morphology of other organs (liver, kidney, and lung) after treatment with compound 11k; and FIG. 11E shows the results of examining the presence or absence of liver and kidney function abnormalities after treatment with compound 11k.
FIG. 12 shows the cell viability after treatment with compound 11k (chemical formula 1-7) in a leukemia cell line (HL-60), a hepatic cancer cell line (HepG2), a colorectal cancer cell line (HCT116), a prostate cancer cell line (Du145), an ovarian cancer cell line (SKOV3), and a non-small cell lung cancer cell line (NCI-H1299 and A549).
FIG. 13 shows the combination index (CI) according to the concentration of combined treatment with compound 11k (formula 1-7) and paclitaxel.
FIG. 14 shows the in vivo anticancer effect when compound 11k (formula 1-7) is administered in combination with paclitaxel. FIG. 14A shows the change in tumor volume upon the co-administration; FIG. 14B shows the change in tumor weight upon the co-administration; FIG. 14C shows the change in tumor size upon the co-administration; FIG. 14D shows the change in body weight upon the co-administration; and FIG. 14E shows the evaluation of liver toxicity and kidney toxicity upon the co-administration.
FIG. 15 shows the cell viability after treatment with the indazole derivative of the present disclosure and the benzopyran derivative of the related art (KR 10-2304532) in the BT474 cell line.
FIG. 16 shows the cell viability after treatment with the indazole derivative of the present disclosure and the benzopyran derivative of the related art (KR 10-2304532) in the JIMT-1 cell line.
FIG. 17 shows the cell viability after treatment with the indazole derivative of the present disclosure and the benzopyran derivative of the related art (KR 10-2304532) in the MDA-MB-231 cell line.

### BEST MODE FOR CARRYING OUT THE INVENTION

The present inventors conducted extensive research on indazole derivatives or pharmaceutically acceptable salts thereof and confirmed the anticancer activity of the derivatives and then created the present disclosure.

More specifically, the present inventors confirmed that the indazole derivative inhibits cancer cell growth and proliferation; induces cell death; and exhibits cytotoxicity by inhibiting Hsp90, and in particular, that it specifically inhibits tumor growth without affecting normal organs.

Based on the results mentioned above, the present disclosure provides an indazole derivative represented by the following [Formula 1], or a pharmaceutically acceptable salt thereof. 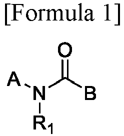 wherein, in Formula 1,
A is 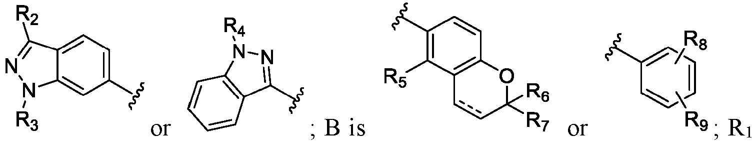 and R₂ are identical or different from each other, and each represents hydrogen or a C₁-C₆ chained alkyl group; R₃ and R₄ are identical or different from each other, and each represents hydrogen or a C₁-C₆ chained alkyl group, wherein at least one hydrogen of the alkyl group is unsubstituted or substituted with a C₁-C₆ alkoxy group, a C₅-C₁₀ heterocycloalkyl group, or a C₅-C₁₀ heteroaryl group, wherein the alkoxy group is unsubstituted or substituted with a C₁-C₆ alkoxy group, and the heterocycloalkyl group is unsubstituted or substituted with a C₁-C₆ chained alkyl group, 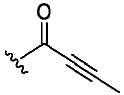 or 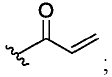 R₅ is a halogen group or a C₁-C₆ alkoxy group; R₆ and R₇ are identical or different from each other, and each represents a C₁-C₆ chained alkyl group; R₈ is NR₁₀C(O)R₁₁, OR₁₂, a C₅-C₁₀ heteroaryl group, or a tetrahydropyranyl amine group; R₉ is hydrogen, a C₁-C₆ alkoxy group, or a C₅-C₁₀ heterocycloalkyl group, wherein the heterocycloalkyl group is unsubstituted or substituted with a C₁-C₆ chained alkyl group; R₁₀ and R₁₁ are identical or different from each other, and each represents a C₁-C₆ chained alkyl group or C₅-C₁₀ aryl group, wherein the alkyl group is unsubstituted or substituted with a methylpiperazinyl group; R₁₂ is a C₅-C₁₀ aryl group or a C₅-C₁₀ benzyl group, wherein the benzyl group is unsubstituted or substituted with a halogen group; - - - may be a single bond or a double bond.

As used herein, the term "substitution" refers to a reaction in which an atom or atomic group contained in a molecule of a compound is replaced with another atom or atomic group.

As used herein, the term "chained alkyl group" means a group derived from linear or branched saturated aliphatic hydrocarbon having a certain number of carbon atoms and at least one valence. Examples of such alkyl groups include, but are not limited to, methyl, ethyl, propyl, isopropyl, butyl, isobutyl, 2-butyl, 3-butyl, pentyl, n-hexyl, and the like.

As used herein, the term "cycloalkyl group", also called cyclic alkyl group, refers to a monovalent group having one or more saturated rings in which all ring members are carbon. Examples of such cycloalkyl groups include, but are not limited to, cyclobutyl groups, cyclopentyl groups, cyclohexyl groups, and the like.

As used herein, the term "heterocycloalkyl group" typically refers to a saturated or unsaturated (but not aromatic) cyclohydrocarbon, which may be optionally unsubstituted, mono-substituted or poly-substituted, wherein at least one of the structure is selected from heteroatom N, O or S.

As used herein, the term "aryl group" means an unsaturated aromatic ring compound having 6 to 20 carbon atoms having a single ring (e.g., phenyl) or a plurality of condensed rings (e.g., naphthyl). Examples of such aryl groups include, but are not limited to, phenyl, naphthyl, etc.

As used herein, the term "heteroaryl group" refers to a single ring or a plurality of condensed rings in which at least one of the atoms constituting the ring is a heteroatom of N, O, or S.

As used herein, the term "alkoxy group" means an atomic group CₙH_{2n 1}O⁻ composed of an oxygen atom bonded to an alkyl group. Examples of such alkoxy groups include, but are not limited to, methoxy, ethoxy, propoxy, or phthoxy groups.

As used herein, the term "halogen group" refers to elements belonging to group 17 of the periodic table, which may include, but are not limited to, fluorine (F), chloride (Cl), bromine (Br), iodine (I), and the like.

As used herein, the term "pharmaceutically acceptable salt" means a formulation of a compound that does not cause serious irritation to the organism to which the compound is administered and does not impair the biological activity and properties of the compound. The pharmaceutically acceptable salt of the compound of the present disclosure may be obtained by reacting the compound of the present disclosure with inorganic acids such as hydrochloric acid, hydrobromic acid, sulfuric acid, nitric acid, and phosphoric acid; sulfonic acids such as methanesulfonic acid, ethanesulfonic acid, and p-toluenesulfonic acid; organic carboxylic acids such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, and salicylic acid. In addition, the pharmaceutically acceptable salt of the compound of the present disclosure may be obtained by reacting the compound of the present disclosure with a base to form salts such as ammonium salt, alkali metal salt such as sodium or potassium salt, alkaline earth metal salt such as calcium or magnesium salt, salts of organic bases such as dicyclohexylamine, N-methyl-D-glucamine, and tris(hydroxymethyl)methylamine, and salts of amino acids such as arginine and lysine.

Furthermore, the indazole derivative or a pharmaceutically acceptable salt thereof may include not only pharmaceutically acceptable salts, but also all salts, hydrates and solvates that may be prepared by conventional methods.

In addition, the present disclosure provides a pharmaceutical composition for use in a method for preventing or treating cancer, including the indazole derivative or a pharmaceutically acceptable salt thereof as an active ingredient.

As used herein, the term "prevention" refers to all actions that inhibit or delay the occurrence, spread, or recurrence of cancer by the administration of the composition of the present disclosure, and the term "treatment" refers to all actions by which the symptoms of the disease are ameliorated or beneficially changed by the administration of a composition of the present disclosure.

As used herein, the term "pharmaceutical composition" means a composition manufactured for the prevention or treatment of the disease, which may be formulated and used in various forms according to conventional methods. For example, the composition may be formulated in oral dosage forms such as powders, granules, tablets, capsules, suspensions, emulsions, syrups, and the like, and may be formulated and used in the form of external preparations, suppositories, and sterile injectable solutions.

As used herein, the phrase "including as an active ingredient" means that the ingredient is included in an amount necessary or sufficient to realize a desired biological effect. In practical application, the amount to be included as an active ingredient may be determined to be an amount to treat the disease of interest and not cause other toxicities. For example, the amount may vary depending on a variety of factors, such as the disease or condition being treated, the form of the composition being administered, the size of the individual, or the severity of the disease or condition. A person of ordinary skill in the art to which the present disclosure pertains will be able to determine empirically the effective amount of an individual composition without undue experimentation.

In addition, the pharmaceutical composition of the present disclosure may, depending on each formulation, further include one or more pharmaceutically acceptable carriers, in addition to the active ingredients described above.

The pharmaceutically acceptable carrier may be saline, sterile water, Ringer's solution, buffered saline, dextrose solution, maltodextrin solution, glycerol, ethanol, and a mixture of one or more of these components, and may further include other conventional additives, such as antioxidants, buffers, bacteriostatic agents, and the like. In addition, the composition may be formulated in injectable forms, such as aqueous solutions, suspensions, emulsions, etc., pills, capsules, granules or tablets by additionally adding diluents, dispersants, surfactants, binders and lubricants. Further, the composition may be preferably formulated depending on the disease or ingredient, by any suitable method in the art, or as disclosed in Remington's Pharmaceutical Science (Mack Publishing Company, Easton PA).

The pharmaceutical composition of the present disclosure may be administered orally or parenterally in a pharmaceutically effective amount, depending on the intended method, and the term "pharmaceutically effective amount" as used herein means an amount sufficient to treat a disease at a reasonable benefit/risk ratio applicable to medical treatment and not cause side effects. The effective dose level may be determined based on factors including the patient's health condition, severity, activity of the drug, sensitivity to the drug, method of administration, time of administration, route of administration and rate of elimination, duration of treatment, drugs used in combination or concurrently, and other factors well known in the medical field.

In addition, the present disclosure may provide a method for preventing or treating cancer, including administering the indazole derivative or a pharmaceutically acceptable salt thereof to a subject.

As used herein, the term "individual" refers to, but is not limited to, any mammal, such as a domestic animal or human in need of prevention, treatment, and/or diagnosis of the diseases, but preferably a human.

As used herein, the term "administration" means providing a predetermined substance to a patient by any appropriate method. The pharmaceutical composition of the present disclosure may be formulated in various forms for administration to an individual, and a representative form for parenteral administration is an injectable form, preferably an isotonic aqueous solution or suspension. The injectable form may be prepared by techniques known in the art using a suitable dispersing or wetting agent and a suspending agent. For example, each component may be dissolved in a saline or buffer solution, and then the mixture may be formulated for injection. The formulations for oral administration include, for example, swallowable tablets, buccal tablets, troches, capsules, elixirs, suspensions, syrups, and wafers, which may include, in addition to an active ingredient, a diluent (e.g., lactose, dextrose, sucrose, mannitol, sorbitol, cellulose, and/or glycine) and a lubricant (e.g., silica, talc, stearic acid and its magnesium or calcium salt, and/or polyethylene glycol). The tablets may include a binder such as magnesium aluminum silicate, starch paste, gelatin, tragacanth, methylcellulose, sodium carboxymethylcellulose, and/or polyvinylpyrrolidine, and in some instances, may further include disintegrating agents such as starch, agar, alginic acid or its sodium salts, absorbents, colorants, flavoring agents, and/or sweeteners. The formulations may be prepared by conventional mixing, granulating, or coating methods.

Further, the pharmaceutical composition of the present disclosure may additionally contain adjuvants such as preservatives, hydrating agents, emulsification accelerators, salts or buffers for osmotic pressure control, and other therapeutically useful substances, and may be formulated according to a conventional method.

The pharmaceutical composition of the present disclosure may be administered via several routes, including oral, transdermal, subcutaneous, intravenous, and intramuscular, and the dosage of the active ingredient may be adequately selected depending on several factors, including the route of administration, the age, gender, and weight of the patient, and the severity of the patient's disease. Additionally, the pharmaceutical composition of the present disclosure may be administered in combination with known compounds, which may enhance the desired effect.

The pharmaceutical composition of the present disclosure may be administered to humans and animals orally or parenterally, for example, intravenously, subcutaneously, intranasally or intraperitoneally. Oral administration also includes sublingual application. Parenteral administration includes injections and drop methods such as subcutaneous injection, intramuscular injection, and intravenous injection.

In the pharmaceutical composition of the present disclosure, the total effective dose of the indazole derivative or a pharmaceutically acceptable salt thereof according to the present disclosure may be administered to a patient in a single dose, or may be administered to a patient in multiple doses over a longer period of time in accordance with a fractionated treatment protocol. The pharmaceutical composition of the present disclosure may vary in the amount of active ingredients depending on the severity of the disease. Typically in an adult, the composition may be administered multiple times per day at an effective dose of 100 µg to 3,000 mg per dose, but is not limited thereto. Further, the concentration of the indazole derivative or a pharmaceutically acceptable salt thereof may vary depending on the route of administration and the number of treatments. In addition, the effective dose for the patient may be determined considering various factors such as the patient's age, body weight, health status, gender, the severity of the disease, and the patient's diet and excretion rate.

In addition, the pharmaceutical composition according to the present disclosure is not particularly limited in terms of formulation, route of administration and method of administration, so long as it exhibits the effects of the present disclosure, and may additionally comprise known medicaments in addition to the indazole derivative or a pharmaceutically acceptable salt thereof as an active ingredient, and may also be used in combination with other known therapies for the treatment of the diseases mentioned herein. The pharmaceutical composition according to the present disclosure may be administered with non-limiting examples of known anticancer agents, and the anticancer agent may be paclitaxel, docetaxel, doxorubicin, sorafenib, vemurafenib, irinotecan, cisplatin, alpharadin, mitoxantrone, cyclophosphamide, vinblastine, carboplatin, actinomycin-D, etoposide, teniposide, bisantrene, homoharringtonine, Gleevec (STI-571), 5-fluorouracil, busulfan, chlorambucil, melphalan, nitrogen mustard, nitrosoureas, and the like.

In addition, the present disclosure provides a cosmetic composition for preventing or ameliorating cancer, including the indazole derivative or a cosmetically acceptable salt thereof as an active ingredient.

As used herein, the term "amelioration" means any action that beneficially changes a parameter related to the condition being treated, for example, at least reducing the degree of symptoms, or improving the condition.

The cosmetic composition includes, for example, the indazole derivative or a cosmetically acceptable salt thereof as an active ingredient, and may be manufactured together with a dermatologically acceptable excipient, in the form of a basic cosmetic composition (a toner, a cream, an essence, a cleanser such as a cleansing foam and cleansing water, a pack, a body oil), a color cosmetic composition (foundation, lipstick, mascara, makeup base), a hair product composition (a shampoo, a rinse, a hair conditioner, a hair gel), and a soap.

The excipients may include, for example, skin softeners, skin penetration enhancers, colorants, fragrances, emulsifiers, thickeners and solvents, and more specifically, starch, glucose, lactose, sucrose, gelatin, malt, rice, flour, chalk, silica gel, sodium stearate, glycerol monostearate, talc, sodium chloride, anhydrous skim milk, glycerol, propylene, glycol, water, ethanol, and the like.

Furthermore, the present disclosure provides a food composition for preventing or ameliorating cancer, including the indazole derivative or a food-chemically acceptable salt thereof as an active ingredient.

The food composition includes, for example, the indazole derivative or a pharmaceutically acceptable salt thereof as an active ingredient, and when the indazole derivative is used as an additive to the food composition, it may be added as such, or in combination with other foods or food ingredients, and may be used as appropriate according to conventional methods. Typically, in the process of manufacturing a food or beverage, the composition of the present disclosure is added in an amount of 15 wt% or less, preferably 10 wt% or less, based on the raw materials. However, when consumption may be long-term for health and hygiene purposes or health control purposes, the amount may be lower than the lower limit of the above specified range, and the amount of active ingredients may be higher than the upper limit of the above specified range as there is no problem in terms of safety. That is, the amount of active ingredients mixed may be appropriately determined by each purpose of use, such as prevention, health, or treatment.

The dosage form of the food composition may be powders, granules, pills, tablets, capsules, as well as in the form of any other general food or beverage.

The food of the present disclosure may be produced by a method commonly used in the art, and may be produced by adding raw materials and ingredients commonly added in the art. Specifically, the raw materials and ingredients may include proteins, carbohydrates, fats, nutrients, seasonings, and flavoring agents. Examples of carbohydrates include, but are not limited to, glucose, fructose, maltose, sucrose, oligosaccharides, dextrin, cyclodextrin, xylitol, sorbitol, erythritol, saccharin, or synthetic flavoring agents.

In addition, the present disclosure provides a feed composition for preventing or treating cancer, including the indazole derivative or a feed-chemically acceptable salt thereof as an active ingredient.

The feed composition includes, for example, the indazole derivative or a feed-chemically acceptable salt thereof as an active ingredient. As used herein, the term "feed" means any natural or artificial diet, meal, and the like, or any component of the meal for consumption and digestion by livestock or suitable for livestock. The feed may contain feed additives or supplementary feeds.

The type of feed is not particularly limited, and any feed commonly used in the relevant technical field may be used. Non-limiting examples of such feed include plant-based feed such as grains, roots and fruits, food processing by-products, algae, fibers, pharmaceutical by-products, fats and oils, starches, cucurbitaceous, or grain by-products; animal-based feeds such as proteins, inorganic materials, fats, mineral materials, fats, single-cell proteins, animal planktons, or food-stuffs. These may be used alone or in combinations of two or more.

The terminology used herein is for the purpose of describing particular embodiments only and is not to be construed as limiting. The singular forms "a," "an," and "the" include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the meaning commonly understood by one of ordinary skill in the art to which the examples belong. It will be further understood that terms defined in commonly used dictionaries, should be interpreted as having a meaning that is consistent with the meaning in the context of the relevant art and should not be interpreted in an idealized or overly formal sense unless expressly so defined herein.

In addition, terms such as first, second, A, B, (a), (b), and the like may be used to describe components of the embodiments. These terms are used only for the purpose of discriminating one constituent element from another constituent element, and do not limit the nature, the sequences, or the orders of the constituent elements. It should be noted that if one component is described as being "connected," "coupled" or "joined" to another component, the former may be directly "connected," "coupled," and "joined" to the latter or "connected", "coupled", and "joined" to the latter via another component.

Hereinafter, embodiments will be described in detail with reference to the accompanying drawings.

### MODE FOR CARRYING OUT THE INVENTION

### Example 1. General preparation method

### Example 1.1. N-alkylation of indazole (Procedure A)

***Method I* :** To a mixture of 6-nitroindazole (1.0 eq), Cs₂CO₃ (1.5 eq) in DMF was added alkyl halide (1.1 eq) and the mixture was stirred at a temperature increasing from room temperature to 60°C over a period of 4 hours. After the reaction was completed, the reaction mixture was cooled to room temperature. Water was added thereto, and then the mixture was extracted twice with ethyl acetate. The organic layer was collected, washed three times with water, dried over MgSO₄, and concentrated in vacuo. The residue was purified by silica gel column chromatography to obtain the desired product.

***Method II*** : To a mixture of 6-nitroindazole (1.0 eq), Ph₃P (1.1 eq), and 2-substituted ethanol (1.1 eq) in anhydrous THF at 0°C was added diisopropyl azodicarboxylate (DIAP, 1.1 eq) and the mixture was stirred at room temperature for 4 hours. After the reaction was completed, the reaction mixture was concentrated in vacuo to remove the solvent. Then, the residue was purified by silica gel column chromatography to obtain the desired product.

***Method III* :** To a mixture of 3-methyl-6-nitroindazole (1.0 eq) and Cs₂CO₃ (1.5 eq) in DMF at 0°C was added 1,3-dibromoethane or 1,3-dibromopropane (1.2 eq). The reaction mixture was stirred at room temperature for 18 hours and quenched with water. The reaction mixture was extracted with EtOAc and washed with water. The organic layer was dried over MgSO₄ and concentrated in vacuo. The residue was purified by silica gel column chromatography to obtain the desired product.

### Example 1.2. Hydrogenation reaction (Procedure B)

To a solution of the starting material (1.0 eq) in MeOH-THF or MeOH co-solution was added 10% Pd/C, the solution was degassed, and refilled with hydrogen gas. The mixture was stirred at room temperature for 2-4 hours. After the reaction was completed, the reaction mixture was filtered through Celite and washed twice with MeOH. The filtrate was concentrated in vacuo, and then the residue was purified by silica gel column chromatography to obtain the desired product.

### Example 1.3. Amide coupling reaction (Procedure C)

***Method I*** : To a solution of carboxylic acid (1.1 eq), HOBt (1.5 eq), triethylamine (2.0 eq), and EDC·HCl (1.5 eq) in DCM at room temperature was added RNH₂ (1.0 eq) for 15 hours. After the reaction was completed, the organic compound was extracted with EtOAc and washed several times with water. The combined organic layer was washed with brine, dried over MgSO₄ and concentrated in vacuo. The residue was purified by silica gel column chromatography to obtain the desired product.

***Method II* :** RNH₂ or R₁R₂NH (1.0 eq) in THF was added to 2M NaOH (aq) and the mixture was stirred at room temperature. Then, acyl chloride (1.2 eq) was added dropwise thereto and the mixture was stirred at room temperature for 2 hours. After the solvent was removed, the mixture was acidified with 1 M HCl (aq). The organic compound was extracted with EtOAc, washed with water, dried over Na₂SO₄, and concentrated in vacuo. The residue was purified by silica gel column chromatography to obtain the desired product.

***Method III*** : To a mixture of carboxylic acid (1.0 eq) and catalytic amount of DMF in DCM was added SOCl₂ (0.5 mL), and the mixture was stirred at room temperature for 2 hours. After the reaction was completed, the solvent was removed. Then, the reaction mixture was dissolved in DCM, and amine (1.0 eq) and Et₃N (2.0 eq) were added at 0°C. After 1 hour at room temperature, the crude mixture was purified by column chromatography to obtain the desired product.

### Example 1.4. Boc deprotection (Procedure D)

TFA (20.0 eq) was added dropwise to a solution of the Boc-protected compound in DCM at 0°C and the solution was stirred at room temperature for 2-4 h. The solvent was removed and then the residue was purified by silica gel column chromatography to obtain the desired product.

### Example 1.5. Reductive amination reaction (Procedure E)

***Method I* :** To a mixture of primary arylamine (1.0 eq) and ketone or aldehyde (1.0 eq) in MeOH was added acetic acid (5.0 eq), followed by NaBH₃CN (3.0 eq) and then the mixture was stirred at 40°C. After 16 hours, the reaction mixture was basified with NaHCO₃ (aq) and then extracted with DCM. The organic layer was concentrated in vacuo, and then the residue was purified by silica gel column chromatography to obtain the desired product.

***Method II* :** To a solution of amine (1.0 eq) and tetrahydro-4H-pyran-4-one (5.0 eq) in DCM (2 mL) was added TFA (0.1 mL), followed by NaBH(OAc)₃ (5.0 eq) and then the mixed solution was stirred at room temperature for 18 hours. The mixture was neutralized with 10% K₂CO₃ (aq), extracted with DCM, and washed with water. The organic layer was dried over Na₂SO₄, concentrated in vacuo, and purified by preparative layer chromatography to obtain the desired product.

### Example 1.6. Preparation of piperidine derivatives (Procedure F)

***Method I* :** To a mixture of EDC·HCl (1.5 eq), tetrolic acid (2.0 eq), Et₃N (2.0 eq) and HOBt (1.5 eq) in DCM was added 2-(piperidin-4-yl)ethyl substituted intermediate (1.0 eq). The mixture was stirred for 15 hours. After being stirred, the mixture was extracted with EtOAc and washed with brine. The organic layer was dried over MgSO₄ and concentrated in vacuo. The residue was purified by silica gel column chromatography to obtain the desired product.

***Method II*** : To a mixture of 2-(piperidin-4-yl)ethyl substituted intermediates (1.0 eq) in DCM was added Et₃N (2.0 eq), followed by acrylic oil chloride (2.0 eq) at 0°C. The reaction mixture was stirred at room temperature for 3 hours, extracted with EtOAc, and washed with brine. The organic layer was dried over MgSO₄ and concentrated in vacuo. The residue was purified by silica gel column chromatography to obtain the desired product.

### Example 1.7. Preparation of piperazine derivatives (Procedure G)

To 2-bromoethyl or 2-bromopropyl indazole in acetonitrile was added *N-*methylpiperazine (3.0 eq), and the mixture was stirred at room temperature for 15 hours. The reaction mixture was diluted with DCM, washed two times with water, dried over MgSO₄, and concentrated in vacuo. The residue was purified by silica gel column chromatography to obtain the desired product.

### Example 1.8. O-alkylation of phenol (Procedure H)

***Method I* :** The mixture of ROH (1.0 eq), RX (1.5 eq), and K₂CO₃ (2.0 eq) in DMF (4 mL) at 40°C was stirred for 3 hours. The reaction mixture was cooled to room temperature, extracted with EtOAc and washed with water. The organic layer was dried over anhydrous Na₂SO₄ and concentrated in vacuo. The residue was purified by silica gel column chromatography to obtain the desired product.

***Method II*** : Alkyl halide (2.0 eq) was added to a solution of ROH (1.0 eq) and 20% NaOH (aq, 10 eq) in EtOH. The reaction mixture was stirred under reflux for 18 hours. After the reaction was completed, the solvent was removed. Then, the residue was acidified with 1 M Hcl (aq) and extracted with EtOAc. The organic layer was washed with water, dried over anhydrous Na₂SO₄ and concentrated in vacuo. The residue was purified by silica gel column chromatography to obtain the desired product.

***Method III* :** To a solution of ROH (1.0 eq) in DMF (2 mL) at room temperature were added K₂CO₃ (2.0 eq), KI (2.0 eq), CuI (0.05 eq), and RX (1.1 eq) over a period of 15 hours. The mixture was extracted with EtOAc and washed with water. The organic layer was dried over anhydrous Na₂SO₄ and concentrated in vacuo. The residue was purified by silica gel column chromatography to obtain the desired product.

### Example 1.9. Preparation of 2,2-dialkyl-2H-chromene (Procedure I)

***Method I* (intramolecular cyclization):** The starting material solution in *o-*xylene (2 mL) was heated at 180°C for 1 hour. The solvent was evaporated, then the residue was extracted with EtOAc and washed with water. The organic layer was dried over Na₂SO₄ and concentrated in vacuo. The residue was purified by silica gel column chromatography to obtain the desired product.

***Method II* (intermolecular cyclization):** 3-methyl-2-butenal (2.0 eq), anhydrous calcium chloride (1.0 eq), and triethylamine (2.0 eq) were added to a solution of a phenol derivative (1.0 eq) in ethanol, and the mixture was refluxed for 2 hours. The solvent was removed and the residue was diluted, extracted with EtOAc, dried over MgSO₄, and concentrated in vacuo. The residue was purified by silica gel column chromatography to obtain the desired product.

### Example 1.10. Phinnick oxidation reaction (Procedure J)

To a mixture of aldehyde (1.0 eq), 30% H₂O₂ (1.0 eq) and NaH₂PO₄ (0.2 eq) in acetonitrile at 0°C was added NaClO₂ (1.5 eq). The reaction mixture was stirred at room temperature for 2 hours. The mixture was quenched with water, extracted with EtOAc, and dried over MgSO₄. The residue was purified by silica gel column chromatography to obtain the desired product.

### Example 1.11. Hydrolysis reaction (Procedure K)

To a solution of the nitrile derivative (1.0 eq) in EtOH (4 mL) and H₂O (1 mL) was added NaOH (10.0 eq). The reaction mixture was stirred at 120°C in a sealed tube for 3 hours. The mixture was acidified with 2N HCl (aq), extracted with EtOAc, dried over anhydrous Na₂SO₄ and concentrated in vacuo. The residue was purified by silica gel column chromatography to obtain the desired product.

### Example 1.12. Suzuki coupling reaction (Procedure L)

A mixture of boronic acid (1.0 eq), RBr (1.0 eq), K₂CO₃ (3.0 eq) and Pd(PPh₃)₄ (0.05 eq) in EtOH: H₂O (1:1) or BuOH: H₂O (1:1) at 90°C or 110°C was stirred. The solvent was evaporated, the crude mixture was dissolved in water, and then filtered through Celite. The filtrate was acidified to pH 3 with formic acid and extracted with EtOAc. The organic layer was dried over anhydrous Na₂SO₄ and concentrated in vacuo. The residue was purified by silica gel column chromatography to obtain the desired product.

### Example 1.13. Benzyl deprotection (Procedure M)

1-chloroethyl chloroformate (1.0 eq) was added to a solution of RBn in DCM and refluxed for 1 hour. NaHCO₃ (aq) was added to the mixed solution to complete the reaction, and the solution was extracted with DCM, dried over MgSO₄ and concentrated in vacuo. The residue was purified by silica gel column chromatography to obtain the desired product.

### Example 1.14. Phthalimide deprotection (Procedure N)

Hydrazine hydrate (5.0 eq) was added to a solution of RPhth (1.0 eq) in EtOH and the mixed solution was stirred at room temperature for 15 hours. The solid residue was filtered and the filtrate was concentrated in vacuo. The residue was used in the next step without purification.

### Example 2. Preparation of 6-aminoindazole derivatives

A method for producing 6-aminoindazole derivatives is shown by the following scheme 1. (a) [Method A] RX, Cs₂CO₃, DMF, room temperature, 15 hours; [Method B] ROH, DEAD, Ph₃P, THF, room temperature, 15 hours; (b) H₂, 10% Pd/C, THF-MeOH, room temperature, 2 hours; (c) **29,** EDC·HCl, HOBt, Et₃N, DCM, room temperature, 15 hours; (d) TFA, DCM, room temperature, 3 hours; (e) For **7k, 8k** and **7m,** [Method A] (i) (HCHO)ₙ, ZnCl₂, MC, (ii) NaBH₄; for **7l, 7n,** [Method B] 1-methylpiperazine, K₂CO₃, CH₃CN, 70°C, 3 hours; (f) For **11o,** [Method A] RCl, Et₃N, MC, from 0°C to room temperature, 3 hours; for **11p,** [Method B] RCOOH, EDC·HCl, HOBt, Et₃N, MC, room temperature, 15 hours.

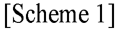

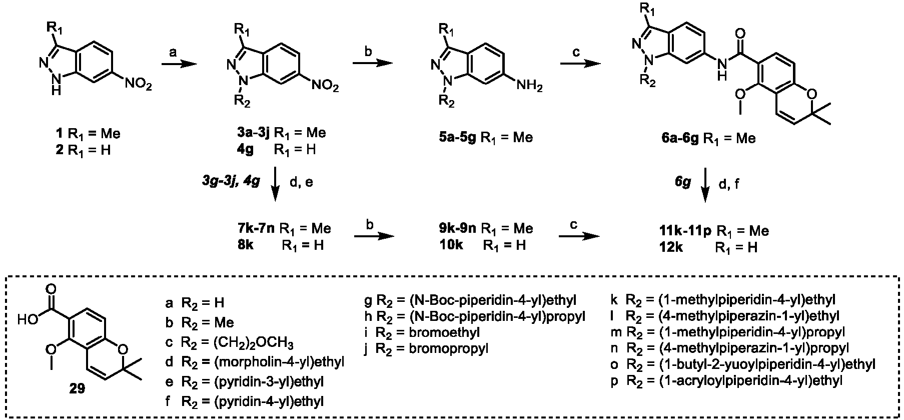

### Example 2.1. Preparation of 5-methoxy-2,2-dimethyl-N-(3-methyl-1H-indazole-6-yl)-2H-chromene-6-carboxamide (formula 1-1, 6a)

### Example 2.1.1. Preparation of 3-methyl-1H- indazol-6-amine (5a)

Using procedure B, compound **5a** was prepared from compound 1 as a red solid at a 98% yield. ¹H NMR (500 MHz, CDCl₃) *δ* 7.33 (d, *J* = 8.55 Hz, 1H), 6.54 (d, *J* = 1.40 Hz, 1H), 6.49 (dd, *J* = 8.55, 1.65 Hz, 1H), 3.36 (brs, 2H), 2.39 (s, 3H).

### Example 2.1.2. Preparation of 5-methoxy-2,2-dimethyl-2H-chromene-6-carbaldehyde (24)

Using procedure I-method II and procedure A-method I, compound **24** was prepared with iodomethane from compound **21** as a pale yellow oil at a 60% yield. ¹H NMR (400 MHz, CDCl₃) δ 10.16 (s, 1H), 7.64 (d, *J* = 8.6 Hz, 1H), 6.64 (d, *J* = 8.5 Hz, 1H), 6.59 (d, *J* = 10.0 Hz, 1H), 5.68 (d, *J* = 10.1 Hz, 1H), 3.89 (s, 3H), 1.45 (s, 6H).

### Example 2.1.3. Preparation of 5-methoxy-2,2-dimethyl-2H-chromene-6-carboxylic acid (29)

Using procedure J, compound **29** was obtained from compound **24** as a white solid at a 69% yield. ¹H NMR (400 MHz, CDCl₃) δ 10.61 (br s, 1H), 7.90 (d, *J* = 8.7 Hz, 1H), 6.69 (d, *J* = 8.7 Hz, 1H), 6.53 (d, *J* = 10.0 Hz, 1H), 5.73 (d, *J* = 10.1 Hz, 1H), 3.94 (s, 3H), 1.46 (s, 6H).

### Example 2.1.4. Preparation of 5-methoxy-2,2-dimethyl-N-(3-methyl-1H-indazol-6-yl)-2H-chromene-6-carboxamide (6a)

Using procedure C-method I, compound **6a** was prepared from compound **5a** and compound **29** as a white solid at an 81% yield. mp 244-245°C. ¹H NMR (500 MHz, CDCl₃) *δ* 9.95 (s, 1H), 8.34 (s, 1H), 7.98 (d, *J* = 8.65 Hz, 1H), 7.60 (d, *J* = 8.60 Hz, 1H), 7.07 (d, *J* = 8.50 Hz, 1H), 6.72 (d, *J* = 8.70 Hz, 1H), 6.59 (d, *J* = 10.0 Hz, 1H), 5.72 (d, *J* = 10.0 Hz, 1H), 3.89 (s, 3H), 2.61 (s, 3H), 1.46 (s, 6H). ESI-MS [M+H]⁺ 364.

### Example 2.2. Preparation of N-(1.3-dimethyl-1H-indazole-6-yl)-5-methoxy-2,2-dimethyl-2H-chromene-6-carboxamide (formula 1-2, 6b)

### Example 2.2.1. Preparation of 1,3-dimethyl-6-nitro-1H-indazole (3b)

Using procedure A-method I, compound **3b** was prepared from compound 1 and iodoethane as a yellow solid at a 62% yield. ¹H NMR (300MHz, CDCl₃) *δ* 8.58 (dd, *J* = 1.83, 0.75 Hz, 1H), 7.81 (dd, *J* = 9.15, 2.01 Hz, 1H), 7.63 (dd, *J* = 9.15, 0.57 Hz, 1H), 4.16 (s, 3H), 2.56 (s, 3H).

### Example 2.2.2. Preparation of 1,3-dimethyl-1H-indazole-6-amine (5b)

Using procedure B, compound **5b** was prepared from compound **3b** as a reddish solid at a 92% yield. ¹H NMR (300MHz, CDCl₃) *δ* 7.37 (d, *J* = 8.22 Hz, 1H), 5,52-6.50 (m, 2H), 3.96 (s, 3H), 3.85 (brs. 2H), 2.46 (s, 3H).

### Example 2.2.3. Preparation of N-(1,3-dimethyl-1H-indazole-6-yl)-5-methoxy-2,2-dimethyl-2H-chromene-6-carboxamide (6b)

Using procedure C-method I, compound **6b** was prepared from compound **5b** and compound **29** prepared according to Example 2.1.3 as a white solid at an 87% yield. mp 166-167°C. ¹H NMR (400 MHz, CDCl₃) *δ* 9.90 (s, 1H), 8.31 (d, *J* = 1.28 Hz, 1H), 7.98 (d, *J* = 8.64 Hz, 1H), 7.95 (d, *J* = 8.48 Hz, 1H), 6.86 (dd, *J* = 8.56, 1.68 Hz, 1H), 6.72 (d, *J* = 8.68 Hz, 1H), 6.59 (d, *J* = 10.0 Hz, 1H), 5.72 (d, *J* = 10.0 Hz, 1H), 3.98 (s, 3H), 3.90 (s, 3H), 2.50 (s, 3H), 1.46 (s, 6H). ESI-MS [M+H]⁺ 378.

### Example 2.3. Preparation of 5-methoxy-N-(1-(2-methoxyethyl)-3-methyl-1H-indazole-6-yl)-2,2-dimethyl-2H-chromene-6-carboxamide (formula 1-3, 6c)

### Example 2.3.1. Preparation of 1-(2-methoxyethyl)-3-methyl-6-nitro-1H-indazole (3c)

Using procedure A-method I, compound **3c** was prepared from compound **1** and 2-bromoethyl methyl ether as a yellow solid at a 42% yield. ¹H NMR (400MHz, CDCl₃) *δ* 8.39 (d, *J* = 2.0 Hz, 1H), 7.95 (dd, *J* = 8.4, 1.6 Hz, 1H), 7.71 (d, *J* = 8.8 Hz, 1H), 4.54 (t, *J* = 5.6 Hz, 2H), 3.81 (t, *J* = 5.2 Hz, 2H), 3.29 (s, 3H), 2.60 (s, 3H).

### Example 2.3.2. Preparation of 1-(2-methoxyethyl)-3-methyl-1H-indazole-6-amine(5c)

Using procedure B, compound **5c** was prepared from compound **3c** as a reddish solid at a 95% yield. ¹H NMR (400MHz, CDCl₃) *δ* 7.37 (d, *J* = 8.0 Hz, 1H), 6.54-6.50 (m, 2H), 4.32 (t, *J* = 6.0 Hz, 2H), 3.82 (brs. 2H), 3.76 (t, *J* = 6.0 Hz, 2H), 3.30 (s, 3H), 2.47 (s, 3H).

### Example 2.3.3. Preparation of 5-methoxy-N-(1-(2-methoxyethyl)-3-methyl-1H-indazole-6-yl)-2,2-dimethyl-2H-chromene-6-carboxamide (6c)

Using procedure C-method I, compound **6c** was prepared from compound **5c** and compound **29** prepared according to Example 2.1.3 as a white solid at a 73% yield. mp 113-114°C. ¹H NMR (400MHz, CDCl₃) *δ* 9.92 (s, 1H), 8.31 (d, *J* = 1.2 Hz, 1H), 7.98 (d, *J* = 8.8 Hz, 1H), 7.54 (d, *J* = 8.8 Hz, 1H), 6.93 (dd, *J* = 8.8, 2.0 Hz, 1H), 6.73 (d, *J* = 8.4 Hz, 1H), 6.60 (d, *J* = 9.6 Hz, 1H), 5.73 (d, *J* = 10 Hz, 1H), 4.48 (t, *J* = 5.6 Hz, 2H), 3.90 (s, 3H), 3.82 *(t, J* = 5.2 Hz, 2H), 3.32 (s, 3H), 2.54 (s, 3H), 1.47 (s, 6H). ESI-MS [M+H]⁺ 422.

### Example 2.4. Preparation of 5-methoxy-2,2-dimethyl-N-(3-methyl-1-(2-morpholinoethyl)-1H-indazole-6-yl)-2H-chromene-6-carboxamide (formula 1-4, 6d)

### Example 2.4.1. Preparation of 4-(2-(3-methyl-6-nitro-1H-indazole-1-yl)ethyl)morpholine (3d)

Using procedure A-method II, compound **3d** was prepared from compound **1** and 4-(2-hydroxyethyl)morpholine as a yellow solid at a 61% yield. ¹H NMR (400MHz, CDCl₃) *δ* 8.38 (d, *J* = 2.0 Hz, 1H), 7.95 (dd, *J* = 8.8, 2.0 Hz, 1H), 7.71 (d, *J* = 8.8 Hz, 1H), 4.49 (t, *J* = 6.0 Hz, 1H), 3.64 (br, 4H), 2.84 (t-like, 2H), 2.59 (s, 2H), 2.50 (br, 4H).

### Example 2.4.2. Preparation of 3-methyl-1-(2-morpholinoethyl)-1H-indazole-6-amine (5d)

Using procedure B, compound **5d** was prepared from compound **3d** as a brown solid at a 91% yield. ¹H NMR (400MHz, CDCl₃) *δ* 7.38 (d, *J* = 9.2 Hz, 1H), 6.53-6.50 (m, 2H), 4.34 (t, *J* = 6.8 Hz, 2H), 3.85 (brs, 1H), 3.72 (t, *J* = 4.0 Hz, 4H), 2.85 (br, 2H), 2.55 (br, 4H), 2.46 (s, 3H).

### Example 2.4.3. Preparation of methoxy-2,2-dimethyl-N-(3-methyl-1-(2-morpholinoethyl)-1H-indazole-6-yl)-2H-chromene-6-carboxamide (6d)

Using procedure C-method I, compound **6d** was prepared from compound **5d** and compound **29** prepared according to Example 2.1.3 as a white solid at an 88% yield. mp 89-90°C. ¹H NMR (400MHz, CDCl₃) *δ* 9.93 (s, 1H), 8.36 (d, *J* = 0.8 Hz, 1H), 7.96 (d, *J* = 4.4 Hz, 1H), 7.52 (d, *J* = 8.4 Hz, 1H), 8.65 (dd, *J* = 8.8, 1.2 Hz, 1H), 6.71 (d, *J* = 8.8 Hz, 1H), 6.58 (d, *J* = 10.0 Hz, 1H), 5.71 (d, *J* = 10.0 Hz, 1H), 4.42 (t, *J* = 7.2 Hz, 2H), 3.90 (s, 3H), 3.67 (t, *J* = 4.4 Hz, 4H), 2.84 (t, *J* = 7.6 Hz, 2H), 2.53-2.51 (m, 6H), 1.45 (s, 6H). ESI-MS [M+H]⁺ 477.

### Example 2.5. Preparation of 5-methoxy-2,2-dimethyl-N-(3-methyl-1-(2-(pyridine-3-yl)ethyl)-1H-indazole-6-yl)-2H-chromene-6-carboxamide (formula 1-5, 6e)

### Example 2.5.1. Preparation of 3-methyl-6-nitro-1-(2-(pyridine-3-yl)ethyl)-1H-indazole (3e)

Using procedure A-method II, compound **3e** was prepared from compound **1** and 2-(pyridine-3-yl)ethanol as a yellow solid at a 73% yield. ¹H NMR (400MHz, CDCl₃) *δ* 8.41 (dd, *J* = 5.2, 2.0 Hz, 1H), 8.25 (d, *J* = 2.0 Hz, 1H), 7.98 (d, *J* = 2.0 Hz, 1H), 7.91 (dd, *J* = 9.2, 2.0 Hz, 1H), 7.70 (d, *J* = 9.2 Hz, 1H), 7.55-7.43 (1H, mix), 7.16 (dd, *J* = 8.0, 4.8 Hz, 1H), 4.59 (t, *J* = 7.2 Hz, 2H), 3.24 (t, *J* = 7.2 Hz, 2H), 2.61 (s, 3H).

### Example 2.5.2. Preparation of 3-methyl-1-(2-(pyridine-3-yl)ethyl)-1H-indazole-6-amine (5e)

Using procedure B, compound **5e** was prepared from compound **3e** as a brown solid at a 92% yield. ¹H NMR (400MHz, CDCl₃) *δ* 8.43 (d, *J* = 4.8 Hz, 1H), 8.42 (s, 1H), 7.37 (d, *J* = 8.4 Hz, 1H), 7.35 (dt, *J* = 7.6, 2.0 Hz, 1H), 7.12 (dd, *J* = 7.6, 4.8 Hz, 1H), 6.48 (dd, *J* = 8.8, 1.6 Hz, 1H), 6.25 (d, *J* = 1.6 Hz, 1H), 4.35 (t, *J* = 7.2 Hz, 2H), 7.38 (brs, 2H), 3.15 (t, *J* = 7.2 Hz, 2H), 2.48 (s, 3H).

### Example 2.5.3. Preparation of 5-methoxy-2,2-dimethyl-N-(3-methyl-1-(2-(pyridine-3-yl)ethyl)-1H-indazole-6-yl)-2H-chromene-6-carboxamide (6e)

Using procedure C-method I, compound **6e** was prepared from compound **5e** and compound **29** prepared according to Example 2.1.3 as a white solid at an 80% yield. mp 119-120°C. ¹H NMR (400MHz, CDCl₃) *δ* 9.91 (s, 1H), 8.42 (dd, *J* = 5.2, 2.0 Hz, 1H), 8.38 (d, *J* = 1.6 Hz, 1H), 8.21 (d, *J* = 0.8 Hz, 1H), 7.97 (d, *J* = 8.8 Hz, 1H), 7.54 (d, *J* = 8.0 Hz, 1H), 4.48 (dt, *J* = 7.6, 1.6 Hz, 1H), 7.17 (dd, *J* = 7.6, 5.6 Hz, 1H), 6.86 (dd, *J* = 8.8, 1.6 Hz, 1H), 6.73 (d, *J* = 8.8 Hz, 1H), 6.60 (d, *J* = 10.0 Hz, 1H), 5.74 (d, *J* = 10.0 Hz, 1H), 4.16 (t, *J* = 7.2 Hz, 2H), 3.90 (s, 3H), 3.22 (t, *J* = 7.6 Hz, 2H), 2.54 (s, 3H), 1.47 (s, 6H). ESI-MS [M+H]⁺ 469.

### Example 2.6. Preparation of 5-methoxy-2,2-dimethyl-N-(3-methyl-1-(2-(pyridine-4-yl)ethyl)-1H-indazole-6-yl)-2H-chromene-6-carboxamide (formula 1-6, 6f)

### Example 2.6.1. Preparation of 3-methyl-6-nitro-1-(2-(pyridine-4-yl)ethyl)-1H-indazole (3f)

Using procedure A-method II, compound **3f** was prepared from compound **1** and 2-(pyridine-4-yl)ethanol as a yellow solid at a 67% yield. ¹H NMR (400MHz, CDCl₃) *δ* 8.44 (dd, *J* = 4.8, 1.6Hz, 2H), 8.08 (d, *J* = 1.6 Hz, 1H), 7.93 (dd, *J* = 8.4, 1.6 Hz, 1H), 7.71 (d, *J* = 8.8 Hz, 1H), 7.02 (d, *J* = 6.0 Hz, 2H), 4.61 (t, *J* = 7.2 Hz, 2H), 3.24 (t, *J* = 6.8 Hz, 2H), 2.61 (s, 3H).

### Example 2.6.2. Preparation of 3-methyl-1-(2-(pyridine-4-yl)ethyl)-1H-indazole-6-amine (5f)

Using procedure B, compound **5f** was prepared from compound **3f** as a red solid at a 90% yield. ¹H NMR (400MHz, CDCl₃) δ 8.45 (dd, *J* = 4.8, 2.0 Hz, 2H), 7.38 (d, *J* = 8.4 Hz, 1H), 7.05 (d, *J* = 6.0 Hz, 2H), 6.50 (dd, *J* = 8.8, 2.0 Hz, 1H), 6.28 (d, *J* = 1.2 Hz, 1H), 4.37 (t, *J* = 7.2 Hz, 2H), 3.79 (s, 2H), 3.14 (t, *J* = 7.6 Hz, 2H), 2.48 (s, 3H).

### Example 2.6.3. Preparation of 5-methoxy-2,2-dimethyl-N-(3-methyl-1-(2-(pyridine-4-yl)ethyl)-1H-indazole-6-yl)-2H-chromene-6-carboxamide (6f)

Using procedure C-method I, compound **6f** was prepared from compound **5f** and compound **29** prepared according to Example 2.1.3 as a white solid at a 89% yield. mp 150-151°C. ¹H NMR (400MHz, CDCl₃) *δ* 9.91 (s, 1H), 8.44 (d, *J* = 4.8 Hz, 2H), 7.97 (d, *J* = 8.8 Hz, 1H), 7.54 (d, *J* = 8.4 Hz, 1H), 7.09 (d, *J* = 5.6 Hz, 2H), 6.83 (dd, *J* = 8.8, 2.0 Hz, 1H), 6.73 (d, *J* = 8.0 Hz, 1H), 6.60 (d, *J* = 10.0 Hz, 1H), 5.73 (d, *J* = 10.0 Hz, 1H), 4.54 (t, *J* = 7.6 Hz, 2H), 3.90 (s, 3H), 3.21 (t, *J* = 8.0 Hz, 2H), 2.54 (s, 3H), 1.47 (s, 6H). ESI-MS [M+H]⁺ 469.

### Example 2.7. Preparation of 5-methoxy-2,2-dimethyl-N-(3-methyl-1-(2-(1-methylpiperidine-4-yl)ethyl)-1H-indazole-6-yl)-2H-chromene-6-carboxamide (formula 1-7, 11k)

### Example 2.7.1. Preparation of tert-butyl 4-(2-(3-methyl-6-nitro-1H-indazole-1-yl)ethyl)piperidine-1-carboxylate (3g)

Using procedure A-method I, compound **3g** was prepared from compound **1** and *tert*-butyl 4-(2-bromoethyl)piperidine-1-carboxylate as a yellow solid at a 63% yield. ¹H NMR (400 MHz, DMSO-*d*₆) δ 8.65 (dd, *J* = 1.9, 0.7 Hz, 1H), 7.92 (dd, *J* = 8.9, 0.7 Hz, 1H), 7.86 (dd, *J* = 8.8, 1.9 Hz, 1H), 4.47 (t, *J* = 7.2 Hz, 2H), 3.92 - 3.81 (m, 2H), 2.67 - 2.53 (m, 2H), 2.51 (s, 3H), 1.73 (q, *J* = 7.1 Hz, 2H), 1.68 - 1.62 (m, 2H), 1.44 - 1.36 (m, 1H), 1.34 (s, 9H), 1.01 (qd, *J* = 12.2, 4.2 Hz, 2H).

### Example 2.7.2. Preparation of 3-methyl-1-(2-(1-methylpiperidin-4-yl)ethyl)-6-nitro-1H-indazole (7k)

Using procedure D and procedure E-method I, compound 7k was prepared from compound 3g as a yellow solid at an 84% yield. ¹H NMR (400MHz, CDCl₃) *δ* 8.27 (d, *J* = 1.6 Hz, 1H), 7.95 (dd, *J* = 9.2, 2.0 Hz, 1H), 7.72 (d, *J* = 8.8 Hz, 1H), 4.39 (t, *J* = 7.2 Hz, 2H), 2.83 (d, *J* = 11.6 Hz, 2H), 2.59 (s, 3H), 2.46 (s, 3H), 2.24 (s, 3H), 1.89-1.84 (m, 4H), 1.73 (d, *J* = 12.8 Hz, 2H), 1.35 (qd, *J* = 12.4, 3.6 Hz, 2H), 1.29-1.21 (m, 1H).

### Example 2.7.3. Preparation of 3-methyl-1-(2-(1-methylpiperidin-4-yl)ethyl)-1H-indazole-6-amine (9k)

Using procedure B, compound **9k** was prepared from compound **7k** as a brown solid at a 98% yield. ¹H NMR (400MHz, CDCl₃) *δ* 7.37 (d, *J* = 8.8 Hz, 1H), 6.50 (dd, *J* = 8.0, 2.0 Hz, 1H), 6.43 (d, *J* = 2.0 Hz, 1H), 4.16 (t, *J* = 7.2 Hz, 2H), 3.83 (s, 2H), 2.79 (d, *J* = 11.6 Hz, 2H), 2.46 (s, 3H), 2.22 (s, 3H), 1.88-1.70 (m, 6H), 1.36-1.20 (m, 3H).

### Example 2.7.4. Preparation of 5-methoxy-2,2-dimethyl-N-(3-methyl-1-(2-(1-methylpiperidin-4-yl)ethyl)-1H-indazole-6-yl)-2H-chromene-6-carboxamide (11k)

Using procedure C-method I, compound **11k** was prepared from compound **9k** and compound **29** prepared according to Example 2.1.3 as a white solid at an 83% yield. mp 103-104°C. ¹H NMR (400MHz, CDCl₃) *δ* 9.44 (s, 1H), 8.33 (d, *J* = 1.2 Hz, 1H), 7.98 (d, *J* = 8.8 Hz, 1H), 7.54 (d, *J* = 8.8 Hz, 1H), 6.85 (dd, *J* = 8.4, 1.6 Hz, 1H), 6.73 (d, *J* = 8.8 Hz, 1H), 6.60 (d, *J* = 9.6 Hz, 1H), 5.73 (d, *J* = 10.0 Hz, 1H), 4.32 (t, *J* = 7.6 Hz, 2H), 3.90 (s, 3H), 2.90 (d, *J* = 9.6 Hz, 2H), 2.53 (s, 3H), 2.29 (s, 3H), 1.98 (t, *J* = 11.2 Hz, 2H), 1.86 (q, *J=* 7.2 Hz, 2H), 1.79 (d, *J* = 12.8 Hz, 2H), 1.46 (s, 6H), 1.46-1.32 (m, 3H). ESI-MS [M+H]⁺ 491.

### Example 2.8. Preparation of 5-methoxy-2,2-dimethyl-N-(3-methyl-1-(2-(4-methylpiperazine-1-yl)ethyl)-1H-indazole-6-yl)-2H-chromene-6-carboxamide (formula 1-8, 111)

### Example 2.8.1. Preparation of 1-(2-bromoethyl)-3-methyl-6-nitro-1H-indazole (3i)

Using procedure A-method III, compound 3i was prepared from compound 1 and 1,2-dibromoethane as a colorless oil at a 49% yield. ¹H NMR (400 MHz, CDCl₃) δ 8.39 (d, *J* = 1.6 Hz, 1H), 7.95 (dd, *J* = 8.8, 2.0 Hz, 1H), 7.70 (d, *J* = 8.8 Hz, 1H), 4.72 (t, *J* = 6.4 Hz, 2H), 3.75 (t, *J* = 6.4 Hz, 2H), 2.59 (s, 3H).

### Example 2.8.2. Preparation of 3-methyl-1-(2-(4-methylpiperazin-1-yl)ethyl)-6-nitro-1H-indazole (71)

Using procedure G, compound **7l** was prepared from compound **3i** as a yellowish solid at a 97% yield. ¹H NMR (400MHz, CDCl₃) δ 8.39 (d, *J* = 1.6 H, 1H), 7.95 (dd, *J* = 8.4, 1.6 Hz, 1H), 7.70 (d, *J* = 9.2 Hz, 1H), 4.48 (t, *J* = 6.8 Hz, 2H), 2.83 (t, *J* = 6.4 Hz, 2H), 2.59 (s, 3H), 2.54 (brs, 4H), 2.40 (brs, 4H), 2.25 (s, 3H).

### Example 2.8.3. Preparation of 3-methyl-1-(2-(4-methylpiperazin-1-yl)ethyl)-1H-indazole-6-amine (91)

Using procedure B, compound **9l** was prepared from compound **7l** as a brown solid at a 94% yield. ¹H NMR (400MHz, CDCl₃) *δ* 7.37 (d, *J* = 8.0 Hz, 1H), 6.52-6.48 (m, 2H), 4.29 (t, *J* = 7.60 Hz, 2H), 3.83 (s, 2H), 2.80 (t, *J* = 6.0 Hz, 2H), 2.57 (br, 4H), 2.47 (s, 3H), 2.43 (br, 4H), 2.27 (s, 3H).

### Example 2.8.4. Preparation of 5-methoxy-2,2-dimethyl-N-(3-methyl-1-(2-(4-methylpiperazin-1-yl)ethyl)-1H-indazole-6-yl)-2H-chromene-6-carboxamide (111)

Using procedure C-method I, compound **11l** was prepared from compound **9l** and compound **29** prepared according to Example 2.1.3 as a white solid at a 78% yield. mp 90-91°C. ¹H NMR (400MHz, CDCl₃) *δ* 9.93 (s, 1H), 8.36 (s, 1H), 7.96 (d, *J* = 4.8 Hz, 1H), 7.53 (d, *J* = 8.4 Hz, 1H), 6.85 (dd, *J* = 8.4, 1.2 Hz, 1H), 6.73 (d, *J* = 8.8 Hz, 1H), 6.60 (d, *J* = 10.4 Hz, 1H), 5.73 (d, *J* = 10.0 Hz, 1H), 4.16 (t, *J* = 6.8 Hz, 2H), 2.66 (br, 8H), 2.52 (s, 3H), 2.36 (s, 3H), 1.46 (s, 6H). ESI-MS [M+H]⁺ 490.

### Example 2.9. Preparation of 5-methoxy-2,2-dimethyl-N-(3-methyl-1-(3-(1-methylpiperidine-4-yl)propyl)-1H-isoindole-6-yl)-2H-chromene-6-carboxamide (formula 1-9, 11m)

### Example 2.9.1. Preparation of tert-butyl 4-(3-(3-methyl-6-nitro-1H-indazole-1-yl)propyl)piperidine-1-carboxylate (3h)

Using procedure A-method I, compound **3h** was prepared from compound **1** and *tert-*butyl 4-(3-bromopropyl)piperidine-1-carboxylate as a yellow solid at a 67% yield. ¹H NMR (400 MHz, CDCl₃) δ 8.28 (d, *J* = 1.8 Hz, 1H), 7.96 (dd, *J* = 8.8, 1.9 Hz, 1H), 7.73 (d, *J* = 8.9 Hz, 1H), 4.36 (t, *J* = 7.2 Hz, 2H), 4.04 (d, *J* = 13.1 Hz, 2H), 2.66 - 2.59 (m, 5H), 1.99 - 1.90 (m, 2H), 1.63 - 1.60 (m, 2H), 1.42 (s, 9H), 1.40 - 1.33 (m, 1H), 1.29 - 1.23 (m, 2H), 1.10 - 0.98 (m, 2H).

### Example 2.9.2. Preparation of 3-methyl-1-(3-(1-methylpiperidin-4-yl)propyl)-6-nitro-1H-indazole (7m)

Using procedure D and procedure E-method I, compound **7m** was prepared from compound **3h** as a yellow solid at an 84% yield. ¹H NMR (400 MHz, MeOD) δ 8.48 (dd, *J* = 1.9, 0.7 Hz, 1H), 7.93 (dd, *J* = 8.8, 1.9 Hz, 1H), 7.86 (dd, *J* = 8.9, 0.7 Hz, 1H), 4.42 (t, *J* = 6.9 Hz, 2H), 2.86 - 2.79 (m, 2H), 2.57 (s, 3H), 2.23 (s, 3H), 2.03 - 1.94 (m, 2H), 1.94 - 1.86 (m, 2H), 1.68 - 1.62 (m, 2H), 1.24 - 1.09 (m, 5H).

### Example 2.9.3. Preparation of 3-methyl-1-(3-(1-methylpiperidin-4-yl)propyl)-1H-indazole-6-amine (9m)

Using procedure B, compound **9m** was prepared from compound **7m** as a light red solid at a 91% yield. ¹H NMR (400 MHz, DMSO-*d*₆) δ 7.24 (d, *J* = 8.5 Hz, 1H), 6.39 (dd, *J* = 8.5, 1.7 Hz, 1H), 6.35 (d, *J* = 1.7 Hz, 1H), 5.21 (s, 2H), 3.98 (t, *J* = 7.0 Hz, 2H), 2.64 (dt, *J* = 11.3, 3.2 Hz, 2H), 2.27 (s, 3H), 2.05 (s, 3H), 1.76 - 1.65 (m, 4H), 1.51 (d, *J* = 11.6 Hz, 2H), 1. 17 - 0.96 (m, 5H).

### Example 2.9.4. Preparation of 5-methoxy-2,2-dimethyl-N-(3-methyl-1-(3-(1-methylpiperidin-4-yl)propyl)-1H-isoindole-6-yl)-2H-chromene-6-carboxamide (11m)

Using procedure C-method I, compound **11m** was prepared from compound **9m** and compound **29** prepared according to Example 2.1.3 as a white solid at an 89% yield. mp 62-63°C. ¹H NMR (400 MHz, CDCl₃) δ 9.95 (s, 1H), 8.35 (d, *J* = 1.7 Hz, 1H), 7.97 (d, *J* = 8.7 Hz, 1H), 7.55 (d, *J* = 8.5 Hz, 1H), 6.84 (dd, *J* = 8.6, 1.7 Hz, 1H), 6.73 (d, *J* = 8.6 Hz, 1H), 6.60 (d, *J* = 10.0 Hz, 1H), 5.74 (d, *J* = 10.0 Hz, 1H), 4.27 (t, *J* = 7.2 Hz, 2H), 3.91 (s, 3H), 3.05 - 2.84 (m, 2H), 2.53 (s, 3H), 2.35 (s, 3H), 2.16 - 2.02 (m, 2H), 1.91 (p, *J* = 7.3 Hz, 2H), 1.73 - 1.67 (m, 2H), 1.47 (s, 6H), 1.42 - 1.25 (m, 5H). FAB-MS [M+H]⁺ 503.

### Example 2.10. Preparation of 5-methoxy-2,2-dimethyl-N-(3-methyl-1-(3-(4-methylpiperazine-1-yl)propyl)-1H-indazole-6-yl)-2H-chromene-6-carboxamide (formula 1-10, 11n)

### Example 2.10.1. Preparation of 1-(3-bromopropyl)-3-methyl-6-nitro-1H-indazole (3j)

Using procedure A-method III, compound **3j** was prepared from compound 1 and 1,3-dibromopropane as a colorless oil at a 45% yield. ¹H NMR (400 MHz, CDCl₃) δ 8.40 (d, *J* = 1.6 Hz, 1H), 7.97 (dd, *J* = 8.8, 1.8 Hz, 1H), 7.73 (d, *J* = 8.8 Hz, 1H), 4.55 (t, *J* = 6.4 Hz, 2H), 3.34 (t, *J* = 6.2 Hz, 2H), 2.60 (s, 3H), 2.50 (p, *J* = 6.3 Hz, 2H).

### Example 2.10.2. Preparation of 3-methyl-1-(3-(4-methylpiperazin-1-yl)propyl)-6-nitro-1H-indazole (7n)

Using procedure G, compound **7n** was prepared from compound **3j** as a colorless oil at a 91% yield. ¹H NMR (400 MHz, CDCl₃) δ 8.44 (d, *J* = 1.9 Hz, 1H), 7.94 (dd, *J* = 8.8, 1.9 Hz, 1H), 7.70 (d, *J* = 8.9 Hz, 1H), 4.46 (t, *J* = 6.3 Hz, 2H), 2.59 (s, 3H), 2.70 - 2.25 (m, 8H), 2.32 (s, 3H), 2.21 (t, *J* = 6.5 Hz, 2H), 2.08 (p, *J* = 6.4 Hz, 3H).

### Example 2.10.3. Preparation of 3-methyl-1-(3-(4-methylpiperazin-1-yl)propyl)-1H-indazol-6-amine (9n)

Using procedure B, compound **9n** was prepared from compound **7n** as a light red solid at a 96% yield. ¹H NMR (400 MHz, DMSO-*d*₆) δ 7.24 (d, *J* = 8.5 Hz, 1H), 6.40 (dd, *J* = 8.5, 1.7 Hz, 1H), 6.35 (d, *J* = 1.3 Hz, 1H), 5.20 (s, 2H), 4.03 (t, *J* = 6.9 Hz, 2H), 2.45 - 2.12 (m, 11H), 2.15 (t, *J* = 7.0 Hz, 2H), 2.10 (s, 3H), 1.82 (p, *J* = 6.9 Hz, 2H).

### Example 2.10.4. Preparation of 5-methoxy-2,2-dimethyl-N-(3-methyl-1-(3-(4-methylpiperazin-1-yl)propyl)-1H-indazol-6-yl)-2H-chromene-6-carboxamide (11n)

Using procedure C-method I, compound **11n** was prepared from compound **9n** and compound **29** prepared according to Example 2.1.3 as a white solid at a 57% yield. mp 88-89°C. ¹H NMR (400 MHz, DMSO-*d*₆) δ 10.21 (s, 1H), 8.16 (s, 1H), 7.57 (d, *J* = 8.6 Hz, 1H), 7.40 (d, *J* = 8.5 Hz, 1H), 7.17 (dd, *J* = 8.6, 1.7 Hz, 1H), 6.64 (d, *J* = 8.5 Hz, 1H), 6.58 (d, *J* = 10.0 Hz, 1H), 5.84 (d, *J* = 10.0 Hz, 1H), 4.22 (t, *J* = 6.7 Hz, 2H), 3.76 (s, 3H), 2.40 (s, 3H), 2.50 - 2.02 (m, 8H), 2.15 (t, *J* = 6.8 Hz, 2H), 2.07 (s, 3H), 1.89 (p, *J* = 6.9 Hz, 2H), 1.38 (s, 6H). FAB-MS [M+H]⁺ 504.

### Example 2.11. Preparation of N-(1-(2-(1-(but-2-inoyl)piperidin-4-yl)ethyl)-3-methyl-1H-indazole-6-yl)-5-methoxy-2,2-dimethyl-2H-chromene-6-carboxamide (formula 1-11, 11o)

### Example 2.11.1. Preparation of tert-butyl 4-(2-(6-amino-3-methyl-1H-indazole-1-yl)ethyl)piperidine-1-carboxylate (5g)

Using procedure B, compound **5g** was prepared from compound **3g** prepared according to Example 2.7.1 as a white solid at a 99% yield. ¹H NMR (400 MHz, CDCl₃) δ 7.40 (d, *J* = 8.6 Hz, 1H), 6.56 (dd, *J* = 8.5, 1.8 Hz, 1H), 6.50 (d, *J* = 1.8 Hz, 1H), 4.19 (t, *J* = 7.4 Hz, 2H), 4.13 - 3.98 (m, 2H), 2.70 - 2.61 (m, 2H), 2.47 (s, 3H), 1.79 (q, *J* = 7.2 Hz, 2H), 1.73 - 1.66 (m, 2H), 1.45 - 1.37 (m, 10H), 1.20 - 1.09 (m, 2H).

### Example 2.11.2. Preparation of tert-butyl 4-(2-(6-(5-methoxy-2,2-dimethyl-2H-chromene-6-carboxamido)-3-methyl-1H-indazol-1-yl)ethyl)piperidine-1-carboxylate (6g)

Using procedure C-method I, compound **6g** was prepared from compound **5g** as a white solid at a 53% yield. ¹H NMR (400 MHz, CDCl₃) δ 10.00 (s, 1H), 8.41 (s, 1H), 7.98 (d, *J* = 8.6 Hz, 1H), 7.59 (d, *J* = 8.6 Hz, 1H), 6.88 (dd, *J* = 8.5, 1.7 Hz, 1H), 6.74 (d, *J* = 8.7 Hz, 1H), 6.60 (d, *J* = 9.9 Hz, 1H), 5.74 (d, *J* = 10.3 Hz, 1H), 4.41 (t, *J* = 7.5 Hz, 2H), 4.10 - 4.02 (m, 2H), 3.91 (s, 3H), 2.71 - 2.62 (m, 2H), 2.59 (s, 3H), 1.88 (q, *J* = 7.0 Hz, 2H), 1.78 - 1.70 (m, 2H), 1.47 (s, 6H), 1.43 (s, 10H), 1.22 - 1.12 (m, 2H).

### Example 2.11.3. Preparation of N-(1-(2-(1-(but-2-ynoyl)piperidin-4-yl)ethyl)-3-methyl-1H-indazole-6-yl)-5-methoxy-2,2-dimethyl-2H-chromene-6-carboxamide (11o)

Using procedure D and procedure F-method I, compound **11o** was prepared from compound **6g** as a white solid at a 26% yield. mp 71-72°C. ¹H NMR (400 MHz, CDCl₃) δ 9.96 (s, 1H), 8.40 (s, 1H), 7.98 (d, *J* = 8.6 Hz, 1H), 7.56 (d, *J* = 8.5 Hz, 1H), 6.84 (dd, *J* = 8.6, 1.6 Hz, 1H), 6.74 (d, *J* = 8.7 Hz, 1H), 6.60 (d, *J* = 10.0 Hz, 1H), 5.74 (d, *J* = 10.0 Hz, 1H), 4.56 - 4.45 (m, 1H), 4.41 - 4.29 (m, 3H), 3.91 (s, 3H), 3.04 - 2.95 (m, 1H), 2.63 - 2.56 (m, 1H), 2.55 (s, 3H), 1.99 (s, 3H), 1.94 - 1.71 (m, 4H), 1.63 - 1.51 (m, 1H), 1.47 (s, 6H), 1.24 - 1.10 (m, 2H). FAB-MS [M+H]⁺ 541.

### Example 2.12. Preparation of N-(1-(2-(1-acryloylpiperidine-4-yl)ethyl)-3-methyl-1H-indazole-6-yl)-5-methoxy-2,2-dimethyl-2H-chromene-6-carboxamide (formula 1-12, 11p)

Using procedure D and F-method II, compound **11p** was prepared from compound **6g** prepared according to Example 2.11.2 as a white solid at a 65% yield. mp 69-70°C. ¹H NMR (400 MHz, CDCl₃) δ 9.98 (s, 1H), 8.40 (d, *J* = 1.3 Hz, 1H), 7.98 (d, *J* = 8.7 Hz, 1H), 7.56 (d, *J* = 8.5 Hz, 1H), 6.84 (dd, *J* = 8.6, 1.7 Hz, 1H), 6.74 (dd, *J* = 8.7, 0.8 Hz, 1H), 6.64 - 6.52 (m, 2H), 6.23 (dd, *J* = 16.9, 2.0 Hz, 1H), 5.74 (d, *J* = 10.0 Hz, 1H), 5.64 (dd, *J* = 10.6, 2.0 Hz, 1H), 4.67 - 4.56 (m, 1H), 4.37 (t, *J* = 7.3 Hz, 2H), 4.01 - 3.94 (m, 1H), 3.91 (s, 3H), 3.07 - 2.94 (m, 1H), 2.64 - 2.56 (m, 1H), 2.55 (s, 3H), 1.90 - 1.80 (m, 4H), 1.58 - 1.53 (m, 1H), 1.47 (s, 6H), 1.23 - 1.15 (m, 2H). FAB-MS [M+H]⁺ 529.

### Example 2.13. Preparation of 5-methoxy-2,2-dimethyl-N-(1-(2-(1-methylpiperidine-4-yl)ethyl)-1H-indazole-6-yl)-2H-chromene-6-carboxamide (formula 1-13, 12k)

### Example 2.13.1. Preparation of tert-butyl 4-(2-(6-nitro-1H-indazole-1-yl)ethyl)piperidine-1-carboxylate (4g)

Using procedure A-method I, compound **4g** was prepared from compound **2** and *tert*-butyl 4-(2-bromoethyl)piperidine-1-carboxylate as a yellow solid at a 47% yield. ¹H NMR (400 MHz, CDCl₃) δ 8.35 (s, 1H), 8.10 (s, 1H), 8.01 (dd, J = 8.9, 2.0 Hz, 1H), 7.83 (dd, J = 8.8, 1.4 Hz, 1H), 4.50 (t, J = 6.4 Hz, 2H), 4.14 - 4.01 (m, 2H), 2.73 - 2.56 (m, 2H), 1.91 (q, J = 7.0, 6.6 Hz, 2H), 1.47 - 1.41 (m, 10H), 1.79 - 1.68 (m, 2H), 1.27 - 1.12 (m, 2H).

### Example 2.13.2. Preparation of 1-(2-(1-methylpiperidin-4-yl)ethyl)-6-nitro-1H-indazole (8k)

Using procedure D and procedure E-method I, compound **8k** was prepared from compound **4g** as a yellow solid at a 58% yield. ¹H NMR (400 MHz, CDCl₃) δ 8.36 - 8.35 (m, 1H), 8.10 (d, *J* = 1.0 Hz, 1H), 8.01 (dd, *J* = 8.8, 1.9 Hz, 1H), 7.83 (dd, *J* = 8.9, 0.7 Hz, 1H), 4.50 (t, *J* = 7.2 Hz, 2H), 3.03 - 2.85 (m, 2H), 2.35 (s, 3H), 2.08 - 2.02 (m, 2H), 1.94 (q, *J* = 7.0 Hz, 2H), 1.83 - 1.75 (m, 2H), 1.58 - 1.47 (m, 2H), 1.35 - 1.25 (m, 1H).

### Example 2.13.3. Preparation of 1-(2-(1-methylpiperidin-4-yl)ethyl)-1H-indazole-6-amine (10k)

Using procedure B, compound **10k** was prepared from compound **8k** as a light red solid at a 92% yield. ¹H NMR (401 MHz, CDCl₃) δ 7.77 (d, *J* = 0.9 Hz, 1H), 7.46 (dd, *J* = 8.6, 0.7 Hz, 1H), 6.55 (dd, *J* = 8.5, 1.8 Hz, 1H), 6.51 (dt, *J* = 1.8, 0.9 Hz, 1H), 4.25 (t, *J* = 7.2 Hz, 2H), 3.86 (s, 2H), 2.94 - 2.84 (m, 2H), 2.29 (s, 3H), 1.99 - 1.93 (m, 2H), 1.83 (q, *J* = 6.9 Hz, 2H), 1.79 - 1.71 (m, 2H), 1.48 - 1.36 (m, 2H), 1.32 - 1.21 (m, 1H).

### Example 2.13.4. Preparation of 5-methoxy-2,2-dimethyl-N-(1-(2-(1-methylpiperidin-4-yl)ethyl)-1H-indazole-6-yl)-2H-chromene-6-carboxamide (12k)

Using procedure C-method I, compound **12k** was prepared from compound **10k** and compound **29** prepared according to Example 2.1.3 as a white solid at a 52% yield. mp 78-79°C. ¹H NMR (400 MHz, CDCl₃) δ 9.96 (s, 1H), 8.43 (s, 1H), 7.98 (d, *J* = 8.7 Hz, 1H), 7.90 (d, *J* = 0.9 Hz, 1H), 7.63 (d, *J* = 8.5 Hz, 1H), 6.88 (dd, *J* = 8.6, 1.7 Hz, 1H), 6.74 (d, *J* = 8.7 Hz, 1H), 6.60 (d, *J* = 10.0 Hz, 1H), 5.73 (d, *J* = 10.0 Hz, 1H), 4.41 (t, *J* = 7.3 Hz, 2H), 3.90 (s, 3H), 2.90 - 2.84 (m, 2H), 2.27 (s, 3H), 1.97 - 1.91 (m, 2H), 1.91 - 1.86 (m, 2H), 1.81 - 1.75 (m, 2H), 1.47 (s, 6H), 1.43 - 1.33 (m, 2H), 1.33 - 1.25 (m, 1H). FAB-MS [M+H]⁺ 475.

### Example 3. Preparation of 3-aminoindazole derivatives

A method for producing 3-aminoindazole derivatives is shown by the following scheme 2. (a) N₂H₄·H₂O, *t*-BuOH, reflux, 18 hours; (b) phthalic anhydride, 1,4-dioxane, reflux, 15 hours; (c) Alkyl halide, Cs₂CO₃, DMF, 60°C, 4 hours; (d) N₂H₄·H₂O, EtOH, room temperature, 15 hours; (e) For **17k,** 4-vinylpyridine, K₃PO₄, H₂O, microwave 180°C, 20 minutes; (f) i) **29,** (COCl)₂, DMF, THF, room temperature, 2 hours; ii) amine, TEA, DCM, room temperature, 1 hour or -20°C, 4 hours; (g) ACE-Cl, DCM, -5°C, 2 hours; (h) NaH, THF, CH₃I, from 0°C to room temperature, 4 hours.

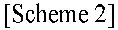

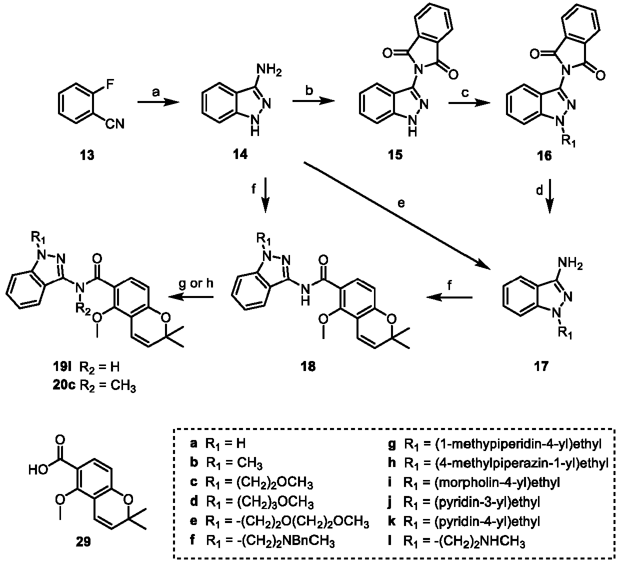

### Example 3.1. Preparation of N-(1H-indazole-3-yl)-5-methoxy-2,2-dimethyl-2H-chromene-6-carboxamide (formula 1-14, 18a)

### Example 3.1.1. Preparation of 1H-indazole-3-amine (14)

A mixture of 2-fluoroaniline (1.0 eq) and hydrazine monohydrate (1.05 eq) in *t-*BuOH was heated to reflux overnight. After the reaction was completed, *t*-BuOH was removed under low pressure. Water was added to the residue, and then the solution was filtered, and washed with water. The filtrate was collected and extracted twice with ethyl acetate. The organic layer was washed with water, dried over MgSO₄ and concentrated. The solids were combined and stored for the next step (86%). ¹H NMR (400 MHz, CD₃OD) *δ* 7.56 (d, *J* = 7.04 Hz, 1H), 7.36-7.26 (m, 2H), 6.98 (ddd, *J* = 7.92, 5.84, 1.88 H, 1H).

### Example 3.1.2. Preparation of 2-(1H-indazole-3-yl)isoindoline-1,3-dione (15)

To a solution of the compound **14** in 1,4-dioxane was added a stoichiometric amount of phthalimide. The mixture was heated to reflux overnight. Then, the mixture was evaporated under vacuum, and the residual solids were collected. The solids were washed with hexane, and dried to obtain off-white solids (yield 89%). ¹H NMR (500 MHz, CDCl₃) *δ* 8.05-7.99 (m, 2H), 7.83-7.80 (m, 2H), 7.55 (d, *J* = 8.2 Hz, 1H), 7.48-7.44 (m, 2H), 7.21-7.18 (m, 1H).

### Example 3.1.3. Preparation of N-(1H-indazole-3-yl)-5-methoxy-2,2-dimethyl-2H-chromene-6-carboxamide (18a)

Using procedure C-method I, compound **18a** was prepared from compound **15** and compound **29** prepared according to Example 2.1.3 as a white solid at an 86% yield. mp 222-223°C. ¹H NMR (400 MHz, CDCl₃) *δ* 10.31 (s, 1H), 8.08 (d, *J* = 8.28 Hz, 1H), 8.01 (d, *J* = 8.72 Hz, 1H), 7.48 (d, *J* = 8.44 Hz, 1H), 7.42 (t, *J* = 7.88 Hz, 1H), 7.18 (t, *J* = 7.64 Hz, 1H), 6.71 (d, *J* = 8.68 Hz, 1H), 6.60 (d, *J* = 10.0 Hz, 1H), 5.71 (d, *J* -= 10.0 Hz, 1H), 3.91 (s, 3H), 1.47 (s, 6H). ESI-MS [M+H]⁺ 350.

### Example 3.2. Preparation of 5-methoxy-N,2,2-trimethyl-N-(1-methyl-1H-indazole-3-yl)-2H-chromene-6-carboxamide (formula 1-15, 18b)

### Example 3.2.1. Preparation of 2-(1-methyl-1H-indazole-3-yl)isoindoline-1,3-dione (16b)

Using procedure A-method I, compound **16b** was prepared from compound **15** prepared according to Example 3.1.2 and iodomethane as a white solid at a 51% yield. ¹H NMR (500 MHz, CDCl₃) *δ* 8.00-7.98 (m, 2H), 7.82-7.80 (m, 2H), 7.54 (d, *J* = 8.2 Hz, 1H), 7.44-7.43 (m, 2H), 7.20-7.17 (m, 1H), 4.13 (s, 3H).

### Example 3.2.2. Preparation of 1-methyl-1H- indazole-3-amine (17b)

Using procedure N, compound **17b** was prepared from compound **16b** as a brown solid at an 87% yield. ¹H NMR (400MHz, CDCl₃) *δ* 7.50 (d, *J* = 8.4, 1H), 7.34-7.27 (m, 2H), 7.00 (m, 1H), 4.07 (brs, 2H), 3.91 (s, 3H).

### Example 3.2.3. Preparation of 5-methoxy-N,2,2-trimethyl-N-(1-methyl-1H-indazole-3-yl)-2H-chromene-6-carboxamide (18b)

Using procedure C-method I, compound **18b** was prepared from compound **17b** and compound **29** prepared according to Example 2.1.3 as a white solid at a 92% yield. mp 103-104°C. ¹H NMR (400MHz, CDCl₃) *δ* 10.10 (s, 1H), 8.07 (d, *J* = 8.25 Hz, 1H), 8.03 (d, *J* = 8.65 Hz, 1H), 7.38 (t, *J* = 7.85 Hz, 1H), 7.29 (d, *J* = 8.55 Hz, 1H), 7.13 (t, *J* = 7.35 Hz, 1H), 6.72 (d, *J* = 8.70 Hz, 1H), 6.60 (d, *J* = 10.05 Hz, 1H), 5.71 (d, *J* = 10.0 Hz, 1H), 3.99 (s, 3H), 3.94 (s, 3H), 1.46 (s, 6H). ESI-MS [M+H]⁺ 364.

### Example 3.3. Preparation of 5-methoxy-N-(1-(2-methoxyethyl)-1H-indazole-3-yl)-2,2-dimethyl-2H-chromene-6-carboxamide (formula 1-16, 18c)

### Example 3.3.1. Preparation of 2-(1-(2-methoxyethyl)-1H-indazole-3-yl)isoindoline-1,3-dione (16c)

Using procedure A-method I, compound **16c** was prepared from compound **15** prepared according to Example 3.1.2 and 2-bromoethyl methyl ether as a white solid at a 67% yield. ¹H NMR (400MHz, CDCl₃) *δ* 8.03-7.98 (m, 2H), 7.84-7.80 (m, 2H), 7.53 (dd, *J* = 9.2, 0.8 Hz, 2H), 7.42 (td, *J* = 7.6, 1.2 Hz, 1H), 7.18 (td, *J* = 8.0, 0.8 Hz, 1H), 4.60 (t, *J* = 5.6 Hz, 2H), 3.87 (t, *J* = 5.6 Hz, 2H), 3.31 (s, 3H).

### Example 3.3.2. Preparation of 1-(2-methoxyethyl)-1H-indazole-3-amine (17c)

Using procedure N, compound **17c** was prepared from compound **16c** as a brown solid in 75% yield. ¹H NMR (400MHz, CDCl₃) *δ* 7.51 (dt, *J* = 8.4, 0.8 Hz, 1H), 7.35-7.26 (m, 2H), 7.00 (ddd, *J* = 8.0, 6.4, 0.8 Hz, 1H), 4.31 (t, *J* = 5.2 Hz, 2H), 4.03 (brs, 2H), 3.75 (t, *J* = 5.6 Hz, 2H), 3.29 (s, 3H).

### Example 3.3.3. Preparation of 5-methoxy-N-(1-(2-methoxyethyl)-1H-indazole-3-yl)-2,2-dimethyl-2H-chromene-6-carboxamide (18c)

Using procedure C-method I, compound **18c** was prepared from compound **17c** and compound **29** prepared according to Example 2.1.3 as a white solid at a 76% yield. mp 92-93°C. ¹H NMR (400MHz, CDCl₃) *δ* 10.07 (s, 1H), 8.06 (d, *J* = 8.8 Hz, 1H), 8.03 (d, *J* = 8.4 Hz, 1H), 7.40-7.35 (m, 2H), 7.12 (ddd, *J* = 8.4, 6.0, 2.0 Hz, 1H), 6.72 (d, *J* = 8.4 Hz, 1H), 6.61 (d, *J* = 10.4 Hz, 1H), 5.72 (d, *J* = 10.0 Hz, 1H), 4.47 (t, *J* = 5.6 Hz, 2H), 3.95 (s, 3H), 3.81 (t, *J* = 5.6 Hz, 2H), 3.30 (s, 3H), 1.50 (s, 6H). ESI-MS [M+H]⁺ 408.

### Example 3.4. Preparation of 5-methoxy-N-(1-(3-methoxypropyl)-1H-indazole-3-yl)-2,2-dimethyl-2H-chromene-6-carboxamide (formula 1-17, 18d)

### Example 3.4.1. Preparation of 2-(1-(3-methoxypropyl)-1H-indazole-3-yl)isoindoline-1,3-dione (16d)

Using procedure A-method I, compound **16d** was prepared from compound **15** prepared according to Example 3.1.2 and 3-bromopropyl methyl ether as a white solid at a 42% yield. ¹H NMR (400MHz, CDCl₃) *δ* 7.99 (dd, *J* = 5.6, 2.8 Hz, 2H), 7.82 (dd, *J* = 5.6, 3,2 Hz, 2H), 7.54 (d, *J* = 8.0 Hz, 1H), 7.50 (d, *J* = 8.8 Hz, 1H), 7.44-7.40 (m, 1H), 7.20-7.16 (m, 1H), 4.55 (t, *J* = 7.2 Hz, 2H), 3.33 (t, *J* = 6.0 Hz, 2H), 3.31 (s, 3H), 2.21 (quintet, *J* = 6.0 Hz, 2H).

### Example 3.4.2. Preparation of 1-(3-methoxypropyl)-1H-indazole-3-amine (17d)

Using procedure N, compound **17d** was prepared from compound **16d** as a brown solid at a 71% yield. ¹H NMR (400MHz, CDCl₃) *δ* 7.49 (d, *J* = 8.4 Hz, 1H), 7.32-7.28 (m, 1H), 7.23 (d, *J* = 8.0 Hz, 1H), 6.98-6.95 (m, 1H), 4.22 (t, *J* = 6.4 Hz, 2H), 4.10 (brs, 2H), 3.24 (s, 3H), 3.24 (t, *J* = 6.0 Hz, 2H), 2.05 (quintet, *J* = 6.0 Hz, 2H).

### Example 3.4.3. Preparation of 5-methoxy-N-(1-(3-methoxypropyl)-1H-indazole-3-yl)-2,2-dimethyl-2H-chromene-6-carboxamide (18d)

Using procedure C-method I, compound **18d** was prepared from compound **17d** and compound **29** prepared according to Example 2.1.3 as a white solid at a 65% yield. mp 95-97°C. ¹H NMR (400MHz, CDCl₃) *δ* 10.09 (s, 1H), 8.07 (d, *J* = 8.4 Hz, 1H), 8.03 (d, *J* = 8.4 Hz, 1H), 7.37-7.36 (m, 2H), 7.15-7.09 (m, 1H), 7.72 (d, *J* = 8.8 Hz, 1H), 6.61 (d, *J* = 10.0 Hz, 1H), 5.71 (d, *J* = 10.0 Hz, 1H), 4.40 (t, *J* = 6.4 Hz, 2H), 3.94 (s, 3H), 3.29 (s, 3H), 3.29 (t, *J* = 5.6 Hz, 2H), 2.14 (quintet, *J* = 6.0 Hz, 2H), 1.47 (s, 6H). ESI-MS [M+H]⁺ 422.

### Example 3.5. Preparation of 5-methoxy-N-(1-(2-(2-methoxyethoxy)ethyl)-1H-indazole-3-yl)-2,2-dimethyl-2H-chromene-6-carboxamide (formula 1-18, 18e)

### Example 3.5.1. Preparation of 2-(1-(2-(2-methoxyethoxy)ethyl)-1H-indazole-3-yl)isoindoline-1,3-dione (16e)

Using procedure A-method I, compound **16e** was prepared from compound **15** prepared according to Example 3.1.2 and 1-bromo-2-(2-methoxyethoxy)ethane as a white solid at a 34% yield. ¹H NMR (400MHz, CDCl₃) *δ* 8.01-7.97 (m, 2H), 7.84-7.80 (m, 2H), 7.56 (d, *J* = 6.0 Hz, 1H), 7.52 (d, *J* = 8.0 Hz, 1H), 7.41 (t, *J* = 8.8 Hz, 1H), 7.17 (t, *J* = 7.6 Hz, 1H), 4.62 (t, *J* = 6.0 Hz, 2H), 3.97 (t, *J* = 6.0 Hz, 2H), 3.55- 3.52 (m, 2H), 3.43-3.41 (m, 2H), 3.29 (s, 3H).

### Example 3.5.2. Preparation of 1-(2-(2-methoxyethoxy)ethyl)-1H-indazole-3-amine (17e)

Using procedure N, compound **17e** was prepared from compound **16e** as a brown solid at a 67% yield. ¹H NMR (400MHz, CDCl₃) *δ* 7.51-7.49 (m, 1H), 7.34-7.29 (m, 2H), 7.01-7.92 (m, 1H), 4.33 (t, *J* = 6.0 Hz, 2H), 3.83 (t, *J* = 6.4 Hz, 2H), 3.51-3.49 (m, 2H), 3.42-3.38 (m, 2H), 3.29 (s, 3H).

### Example 3.5.3. Preparation of 5-methoxy-N-(1-(2-(2-methoxyethoxy)ethyl)-1H-indazole-3-yl)-2,2-dimethyl-2H-chromene-6-carboxamide (18e)

Using procedure C-method I, compound **18e** was prepared from compound **17e** and compound **29** prepared according to Example 2.1.3 as a white solid at a 64% yield. mp 104-105°C. ¹H NMR (400MHz, CDCl₃) *δ* 10.09 (s, 1H), 8.05 (d, *J* = 8.0 Hz, 1H), 8.03 (d, *J* = 8.4 Hz, 1H), 7.42 (d, *J* = 8.4 Hz, 1H), 7.36 (t, *J* = 6.3 Hz, 1H), 7.12 (t, *J* = 7.2 Hz, 1H), 6.72 (d, *J* = 8.4 Hz, 1H), 6.61 (d, *J* = 10.0 Hz, 1H), 5.72 (d, *J* = 10.4 Hz, 1H), 4.49 (t, *J* = 6.0 Hz, 2H), 3.95 (s, 3H), 3.91 (t, *J* = 6.0 Hz, 2H), 3.54-3.52 (m, 2H), 3.44-3.41 (m, 2H), 3.31 (s, 3H), 1.47 (s, 6H). ESI-MS [M+H]⁺ 452.

### Example 3.6. Preparation of 5-methoxy-2,2-dimethyl-N-(1-(2-(methylamino)ethyl)-1H-indazole-3-yl)-2H-chromene-6-carboxamide (formula 1-19, 19l)

Using procedure M, compound **19l** was prepared from compound **18f** as a white solid at a 56% yield. mp 92-93°C. ¹H NMR (400MHz, CDCl₃) *δ* 10.08 (s, 1H), 7.98 (d, *J* = 8.0 Hz, 1H), 7.95 (d, *J* = 8.8 Hz, 1H), 7.39 (d, *J* = 8.8u Hz, 1H), 7.36-7.32 (m, 1H), 7.10-7.06 (m, 1H), 6.69 (d, *J* = 8.4 Hz, 1H), 6.59 (d, *J* = 10.0 Hz, 1H), 5.71 (d, *J* = 10.0 Hz, 1H), 4.52 (t, *J* = 6.0 Hz, 2H), 3.92 (s, 3H), 3.19 (t, *J* = 5.6 Hz, 2H), 2.51 (s, 3H), 1.46 (s, 6H). ESI-MS [M+H]⁺ 407.

### Example 3.7. Preparation of 5-methoxy-2,2-dimethyl-N-(1-(2-(1-methylpiperidine-4-yl)ethyl)-1H-indazole-3-yl)-2H-chromene-6-carboxamide (formula 1-20, 18g)

Using procedure C-method I, compound **18g** was prepared from compound **17g** and compound **29** prepared according to Example 2.1.3 as a white solid. mp 97-98°C. ¹H NMR (400MHz, CDCl₃) *δ* 10.08 (s, 1H), 8.08 (d, *J* = 8.0 Hz, 1H), 8.03 (d, 8.8 Hz, 1H), 7.39-7.35 (m, 1H), 7.29 (d, *J* = 8.4 Hz, 1H), 7.14-7.10 (m, 1H), 6.72 (d, *J* = 8.8 Hz, 1H), 6.61 (d, *J* = 10.0 Hz, 1H), 7.72 (d, *J* = 10.4 Hz, 1H), 4.32 (t, *J* = 7.2 Hz, 2H), 3.94 (s, 3H), 2.83 (d, *J* = 11.6 Hz, 2H), 2.25 (s, 3H), 1.91-1.82 (m., 4H), 1.75 (d, *J* = 12.0 Hz, 2H), 1.48 (s, 6H), 1.42-1.26 (m, 3H). ESI-MS [M+H]⁺ 475.

### Example 3.8. Preparation of 5-methoxy-2,2-dimethyl-N-(1-(2-(4-methylpiperazine-1-yl)ethyl)-1H-indazole-3-yl)-2H-chromene-6-carboxamide (formula 1-21, 18h)

Using procedure C-method I, compound **18h** was prepared from compound **17h** and compound **29** prepared according to Example 2.1.3 as a white solid. mp 102-103°C. ¹H NMR (400MHz, CDCl₃) *δ* 10.08 (s, 1H), 8.06 (d, *J* = 8.0 Hz, 1H), 8.02 (dd, *J* = 8.8, 2.8 Hz, 1H), 7.38-7.32 (m, 2H), 7.11 (t, *J* = 6.8 Hz, 1H), 6.60 (d, *J* = 10Hz, 1H), 5.71 (d, *J* = 10.4 Hz, 1H), 4.42 (t, *J* = 6.8 Hz, 2H), 3.94 (s, 3H), 2.86 (t, *J* = 6.4 Hz, 2H), 2.58-2.42 (brm, 8H), 2.28 (s, 3H), 1.46 (s, 6H). ESI-MS [M+H]⁺ 476.

### Example 3.9. Preparation of 5-methoxy-2,2-dimethyl-N-(1-(2-morpholinoethyl)-1H-indazole-3-yl)-2H-chromene-6-carboxamide (formula 1-22, 18i)

### Example 3.9.1. Preparation of 2-(1-(2-morpholinoethyl)-1H-indazole-3-yl)isoindoline-1,3-dione (16i)

Using procedure A-method I, compound **16i** was prepared from compound **15** prepared according to Example 3.1.2 and 4-(2-bromoethyl)morpholine as a white solid at a 61% yield. ¹H NMR (400MHz, CDCl₃) *δ* 8.02-7.95 (m, 2H), 7.85-7.80 (m, 2H), 7.54 (d, *J* = 8.0 Hz, 1H), 7.51 (br, 1H), 7.44 (t, *J* = 8.4 Hz, 1H), 7.20 (t, *J* = 7.6 Hz, 1H), 4.61 (br, 2H), 3.71 (br, 4H), 2.98 (br, 2H), 2.56 (br, 4H).

### Example 3.9.2. Preparation of 1-(2-morpholinoethyl)-1H-indazole-3-amine (171)

Using procedure N, compound **17i** was prepared from compound **16i** as a brown solid at a 71% yield. ¹H NMR (400MHz, CDCl₃) *δ* 7.51 (d, *J* = 5.6 Hz, 1H), 7.34 (t, *J* = 7.2 Hz, 1H), 7.27 (d-like, 1H), 7.02 (t, *J* = 7.6 Hz, 1H), 4.33 (br, 2H), 4.04 (br, 2H), 3.70 (br, 4H), 2.85 (br, 2H), 2.55 (br, 4H).

### Example 3.9.3. Preparation of 5-methoxy-2,2-dimethyl-N-(1-(2-morpholinoethyl)-1H-indazole-3-yl)-2H-chromene-6-carboxamide (18i)

Using procedure C-method I, compound **18i** was prepared from compound **17i** and compound **29** prepared according to Example 2.1.3 as a white solid at an 85% yield. mp 84-85°C. ¹H NMR (400MHz, CDCl₃) *δ* 10.09 (s, 1H), 8.07 (d, *J* = 8.0 Hz, 1H), 8.02 (d, *J* = 8.4 Hz, 1H), 7.39-7.33 (m, 2H), 7.14-7.10 (m, 1H), 6.71 (d, *J* = 9.2 Hz, 1H), 6.59 (d, *J* = 9.6 Hz, 1H), 5.71 (d, *J* = 10.4 Hz, 1H), 4.43 (t, *J* = 6.8 Hz, 2H), 3.94 (s, 3H), 3.67 (t, *J* = 4.4 Hz, 4H), 2.85 (t, *J* = 7.2 Hz, 2H), 2.52 (t, *J* = 4.0 Hz, 4H), 1.46 (s, 6H). ESI-MS [M+H]⁺ 463.

### Example 3.10. Preparation of 5-methoxy-2,2-dimethyl-N-(1-(2-(pyridine-3-yl)ethyl)-1H-indazole-3-yl)-2H-chromene-6-carboxamide (formula 1-23, 18j)

### Example 3.10.1. Preparation of 2-(1-(2-(pyridine-3-yl)ethyl)-1H-indazole-3-yl)isoindoline-1,3-dione (16j)

Using procedure A-method I, compound **16j** was prepared from compound **15** prepared according to Example 3.1.2 and 2-(pyridine-3-yl)ethyl 4-methylbenzenesulfonate as an off-white solid at a 67% yield. ¹H NMR (400MHz, CDCl₃) *δ* 8.49 (d, *J =* 1.6 Hz, 1H), 8.42 (d, *J =* 3.,6 Hz, 1H), 8.03=7.99 (m, 2H), 7.86-7.81 (m, 2H), 7.52 (d, *J =* 8.4 Hz, 1H), 7.36-7.31 (m, 2H), 7.21 (d, *J =* 6.0 Hz, 1H), 7.15 (t, *J=* 7.2 Hz, 1H), 7.09 (dd, *J=* 7.6, 5.2 Hz, 1H), 4.64 (t, *J* = 6.8 Hz, 2H), 3.30 (t, *J=* 7.2 Hz, 2H).

### Example 3.10.2. Preparation of 1-(2-(pyridine-3-yl)ethyl)-1H-indazole-3-amine (17j)

Using procedure N, compound **17j** was prepared from compound **16j** as a brown solid at an 87% yield. ¹H NMR (400MHz, CDCl₃) δ 8.41-8.38 (m, 2H), 7.49 (d. *J =* 8.0 Hz, 1H), 7.32 (dt, *J* = 7.6, 2.0 Hz, 1H), 7.21 (d, *J* = 7.2 Hz, 1H), 7.08 (dd, *J* = 7.6, 4.4 Hz, 1H), 6.98-6.95 (m, 2H), 4.34 (t, *J =* 7.6 Hz, 2H), 4.08 (brs, 2H), 3.13 (t, *J =* 6.8 Hz, 2H).

### Example 3.10.3. Preparation of 5-methoxy-2,2-dimethyl-N-(1-(2-(pyridine-3-yl)ethyl)-1H-indazole-3-yl)-2H-chromene-6-carboxamide (18j)

Using procedure C-method I, compound **18j** was prepared from compound **17j** and compound **29** prepared according to Example 2.1.3 as a white solid at an 89% yield. ¹H NMR (400MHz, CDCl₃) *δ* 10.12 (s, 1H), 8.46 (s, 1H), 8.43 (d, *J =* 4.0 Hz, 1H), 8.08 (d, *J=* 8.0 Hz, 1H), 8.04 (d, *J =* 8.8 Hz, 1H), 7.34 (dt-like, *J* = 7.6 Hz, 1H), 7.32-7.28 (m, 1H), 7.13-7.08 (m, 3H), 7.73 (d, *J =* 8.4 Hz, 1H), 6.62 (d, *J =* 10.4 Hz, 1H), 5.73 (d, *J =* 9.6 Hz, 1H), 4.51 (t, *J=* 6.8 Hz, 2H), 3.96 (s, 3H), 3.22 (t, *J =* 7.2 Hz, 2H), 1.47 (s, 6H). ESI-MS [M+H]⁺ 455.

### Example 3.11. Preparation of 5-methoxy-2,2-dimethyl-N-(1-(2-(pyridine-4-yl)ethyl)-1H-indazole-3-yl)-2H-chromene-6-carboxamide (formula 1-24, 18k)

### Example 3.11.1. Preparation of 1-(2-(pyridine-4-yl)ethyl)-1H-indazole-3-amine (17k)

A mixture of 3-aminoindazole (1.0 eq), potassium phosphate (2.0 eq), 4-vinylpyridine (1.5 eq) and TBAB (20 mol%) in water was added to a microwave glass vial with a magnetic stir bar. The vial was treated in a microwave at 180°C for 20 minutes. After the reaction was completed, the mixture was cooled to room temperature and extracted with ethyl acetate. The ethyl acetate layer was washed with brine, dried over MgSO₄ and concentrated. The residue was purified by silica gel column chromatography using ethyl acetate/n-hexane (1/2) as an eluent to obtain an off-white solid at a 56% yield. ¹H NMR (400MHz, CDCl₃) *δ* 8.41 (br, 2H), 7.49 (d, *J =* 8.0 Hz, 1H), 7.25-7.21 (m, 1H), 7.08-6.95 (m, 3H), 4.35 (t, *J=* 7.2 Hz, 2H), 3.11 (t, *J=* 7.2 Hz, 2H).

### Example 3.11.2. Preparation of 5-methoxy-2,2-dimethyl-N-(1-(2-(pyridine-4-yl)ethyl)-1H-indazole-3-yl)-2H-chromene-6-carboxamide (18k)

Using procedure C-method I, compound **18k** was prepared from compound **17k** and compound **29** prepared according to Example 2.1.3 as a white solid at a 93% yield. mp 68-69°C. ¹H NMR (400MHz, CDCl₃) *δ* 10.11 (s, 1H), 8.46 (br, 2H), 8.08 (d, *J =* 8.0 Hz, 1H), 8.03 (d, *J =* 8.4 Hz, 1H), 7.30 (t, *J =* 8.4 Hz, 1H), 7.13-7.05 (m, 4H), 6.72 (d, *J* = 8.8 Hz, 1H), 6.61 (d, *J =* 10.4 Hz, 1H), 5.72 (d, *J =* 10.4 Hz, 1H), 4.52 (t, *J =* 7.2 Hz, 2H), 3.94 (s, 3H), 3.17 (t, *J =* 6.8 Hz, 2H), 1.47 (s, 6H). ESI-MS [M+H]⁺ 455.

### Example 3.12. Preparation of 5-methoxy-N-(1-(2-methoxyethyl)-1H-indazole-3-yl)-N,2,2-trimethyl-2H-chromene-6-carboxamide (formula 1-25, 20c)

Using procedure C-method I, compound **18c** was obtained from compound **17c** prepared according to Example 3.3.2. and compound **29** prepared according to Example 2.1.3. To a solution of compound **18c** in anhydrous THF at 0°C in the presence of nitrogen gas was added NaH (1.1 eq). The mixture was stirred and a portion of iodomethane (3.0 eq) was added to the mixture all at once. The mixture was removed from an ice bath. After being removed from the ice bath, the mixture was stirred at room temperature for an additional 4 hours. Next, the reaction mixture was quenched with water, extracted with ethyl acetate, dried over MgSO₄, and concentrated. The residue was purified by silica gel chromatography using ethyl acetate/hexane (1/1) as an eluent to obtain compound **20c** (white solid, yield 94%). mp 78-79°C. ¹H NMR (400MHz, CD₃OD) *δ* 7.56 (d, *J =* 8.4 Hz, 1H), 7.43 (d, *J =* 7.6 Hz, 1H), 7.31 (t, *J =* 7.2 Hz, 1H), 7.01 (t, *J =* 8.0 Hz, 1H), 6.82 (d, *J =* 8.0 Hz, 1H), 6.39 (d, *J =* 8.0 Hz, 1H), 6.16 (d, *J =* 10.0 Hz, 1H), 5.60 (d, *J =* 10.0 Hz, 1H), 4.29 (t, *J =* 5.6 Hz, 2H), 3.85 (s, 3H), 3.54 (t, *J* = 5.6 Hz, 2H), 3.50 (s, 3H), 3.07 (s, 3H), 1.25 (s, 6H). ESI-MS [M+H]⁺ 422.

### Example 4. Preparation of 2-chromene-6-carboxylic acid derivatives

A method for producing 2-chromene-6-carboxylic acid derivatives is shown by following scheme 3. (a) [Method A] 3-methyl-2-butenal, CaCl₂, Et₃N, EtOH, reflux, 2 hours; [Method B] (i) R₂C(OH)CCH, *conc.* HCl, CuCl, Cu, CaCl₂, 0°C, 1 hour, (ii) CuI, K₂CO₃, KI, DMF, room temperature, 15 hours, (iii) o-xylene, 180°C, 1 hour; (b) For **24, 25,** RI, K₂CO₃, DMF, 40°C, 3 hours; (c) For **29-32,** [Method A] 27% H₂O₂ (aq), NaClO₂, NaH₂PO₄, CH₃CN, 0°C, 2 hours, for **33,** [Method B] NaOH, EtOH, H₂O, 120°C, 2 hours; (d) **9k,** EDC.HCl, HOBt, Et₃N, MC, room temperature, 15 hours; (e) H₂, 10% Pd/C, MeOH, room temperature, 3 hours.

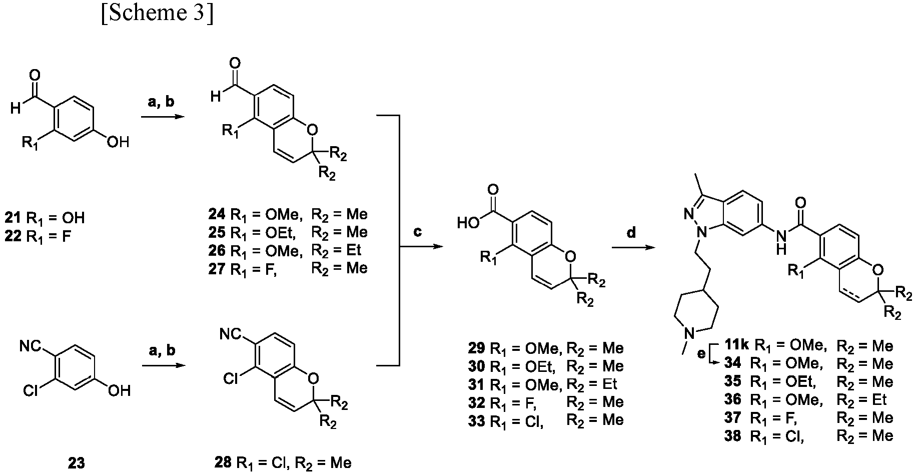

### Example 4.1. Preparation of 5-methoxy-2,2-dimethyl-N-(3-methyl-1-(2-(1-methylpiperidine-4-yl)ethyl)-1H-indazole-6-yl)-chromene-6-carboxamide (formula 1-26, 34)

Using procedure B, compound **34** was prepared from compound **11k** prepared according to Example 2.7.4 as a white solid at a 99% yield. mp 74-75°C. ¹H NMR (400 MHz, CDCl₃) δ 9.91 (s, 1H), 8.34 (d, *J =* 1.7 Hz, 1H), 7.94 (d, *J =* 8.8 Hz, 1H), 7.54 (d, *J =* 8.5 Hz, 1H), 6.85 (dd, *J = 8.5,* 1.7 Hz, 1H), 6.73 (d, *J =* 8.8 Hz, 1H), 4.32 (t, *J =* 7.4 Hz, 2H), 3.89 (s, 3H), 2.95 - 2.87 (m, 2H), 2.81 (t, *J=* 6.7 Hz, 2H), 2.53 (s, 3H), 2.30 (s, 3H), 2.03 - 1.92 (m, 2H), 1.91 - 1.75 (m, 6H), 1.46 - 1.39 (m, 2H), 1.36 (s, 6H), 1.34 - 1.30 (m, 1H). FAB-MS [M+H]⁺ 491.

### Example 4.2. Preparation of 5-ethoxy-2,2-dimethyl-N-(3-methyl-1-(2-(1-methylpiperidine-4-yl)ethyl)-1H-indazole-6-yl)-2H-chromene-6-carboxamide (formula 1-27, 35)

### Example 4.2.1. Preparation of 5-ethoxy-2,2-dimethyl-2H-chromene-6-carbaldehyde (25)

Using procedure I-method II and procedure A-method I, compound **25** was prepared with iodomethane from compound **21** as a pale yellow oil at a 63% yield. ¹H NMR (400 MHz, CDCl₃) δ 10.17 (s, 1H), 7.65 (d, *J =* 8.6 Hz, 1H), 6.63 (d, *J =* 8.6 Hz, 1H), 6.57 (dd, *J=* 10.0, 0.8 Hz, 1H), 5.66 (d, *J =* 10.0 Hz, 1H), 4.03 (q, *J =* 7.0 Hz, 2H), 1.45 (s, 6H), 1.43 (t, *J =* 7.1 Hz, 3H).

### Example 4.2.2. Preparation of 5-ethoxy-2,2-dimethyl-2H-chromene-6-carboxylic acid (30)

Using procedure J, compound **30** was prepared from compound **25** as a white solid at a 55% yield. ¹H NMR (400 MHz, DMSO-*d*₆) δ 12.46 (s, 1H), 7.53 (d, *J =* 8.6 Hz, 1H), 6.58 - 6.52 (m, 2H), 5.78 (d, *J =* 10.0 Hz, 1H), 3.87 (q, *J =* 7.0 Hz, 2H), 1.35 (s, 6H), 1.26 (t, *J=* 7.0 Hz, 3H).

### Example 4.2.3. Preparation of 5-ethoxy-2,2-dimethyl-N-(3-methyl-1-(2-(1-methylpiperidin-4-yl)ethyl)-1H-indazole-6-yl)-2H-chromene-6-carboxamide (35)

Using procedure C-method I, compound **35** was prepared from compound **30** and compound **9k** prepared according to Example 2.7.3 as a white solid at a 30% yield. mp 72-73°C. ¹H NMR (400 MHz, CDCl₃) δ 10.04 (s, 1H), 8.36 (s, 1H), 7.96 (d, *J=* 8.7 Hz, 1H), 7.55 (d, *J =* 8.5 Hz, 1H), 6.84 (dd, *J =* 8.5, 1.8 Hz, 1H), 6.72 (d, *J =* 8.7 Hz, 1H), 6.57 (d, *J=* 10.0 Hz, 1H), 5.72 (d, *J* = 10.0 Hz, 1H), 4.32 (t, *J =* 7.3 Hz, 2H), 4.04 (q, *J =* 7.1 Hz, 2H), 3.05 - 2.95 (m, 2H), 2.52 (s, 3H), 2.36 (s, 3H), 2.17 - 2.06 (m, 2H), 1.87 (q, *J=* 7.1 Hz, 2H), 1.84 - 1.77 (m, 2H), 1.54 - 1.47 (m, 5H), 1.46 (s, 6H), 1.39 - 1.31 (m, 1H). FAB-MS [M+H]⁺ 503.

### Example 4.3. Preparation of 2.2-diethyl-5-methoxy-N-(3-methyl-1-(2-(1-methylpiperidine-4-yl)ethyl)-1H-indazole-6-yl)-2H-chromene-6-carboxamide (formula 1-28, 36)

### Example 4.3.1. Preparation of 2,2-diethyl-5-methoxy-2H-chromene-6-carbaldehyde (26)

Using procedure H-method III, procedure I-method I, and procedure A-method A, compound **26** was prepared from 3-chloro-3-ethylpent-1-yne and compound **21** as a colorless oil at a 41% yield. ¹H NMR (400 MHz, CDCl₃) δ 10.14 (s, 1H), 7.63 (d, *J =* 8.6 Hz, 1H), 6.69 (d, *J =* 10.2 Hz, 1H), 6.62 (d, *J=* 8.6 Hz, 1H), 5.53 (d, *J =* 10.3 Hz, 1H), 3.88 (s, 3H), 1.86 - 1.57 (m, 4H), 0.93 (t, *J =* 7.5 Hz, 6H).

### Example 4.3.2. Preparation of 2,2-diethyl-5-methoxy-2H-chromene-6-carboxylic acid (31)

Using procedure J, compound **31** was prepared from compound **26** as a white solid at a 67% yield. ¹H NMR (400 MHz, CDCl₃) δ 10.73 (s, 1H), 7.88 (d, *J =* 8.7 Hz, 1H), 6.68 (dd, *J* = 8.7, 0.8 Hz, 1H), 6.63 (dd, *J* = 10.2, 0.8 Hz, 1H), 5.59 (d, *J* = 10.3 Hz, 1H), 3.93 (s, 3H), 1.85 - 1.60 (m, 4H), 0.93 (t, *J =* 7.5 Hz, 6H).

### Example 4.3.3. Preparation of 2,2-diethyl-5-methoxy-N-(3-methyl-1-(2-(1-methylpiperidin-4-yl)ethyl)-1H-indazole-6-yl)-2H-chromene-6-carboxamide (36)

Using procedure C-method I, compound **36** was prepared from compound **31** and compound **9k** prepared according to Example 2.7.3 as a white solid at a 53% yield. mp 66-67°C. ¹H NMR (400 MHz, DMSO-*d*₆) δ 10.21 (s, 1H), 8.14 (s, 1H), 7.58 (d, *J =* 8.7 Hz, 1H), 7.37 (d, *J =* 8.4 Hz, 1H), 7.19 (dd, *J =* 8.7, 1.7 Hz, 1H), 6.69 (d, *J =* 10.3 Hz, 1H), 6.62 (dd, *J =* 8.5*,* 0.7 Hz, 1H), 5.71 (d, *J =* 10.3 Hz, 1H), 4.23 (t, *J =* 7.1 Hz, 2H), 3.74 (s, 3H), 2.94 - 2.80 (m, 2H), 2.40 (s, 3H), 2.28 (s, 3H), 2.21 - 2.01 (m, 2H), 1.77 - 1.55 (m, 8H), 1.29 - 1.18 (m, 3H), 0.85 (t, *J* = 7.4 Hz, 6H). FAB-MS [M+H]⁺ 517.

### Example 4.4. Preparation of 5-fluoro-2,2-dimethyl-N-(3-methyl-1-(2-(1-methylpiperidine-4-yl)ethyl)-1H-indazole-6-yl)-2H-chromene-6-carboxamide (formula 1-29, 37)

### Example 4.4.1. Preparation of 5-fluoro-2,2-dimethyl-2H-chromene-6-carbaldehyde (27)

Using procedure H-method III and procedure I-method I, compound **27** was prepared from 3-chloro-3-methylbut-1-yne and compound **22** as a colorless oil at a 21% yield. ¹H NMR (400 MHz, CDCl₃) δ 10.16 (d, *J =* 0.8 Hz, 1H), 7.63 (dd, *J =* 8.6, 7.9 Hz, 1H), 6.64 (dd, *J=* 8.6, 0.9 Hz, 1H), 6.57 (dd, *J=* 10.1, 0.8 Hz, 1H), 5.70 (d, *J =* 10.1 Hz, 1H), 1.47 (s, 6H).

### Example 4.4.2. Preparation of 5-fluoro-2,2-dimethyl-2H-chromene-6-carboxylic acid (32)

Using procedure J, compound **32** was prepared from compound **27** as a white solid at an 84% yield. ¹H NMR (400 MHz, DMSO-*d*₆) δ 7.57 (t, *J =* 8.6 Hz, 1H), 6.61 (d, *J* = 8.6 Hz, 1H), 6.52 (d, *J=* 10.1 Hz, 1H), 5.85 (d, *J=* 10.0 Hz, 1H), 1.37 (s, 6H).

### Example 4.4.3. Preparation of 5-fluoro-2,2-dimethyl-N-(3-methyl-1-(2-(1-methylpiperidin-4-yl)ethyl)-1H-indazole-6-yl)-2H-chromene-6-carboxamide (37)

Using procedure C-method I, compound **37** was prepared from compound **32** and compound **9k** prepared according to Example 2.7.3 as a white solid at a 40% yield. mp 69-70°C. ¹H NMR (400 MHz, DMSO-*d*₆) δ 10.32 (s, 1H), 8.11 (s, 1H), 7.59 (d, *J =* 8.8 Hz, 1H), 7.43 (t, *J =* 8.4 Hz, 1H), 7.18 (dd, *J* = 8.7, 1.6 Hz, 1H), 6.71 (d, *J =* 8.5 Hz, 1H), 6.58 (d, *J=* 10.0 Hz, 1H), 5.92 (d, *J =* 10.0 Hz, 1H), 4.23 (t, *J=* 7.1 Hz, 2H), 3.03 - 2.84 (m, 2H), 2.40 (s, 3H), 2.32 (s, 3H), 2.26 - 2.02 (m, 2H), 1.79 - 1.65 (m, 4H), 1.40 (s, 6H), 1.30 - 1.20 (m, 3H). FAB-MS [M+H]⁺ 477.

### Example 4.5. Preparation of 5-chloro-2,2-dimethyl-N-(3-methyl-1-(2-(1-methylpiperidine-4-yl)ethyl)-1H-indazole-6-yl)-2H-chromene-6-carboxamide (formula 1-30, 38)

### Example 4.5.1. Preparation of 5-chloro-2,2-dimethyl-2H-chromene-6-carbonitrile (28)

Using procedure H-method III and procedure I-method I, compound **28** was prepared from 3-chloro-3-methylbut-1-yne and compound **23** as a white solid at a 50% yield. ¹H NMR (400 MHz, CDCl₃) δ 7.38 (d, *J* = 8.5 Hz, 1H), 6.72 (d, *J* = 8.5 Hz, 1H), 6.66 (d, *J =* 10.2 Hz, 1H), 5.80 (d, *J =* 10.2 Hz, 1H), 1.45 (s, 6H).

### Example 4.5.2. Preparation of 5-chloro-2,2-dimethyl-2H-chromene-6-carboxylic acid (33)

Using procedure K, compound **33** was prepared from compound **28** as a white solid at an 87% yield. ¹H NMR (400 MHz, DMSO-*d*₆) δ 13.03 (br s, 1H), 7.55 (d, *J=* 8.5 Hz, 1H), 6.76 (d, *J =* 8.6 Hz, 1H), 6.70 (d, *J =* 10.2 Hz, 1H), 5.95 (d, *J =* 10.1 Hz, 1H), 1.37 (s, 6H).

### Example 4.5.3. Preparation of 5-chloro-2,2-dimethyl-N-(3-methyl-1-(2-(1-methylpiperidin-4-yl)ethyl)-1H-indazole-6-yl)-2H-chromene-6-carboxamide (38)

Using procedure C-method I, compound **38** was prepared from compound **33** and compound **9k** prepared according to Example 2.7.3 as a white solid at a 35% yield. mp 146-147°C. ¹H NMR (400 MHz, DMSO-*d*₆) δ 10.50 (s, 1H), 8.11 (d, *J =* 1.6 Hz, 1H), 7.58 (d, *J=* 8.6 Hz, 1H), 7.32 (d, *J=* 8.3 Hz, 1H), 7.17 (dd, *J* = 8.7, 1.6 Hz, 1H), 6.82 (d, *J=* 8.4 Hz, 1H), 6.69 (d, *J* = 10.1 Hz, 1H), 5.99 (d, *J =* 10.1 Hz, 1H), 4.21 (t, *J* = 7.2 Hz, 2H), 2.70 - 2.62 (m, 2H), 2.40 (s, 3H), 2.06 (s, 3H), 1.74 - 1.62 (m, 6H), 1.39 (s, 6H), 1.17 - 1.10 (m, 3H). FAB-MS [M+H]⁺ 493.

### Example 5. Preparation of 4-methylamino benzamide derivatives

A method for producing 4-methylamino benzamide derivatives is shown by the following scheme 4. (a) BnBr, K₂CO₃, DMF, room temperature, 18 hours; (b) RCl, Et₃N, MC, room temperature, 3 hours; (c) H₂, Pd/C, MeOH, room temperature, 3 hours, (d) BzCl, NaOH, THF, H₂O, room temperature, 2 hours; (e) **9k,** EDC·HCl, HOBt, Et₃N, MC, room temperature, 15 hours.

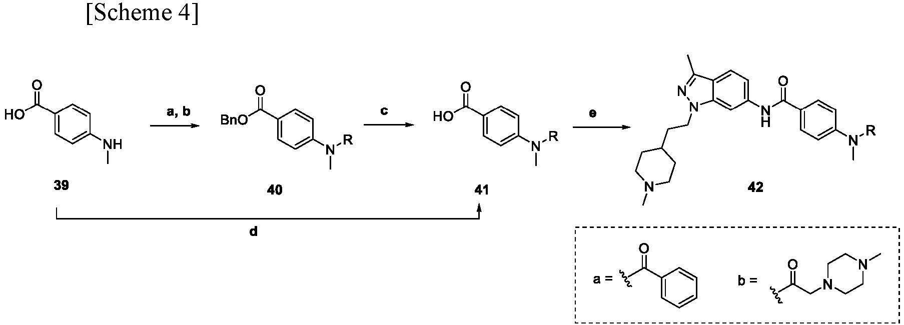

### Example 5.1. Preparation of N-methyl-N-(4-((3-methyl-1-(2-(1-methylpiperidin-4-yl)ethyl)-1H-indazole-6-yl)carbamoyl)phenyl)benzamide (formula 1-31, 42a)

### Example 5.1.1. Preparation of 4-(N-methylbenzamido)benzoic acid (41a)

Using procedure C-method II, compound **41a** was prepared from compound **39** and benzoyl chloride as a white solid at a 36% yield. ¹H NMR (400 MHz, CDCl₃) δ 7.94 (d, *J* = 8.6 Hz, 2H), 7.32 - 7.25 (m, 3H), 7.18 (t, *J =* 7.5 Hz, 2H), 7.10 (d, *J =* 8.6 Hz, 2H), 3.53 (s, 3H).

### Example 5.1.2. Preparation of N-methyl-N-(4-((3-methyl-1-(2-(1-methylpiperidin-4-yl)ethyl)-1H-indazole-6-yl)carbamoyl)phenyl)benzamide (42a)

Using procedure C-method I, compound **42a** was prepared from compound **41a** and compound **9k** prepared according to Example 2.7.3 as a white solid at a 33% yield. mp 201-202°C. ¹H NMR (400 MHz, CDCl₃) δ 8.17 - 8.08 (m, 2H), 7.79 - 7.73 (m, 2H), 7.53 (d, *J =* 8.4 Hz, 1H), 7.32 - 7.24 (m, 3H), 7.21 - 7.16 (m, 2H), 7.12 (d, *J =* 8.5 Hz, 2H), 6.92 (dd, *J =* 8.6, 1.7 Hz, 1H), 4.27 (t, *J =* 7.5 Hz, 2H), 3.51 (s, 3H), 2.90 - 2.76 (m, 2H), 2.51 (s, 3H), 2.23 (s, 3H), 1.92 - 1.77 (m, 4H), 1.76 - 1.69 (m, 2H), 1.38 - 1.26 (m, 3H). FAB-MS [M+H]⁺ 510.

### Example 5.2. Preparation of N-(3-methyl-1-(2-(1-methylpiperidin-4-yl)ethyl)-1H-indazole-6-yl)-4-(N-methyl-2-(4-methylpiperazin-1-yl)acetamido)benzamide (formula 1-32, 42b)

### Example 5.2.1. Preparation of benzyl 4-(N-methyl-2-(4-methylpiperazin-1-yl)acetamido)benzoate (40b)

Using procedure H-method I, compound **39** and benzyl bromide were acylated with chloroacetyl chloride and then substituted with 1-methylpiperazine in the presence of Et₃N in MC to afford compound **40b** as a white solid at a 62% yield. ¹H NMR (400 MHz, CDCl₃) δ 8.11 (d, *J =* 8.4 Hz, 2H), 7.48 - 7.33 (m, 5H), 7.28 (d, *J =* 8.6 Hz, 2H), 5.37 (s, 2H), 3.28 (s, 3H), 2.96 (s, 2H), 2.70 - 2.37 (m, 8H), 2.32 (s, 3H).

### Example 5.2.2. Preparation of 4-(N-methyl-2-(4-methylpiperazin-1-yl)acetamido)benzoic acid (41b)

Using procedure B, compound **41b** was prepared from compound **40b** as a light yellow solid at a 97% yield. ¹H NMR (400 MHz, MeOD) δ 7.99 (d, *J =* 8.4 Hz, 2H), 7.28 (d, *J =* 8.4 Hz, 2H), 3.26 (s, 3H), 3.03 (s, 2H), 2.96 - 2.79 (m, 4H), 2.74 - 2.43 (m, 7H).

### Example 5.2.3. Preparation of N-(3-methyl-1-(2-(1-methylpiperidin-4-yl)ethyl)-1H-indazole-6-yl)-4-(N-methyl-2-(4-methylpiperazin-1-yl)acetamido)benzamide (42b)

Using procedure C-method III, compound **42b** was prepared from compound **41b** and compound **9k** prepared according to Example 2.7.3 as a white solid at a 18% yield. mp 52-53°C. ¹H NMR (400 MHz, DMSO-*d*₆) δ 10.36 (s, 1H), 8.12 (s, 1H), 7.99 (d, *J =* 8.3 Hz, 2H), 7.61 (d, *J=* 9.0 Hz, 1H), 7.47 (d, *J =* 8.3 Hz, 2H), 7.34 (dd, *J=* 8.7, 1.6 Hz, 1H), 4.23 (t, *J =* 7.3 Hz, 2H), 3.21 (s, 3H), 2.98 (s, 2H), 2.69 - 2.63 (m, 2H), 2.41 (s, 3H), 2.38 - 2.10 (m, 8H), 2.06 (s, 3H), 2.06 (s, 3H), 1.74 - 1.66 (m, 4H), 1.66 - 1.62 (m, 2H), 1.17 - 1.12 (m, 3H). FAB-MS [M+H]⁺ 546.

### Example 6. Preparation of 2,4-disubstituted benzamide derivatives

A method for producing 2,4-disubstituted benzamide derivatives is shown by the following scheme 5. (a) Boc₂O, DMAP, t-BuOH, MC, room temperature, 20 hours; (b) 1-methylpiperidine, room temperature, 12 hours; (c) TFA, MC, room temperature, 3 hours; (d) (i) SOCl₂, MC, DMF, room temperature, 2 hours; (ii) **9k,** Et₃N, MC, room temperature, 1 hour; (e) H₂, 10% Pd/C, MeOH, room temperature, 3 hours; (f) Tetrahydro-4H-pyran-4-one, NaBH(OAc)₃, TFA, MC, room temperature, 18 hours.

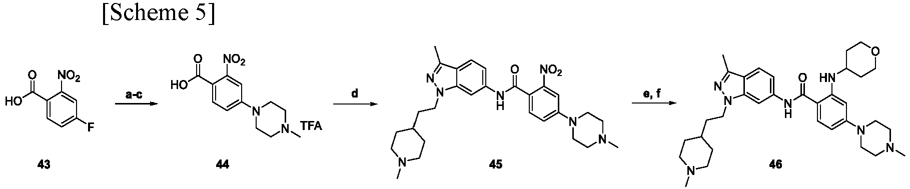

### Example 6.1. Preparation of N-(3-methyl-1-(2-(1-methylpiperidin-4-yl)ethyl)-1H-indazole-6-yl)-4-(4-methylpiperazin-1-yl)-2-((tetrahydro-2H-pyran-4-yl)amino)benzamide (formula 1-33, 46)

### Example 6.1.1. Preparation of tert-butyl 4-(4-methylpiperazin-1-yl)-2-nitrobenzoate (44)

Compound **43** (1.0 eq) was dissolved in a mixture of *t*-BuOH (2 mL) and MC (2 mL). Next, (Boc)₂O (2.0 eq) and 4-(*N*,*N*-dimethylamino)pyridine (0.3 eq) were added to the mixture. The reaction mixture was stirred at room temperature for 20 hours. The solvent was evaporated to dryness, the residue was diluted with EA, and then washed with 1M HCl and water. The organic layer was dried over anhydrous Na₂SO₄ and evaporated to dryness. 1-methylpiperazine (2 mL) was added to the residue. The mixture was stirred at room temperature for 12 hours. Then, water (20 mL) was added to the mixed solution, and the mixture was stirred at room temperature for 12 hours. The solid was filtered, washed with water and dried at 40°C to obtain a yellow solid, which was deprotected with TFA (2 mL) in MC (2 mL) to afford compound **44** as a yellow solid at a 68% yield (TFA salt). ¹H NMR (400 MHz, MeOD) δ 7.82 (d, *J =* 8.8 Hz, 1H), 7.28 (d, *J=* 2.5 Hz, 1H), 7.18 (dd, *J=* 8.8, 2.6 Hz, 1H), 3.69 - 3.09 (m, 8H), 2.94 (s, 3H).

### Example 6.1.2. Preparation of N-(3-methyl-1-(2-(1-methylpiperidin-4-yl)ethyl)-1H-indazole-6-yl)-4-(4-methylpiperazin-1-yl)-2-nitrobenzamide (45)

Using procedure C-method III, compound **45** was prepared from compound **44** and compound **9k** prepared according to Example 2.7.3 as a yellow solid at a 52% yield. ¹H NMR (400 MHz, DMSO-*d₆*) δ 10.36 (s, 1H), 8.12 (s, 1H), 7.99 (d, J = 8.3 Hz, 2H), 7.61 (d, J = 9.0 Hz, 1H), 7.47 (d, J = 8.3 Hz, 2H), 7.34 (dd, J = 8.7, 1.6 Hz, 1H), 4.23 (t, J = 7.3 Hz, 2H), 3.21 (s, 3H), 2.98 (s, 2H), 2.69 - 2.63 (m, 2H), 2.41 (s, 3H), 2.38 - 2.10 (m, 8H), 2.06 (s, 3H), 2.06 (s, 3H), 1.74 - 1.66 (m, 4H), 1.66 - 1.62 (m, 2H), 1.17 - 1.12 (m, 3H).

### Example 6.1.3. Preparation of N-(3-methyl-1-(2-(1-methylpiperidin-4-yl)ethyl)-1H-indazole-6-yl)-4-(4-methylpiperazin-1-yl)-2-((tetrahydro-2H-pyran-4-yl)amino)benzamide (46)

Using procedure B and procedure E-method II, compound **46** was prepared from compound 45 as a white solid at a 34% yield. mp 123-124°C. ¹H NMR (400 MHz, CDCl₃) δ 7.93 (d, *J=* 7.4 Hz, 1H), 7.90 (d, *J =* 1.7 Hz, 1H), 7.84 (s, 1H), 7.53 (d, *J =* 8.5 Hz, 1H), 7.43 (d, *J =* 8.9 Hz, 1H), 6.89 (dd, *J =* 8.6, 1.7 Hz, 1H), 6.21 (dd, *J =* 8.9, 2.3 Hz, 1H), 6.10 (d, *J=* 2.3 Hz, 1H), 4.29 (t, *J=* 7.4 Hz, 2H), 3.99 (dt, *J =* 11.9, 4.0 Hz, 2H), 3.64 - 3.49 (m, 3H), 3.29 (t, *J=* 5.1 Hz, 4H), 2.90 - 2.82 (m, 2H), 2.55 (t, *J =* 5.1 Hz, 4H), 2.52 (s, 3H), 2.35 (s, 3H), 2.26 (s, 3H), 2.08 - 2.00 (m, 2H), 1.98 - 1.89 (m, 2H), 1.84 (q, *J =* 7.0 Hz, 2H), 1.80 - 1.72 (m, 2H), 1.70 - 1.56 (m, 2H), 1.46 - 1.33 (m, 2H), 1.32 - 1.25 (m, 1H). FAB-MS [M+H]⁺ 574.

### Example 7. Preparation of pyridyl benzamide derivatives

A method for producing pyridyl benzamide derivatives is shown by the following scheme 6. (a) [Method A] *m-* or *p*-Bromopyridine, Pd(PPh₃)₄, K₂CO₃, EtOH, H₂O, 90°C, 18 hours, [Method B] *m-* or *p*-Pyridinylboronic acid, Pd(PPh₃)₄, K₂CO₃, *n*-BuOH, H₂O, 110°C, 18 hours; (b) **9k,** EDC·HCl, HOBt, Et₃N, MC, room temperature, 15 hours.

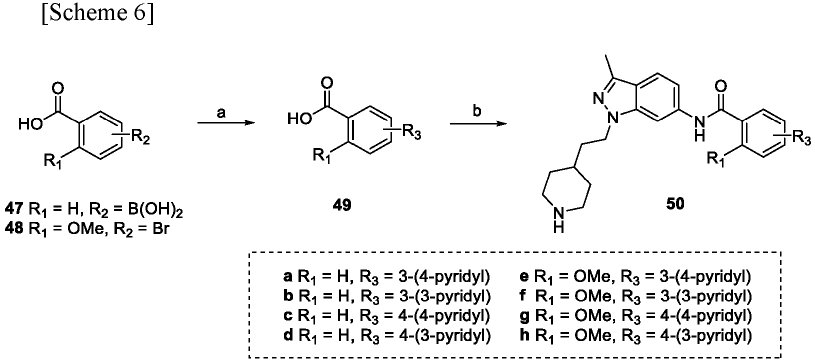

### Example 7.1. Preparation of N-(3-methyl-1-(2-(1-methylpiperidin-4-yl)ethyl)-1H-indazole-6-yl)-3-(pyridine-4-yl)benzamide (formula 1-34, 50a)

### Example 7.1.1. Preparation of 3-(pyridin-4-yl)benzoic acid (49a)

Using procedure L, 3-carboxyphenylboronic acid and 4-bromopyridine were treated with EtOH to obtain compound **49a** as a white solid at a 73% yield. ¹H NMR (401 MHz, DMSO-*d*₆) δ 8.63 (dd, *J=* 4.6, 1.6 Hz, 2H), 8.25 (t, *J =* 1.8 Hz, 1H), 8.05 - 7.97 (m, 2H), 7.72 (dd, *J =* 4.6, 1.7 Hz, 2H), 7.63 (t, *J =* 7.8 Hz, 1H).

### Example 7.1.2. Preparation of N-(3-methyl-1-(2-(1-methylpiperidin-4-yl)ethyl)-1H-indazole-6-yl)-3-(pyridin-4-yl)benzamide (50a)

Using procedure C-method I, compound **50a** was prepared from compound **49a** and compound **9k** prepared according to Example 2.7.3 as a white solid at a 41% yield. mp 57-58°C. ¹H NMR (400 MHz, CDCl₃) δ 8.72 - 8.58 (m, 2H), 8.49 (s, 1H), 8.20 (s, 1H), 8.17 (s, 1H), 7.96 (d, *J* = 7.8 Hz, 1H), 7.80 (d, *J =* 7.9 Hz, 1H), 7.61 (t, *J=* 7.7 Hz, 1H), 7.57 (d, *J =* 8.6 Hz, 1H), 7.52 (d, *J =* 6.2 Hz, 2H), 7.04 (dd, *J =* 8.6, 1.7 Hz, 1H), 4.29 (t, *J=* 7.5 Hz, 2H), 2.89 - 2.81 (m, 2H), 2.53 (s, 3H), 2.26 (s, 3H), 1.98 - 1.89 (m, 2H), 1.88 - 1.81 (m, 2H), 1.78 - 1.70 (m, 2H), 1.44 - 1.33 (m, 2H), 1.32 - 1.27 (m, 1H). FAB-MS [M+H]⁺ 454.

### Example 7.2. Preparation of N-(3-methyl-1-(2-(1-methylpiperidin-4-yl)ethyl)-1H-indazole-6-yl)-3-(pyridine-3-yl)benzamide (formula 1-35, 50b)

### Example 7.2.1. Preparation of 3-(pyridin-3-yl)benzoic acid (49b)

Using procedure L, 3-carboxyphenylboronic acid and 3-bromopyridine were treated with EtOH in solvent H₂O to afford compound **49b** as a white solid at a 67% yield. ¹H NMR (400 MHz, DMSO-*d*₆) δ 8.88 (d, *J =* 2.6 Hz, 1H), 8.57 (dd, *J=* 4.7, 1.6 Hz, 1H), 8.17 (t, *J* = 1.9 Hz, 1H), 8.09 (dt, *J =* 8.0, 2.0 Hz, 1H), 7.99 - 7.89 (m, 2H), 7.60 (t, *J* = 7.8 Hz, 1H), 7.48 (dd, *J=* 7.9, 4.7 Hz, 1H).

### Example 7.2.2. Preparation of N-(3-methyl-1-(2-(1-methylpiperidin-4-yl)ethyl)-1H-indazole-6-yl)-3-(pyridin-3-yl)benzamide (50b)

Using procedure C-method I, compound **50b** was prepared from compound **49b** and compound **9k** prepared according to Example 2.7.3 as a white solid at a 60% yield. mp 56-57°C. ¹H NMR (400 MHz, CDCl₃) δ 8.83 (s, 1H), 8.59 (d, *J =* 4.9 Hz, 1H), 8.53 (s, 1H), 8.20 (s, 1H), 8.11 (t, *J =* 1.8 Hz, 1H), 7.94 - 7.84 (m, 2H), 7.74 - 7.70 (m, 1H), 7.61 - 7.50 (m, 2H), 7.35 (dd, *J =* 7.9, 4.8 Hz, 1H), 7.02 (dd, *J =* 8.6, 1.7 Hz, 1H), 4.28 (t, *J=* 7.5 Hz, 2H), 2.86 - 2.77 (m, 2H), 2.52 (s, 3H), 2.22 (s, 3H), 1.95 - 1.79 (m, 4H), 1.76 - 1.68 (m, 2H), 1.40 - 1.25 (m, 3H). FAB-MS [M+H]⁺ 454.

### Example 7.3. Preparation of N-(3-methyl-1-(2-(1-methylpiperidin-4-yl)ethyl)-1H-indazole-6-yl)-4-(pyridine-4-yl)benzamide (formula 1-36, 50c)

### Example 7.3.1. Preparation of 4-(pyridin-4-yl)benzoic acid (49c)

Using procedure L, 4-carboxyphenylboronic acid and 4-bromopyridine were treated with EtOH in solvent H₂O to afford compound **49c** as a white solid at an 83% yield. ¹H NMR (400 MHz, DMSO-*d*₆) δ 8.64 (dd, *J =* 4.6, 1.7 Hz, 2H), 8.03 (d, *J=* 8.4 Hz, 2H), 7.90 (d, *J =* 8.4 Hz, 2H), 7.73 (dd, *J =* 4.3, 1.6 Hz, 2H).

### Example 7.3.2. Preparation of N-(3-methyl-1-(2-(1-methylpiperidin-4-yl)ethyl)-1H-indazole-6-yl)-4-(pyridin-4-yl)benzamide (50c)

Using procedure C-method I, compound **50c** was prepared from compound **49c** and compound **9k** prepared according to Example 2.7.3 as a white solid at a 41% yield. mp 156-157°C. ¹H NMR (400 MHz, CDCl₃) δ 8.70 (d, *J =* 5.5 Hz, 2H), 8.23 - 8.20 (m, 2H), 8.03 (d, *J =* 8.4 Hz, 2H), 7.75 (d, *J =* 8.5 Hz, 2H), 7.58 (d, *J =* 8.5 Hz, 1H), 7.53 (dd, *J=* 4.7, 1.5 Hz, 2H), 6.98 (dd, *J =* 8.5*,* 1.7 Hz, 1H), 4.31 (t, *J =* 7.5 Hz, 2H), 2.89 - 2.80 (m, 2H), 2.54 (s, 3H), 2.26 (s, 3H), 1.97 - 1.89 (m, 2H), 1.88 - 1.82 (m, 2H), 1.78 - 1.72 (m, 2H), 1.43 - 1.33 (m, 2H), 1.31 - 1.26 (m, 1H). FAB-MS [M+H]⁺ 454.

### Example 7.4. Preparation of N-(3-methyl-1-(2-(1-methylpiperidin-4-yl)ethyl)-1H-indazole-6-yl)-4-(pyridine-3-yl)benzamide (formula 1-37, 50d)

### Example 7.4.1. Preparation of 4-(pyridin-3-yl)benzoic acid (49d)

Using procedure L, 4-carboxyphenylboronic acid and 3-bromopyridine were treated with EtOH in solvent H₂O to afford compound **49d** as a white solid at a 44% yield. ¹H NMR (400 MHz, DMSO-*d*₆) δ 8.92 (d, *J=* 2.4 Hz, 1H), 8.58 (dd, *J=* 4.8, 1.6 Hz, 1H), 8.11 (dt, *J=* 8.0, 2.0 Hz, 1H), 8.01 (d, *J* = 8.3 Hz, 2H), 7.83 (d, *J =* 8.4 Hz, 2H), 7.49 (dd, *J=* 8.0, 4.8 Hz, 1H).

### Example 7.4.2. Preparation of N-(3-methyl-1-(2-(1-methylpiperidin-4-yl)ethyl)-1H-indazole-6-yl)-4-(pyridin-3-yl)benzamide (50d)

Using procedure C-method I, compound **50d** was prepared from compound **49d** and compound **9k** prepared according to Example 2.7.3 as a white solid at a 35% yield. mp 71-72°C. ¹H NMR (400 MHz, CDCl₃) δ 8.86 (s, 1H), 8.64 (d, *J =* 4.8 Hz, 1H), 8.28 (s, 1H), 8.21 (d, *J=* 1.7 Hz, 1H), 8.02 (d, *J =* 8.1 Hz, 2H), 7.91 (d, *J =* 8.0 Hz, 1H), 7.70 (d, *J=* 8.1 Hz, 2H), 7.58 (d, *J=* 8.5 Hz, 1H), 7.40 (dd, *J =* 7.9, 4.8 Hz, 1H), 7.01 (dd, *J =* 8.6, 1.8 Hz, 1H), 4.31 (t, *J=* 7.4 Hz, 2H), 2.97 - 2.85 (m, 2H), 2.53 (s, 3H), 2.29 (s, 3H), 2.03 - 1.94 (m, 2H), 1.91 - 1.84 (m, 2H), 1.78 - 1.73 (m, 2H), 1.48 - 1.36 (m, 2H), 1.34 - 1.28 (m, 1H). FAB-MS [M+H]⁺ 454.

### Example 7.5. Preparation of 2-methoxy-N-(3-methyl-1-(2-(1-methylpiperidin-4-yl)ethyl)-1H-indazole-6-yl)-3-(pyridine-4-yl)benzamide (formula 1-38, 50e)

### Example 7.5.1. Preparation of 2-methoxy-3-(pyridin-4-yl)benzoic acid (49e)

Using procedure L, 3-bromo-2-methoxybenzoic acid and 4-pyridinylboronic acid were treated with BuOH in solvent H₂O to afford compound **49e** as a white solid at a 55% yield. ¹H NMR (400 MHz, MeOD) δ 8.68 - 8.49 (m, 2H), 7.82 (dd, *J* = 7.8, 1.8 Hz, 1H), 7.65 (d, *J=* 5.1 Hz, 2H), 7.57 (dd, *J =* 7.7, 1.7 Hz, 1H), 7.29 (t, *J =* 7.7 Hz, 1H), 3.53 (s, 3H).

### Example 7.5.2. Preparation of 2-methoxy-N-(3-methyl-1-(2-(1-methylpiperidin-4-yl)ethyl)-1H-indazole-6-yl)-3-(pyridin-4-yl)benzamide (50e)

Using procedure C-method I, compound **50e** was prepared from compound **49e** and compound **9k** prepared according to Example 2.7.3 as a white solid at a 14% yield. mp 147-148°C. ¹H NMR (400 MHz, CDCl₃) δ 10.10 (s, 1H), 8.94 - 8.54 (m, 2H), 8.35 (d, *J=* 1.7 Hz, 1H), 8.28 (dd, *J =* 7.9, 1.8 Hz, 1H), 7.61 - 7.50 (m, 4H), 7.41 (t, *J =* 7.7 Hz, 1H), 6.90 (dd, *J =* 8.6, 1.7 Hz, 1H), 4.34 (t, *J =* 7.3 Hz, 2H), 3.56 (s, 3H), 3.03 - 2.86 (m, 2H), 2.54 (s, 3H), 2.33 (s, 3H), 2.14 - 1.98 (m, 2H), 1.88 (q, *J =* 7.0 Hz, 2H), 1.85 - 1.77 (m, 2H), 1.55 - 1.41 (m, 2H), 1.38 - 1.29 (m, 1H). FAB-MS [M+H]⁺ 484.

### Example 7.6. Preparation of 2-methoxy-N-(3-methyl-1-(2-(1-methylpiperidin-4-yl)ethyl)-1H-indazole-6-yl)-3-(pyridine-3-yl)benzamide (formula 1-39, 50f)

### Example 7.6.1. Preparation of 2-methoxy-3-(pyridin-3-yl)benzoic acid (49f)

Using procedure L, 3-bromo-2-methoxybenzoic acid and 3-pyridinylboronic acid were treated with BtOH in solvent H₂O to afford compound **49f** as a white solid at a 36% yield. ¹H NMR (401 MHz, MeOD) δ 8.71 (d, *J =* 2.3 Hz, 1H), 8.53 (dd, *J =* 4.9, 1.6 Hz, 1H), 8.04 (dt, *J* = 7.9, 1.9 Hz, 1H), 7.82 (dd, *J =* 7.8, 1.8 Hz, 1H), 7.56 (dd, *J* = 7.6, 1.8 Hz, 1H), 7.52 (dd, *J =* 7.9, 5.0 Hz, 1H), 7.34 - 7.26 (m, 1H), 3.50 (s, 3H).

### Example 7.6.2. Preparation of 2-methoxy-N-(3-methyl-1-(2-(1-methylpiperidin-4-yl)ethyl)-1H-indazole-6-yl)-3-(pyridin-3-yl)benzamide (50f)

Using procedure C-method I, compound **50f** was prepared from compound **49f** and compound **9k** prepared according to Example 2.7.3 as a white solid at a 56% yield. mp 105-106°C. ¹H NMR (400 MHz, CDCl₃) δ 10.13 (s, 1H), 9.01 - 8.77 (m, 1H), 8.77 - 8.55 (m, 1H), 8.35 (d, *J=* 1.7 Hz, 1H), 8.26 (dd, *J =* 7.8, 1.8 Hz, 1H), 7.95 (dt, *J =* 7.9, 1.9 Hz, 1H), 7.57 (d, *J=* 8.5 Hz, 1H), 7.53 (dd, *J =* 7.6, 1.8 Hz, 1H), 7.48 - 7.37 (m, 2H), 6.90 (dd, *J =* 8.5*,* 1.7 Hz, 1H), 4.34 (t, *J =* 7.4 Hz, 2H), 3.54 (s, 3H), 2.99 - 2.88 (m, 2H), 2.54 (s, 3H), 2.31 (s, 3H), 2.09 - 1.95 (m, 2H), 1.88 (q, *J =* 7.0 Hz, 2H), 1.84 - 1.77 (m, 2H), 1.52 - 1.40 (m, 2H), 1.38 - 1.27 (m, 1H). FAB-MS [M+H]⁺ 484.

### Example 7.7. Preparation of 2-methoxy-N-(3-methyl-1-(2-(1-methylpiperidin-4-yl)ethyl)-1H-indazole-6-yl)-4-(pyridine-4-yl)benzamide (formula 1-40, 50g)

### Example 7.7.1. Preparation of 2-methoxy-4-(pyridin-4-yl)benzoic acid (49g)

Using procedure L, 4-bromo-2-methoxybenzoic acid and 4-pyridinylboronic acid were treated with EtOH in solvent H₂O to afford compound **49g** as a white solid at a 74% yield. ¹H NMR (400 MHz, DMSO-*d*₆) δ 8.63 (dd, *J =* 4.5, 1.6 Hz, 2H), 7.75 (dd, *J =* 4.4, 1.7 Hz, 2H), 7.69 (d, *J =* 7.9 Hz, 1H), 7.41 (d, *J =* 1.6 Hz, 1H), 7.36 (dd, *J =* 7.9, 1.7 Hz, 1H), 3.89 (s, 3H).

### Example 7.7.2. Preparation of 2-methoxy-N-(3-methyl-1-(2-(1-methylpiperidin-4-yl)ethyl)-1H-indazole-6-yl)-4-(pyridin-4-yl)benzamide (50g)

Using procedure C-method I, compound **50g** was prepared from compound **49g** and compound **9k** prepared according to Example 2.7.3 as a white solid at a 42% yield. mp 138-139°C. ¹H NMR (400 MHz, CDCl₃) δ 10.01 (s, 1H), 8.71 (d, *J =* 4.9 Hz, 2H), 8.41 (d, *J =* 8.1 Hz, 1H), 8.36 (d, *J =* 1.7 Hz, 1H), 7.55 (d, *J =* 8.5 Hz, 1H), 7.53 (d, *J =* 6.1 Hz, 2H), 7.41 (dd, *J =* 8.2, 1.6 Hz, 1H), 7.25 (d, *J =* 1.2 Hz, 1H), 6.85 (dd, *J= 8.6,* 1.7 Hz, 1H), 4.33 (t, *J=* 7.4 Hz, 2H), 4.18 (s, 3H), 2.90 - 2.84 (m, 2H), 2.53 (s, 3H), 2.26 (s, 3H), 1.97 - 1.90 (m, 2H), 1.86 (q, *J =* 6.7 Hz, 2H), 1.82 - 1.75 (m, 2H), 1.43 - 1.32 (m, 3H). FAB-MS [M+H]⁺ 484.

### Example 7.8. Preparation of 2-methoxy-N-(3-methyl-1-(2-(1-methylpiperidin-4-yl)ethyl)-1H-indazole-6-yl)-4-(pyridine-3-yl)benzamide (formula 1-41, 50h)

### Example 7.8.1. Preparation of 2-methoxy-4-(pyridin-3-yl)benzoic acid (49h)

Using procedure L, 4-bromo-2-methoxybenzoic acid and 3-pyridinylboronic acid were treated with EtOH in solvent H₂O to afford compound **49h** as a white solid at a 57% yield. ¹H NMR (400 MHz, DMSO-*d*₆) δ 8.94 (d, *J =* 2.3 Hz, 1H), 8.58 (dd, *J =* 4.8, 1.6 Hz, 1H), 8.13 (dt, *J* = 8.0, 2.0 Hz, 1H), 7.71 (d, *J* = 7.9 Hz, 1H), 7.48 (dd, *J* = 8.0, 4.8 Hz, 1H), 7.38 (d, *J* = 1.4 Hz, 1H), 7.31 (dd, *J* = 8.0, 1.6 Hz, 1H), 3.89 (s, 3H).

### Example 7.8.2. Preparation of 2-methoxy-N-(3-methyl-1-(2-(1-methylpiperidin-4-yl)ethyl)-1H-indazole-6-yl)-4-(pyridin-3-yl)benzamide (50h)

Using procedure C-method I, compound **50h** was prepared from compound **49h** and compound **9k** prepared according to Example 2.7.3 as a white solid at a 39% yield. mp 149-150°C. ¹H NMR (400 MHz, CDCl₃) δ 10.02 (s, 1H), 8.96 - 8.78 (m, 1H), 8.73 - 8.60 (m, 1H), 8.40 (d, *J =* 8.1 Hz, 1H), 8.36 (d, *J =* 1.6 Hz, 1H), 7.92 (dt, *J =* 8.0, 1.8 Hz, 1H), 7.55 (d, *J =* 8.5 Hz, 1H), 7.41 (dd, *J =* 8.0, 4.8 Hz, 1H), 7.36 (dd, *J =* 8.1, 1.6 Hz, 1H), 7.22 (d, *J=* 1.7 Hz, 1H), 6.85 (dd, *J* = 8.6*,* 1.7 Hz, 1H), 4.33 (t, *J =* 7.4 Hz, 2H), 4.17 (s, 3H), 2.98 - 2.89 (m, 2H), 2.53 (s, 3H), 2.31 (s, 3H), 2.07 - 1.98 (m, 2H), 1.87 (q, *J =* 6.9 Hz, 2H), 1.83 - 1.75 (m, 2H), 1.50 - 1.38 (m, 2H), 1.35 - 1.27 (m, 1H). FAB-MS [M+H]⁺ 484.

### Example 8. Preparation of phenoxy, benzyloxy benzamide derivatives

A method for producing phenoxy, benzyloxy benzamide derivatives is shown by the following scheme 7. (a) phenol, K₂CO₃, DMF, 140°C, 6 hours; (b) R₂Br, K₂CO₃, DMF, 40°C, 3 hours; (c) [Method A] NaOH, EtOH, H₂O, 120°C, 3 hours, [Method B] NaClO₂, NaH₂PO₄, 27% H₂O₂ in H₂O, ACN, from 0°C to room temperature, 2 hours; (d) **9k,** EDC·HCl, HOBt, Et₃N, MC, room temperature, 15 hours.

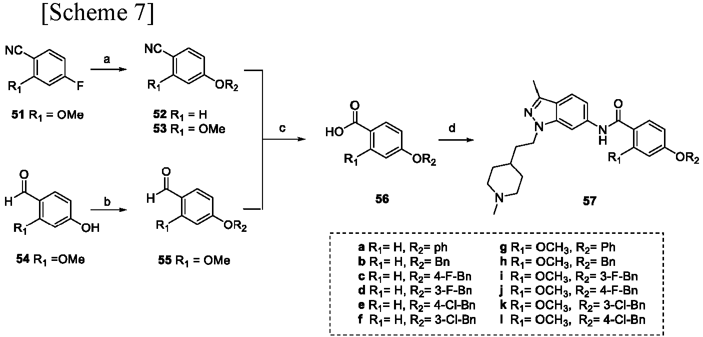

### Example 8.1. Preparation of N-(3-methyl-1-(2-(1-methylpiperidin-4-yl)ethyl)-1H-indazole-6-yl)-4-phenoxybenzamide (formula 1-42, 57a)

Using procedure C-method I, compound **57a** was prepared from compound **56a** and compound **9k** prepared according to Example 2.7.3 as a white solid at a 67% yield. mp 136-137°C. ¹H NMR (400 MHz, CDCl₃) δ 8.20 (d, *J* = 1.8 Hz, 1H), 7.89 (s, 1H), 7.87 (d, *J =* 8.8 Hz, 2H), 7.57 (d, *J =* 8.5 Hz, 1H), 7.40 (dd, *J =* 8.6, 7.4 Hz, 2H), 7.22 - 7.17 (m, 1H), 7.09 - 7.04 (m, 4H), 6.90 (dd, *J= 8.6,* 1.7 Hz, 1H), 4.31 (t, *J =* 7.5 Hz, 2H), 2.87 - 2.76 (m, 2H), 2.54 (s, 3H), 2.23 (s, 3H), 1.94 - 1.82 (m, 4H), 1.79 - 1.72 (m, 2H), 1.39 - 1.28 (m, 3H). FAB-MS [M+H]⁺ 469.

### Example 8.2. Preparation of 4-(benzyloxy)-N-(3-methyl-1-(2-(1-methylpiperidin-4-yl)ethyl)-1H-indazole-6-yl)benzamide (formula 1-43, 57b)

Using procedure C-method I, compound **57b** was prepared from compound **56b** and compound **9k** prepared according to Example 2.7.3 as a white solid at a 73% yield. mp 150-151°C. ¹H NMR (400 MHz, CDCl₃) δ 8.16 (d, *J =* 1.9 Hz, 1H), 8.01 (s, 1H), 7.88 (d, *J =* 8.8 Hz, 2H), 7.56 (d, *J =* 8.5 Hz, 1H), 7.46 - 7.32 (m, 5H), 7.06 (d, *J =* 8.8 Hz, 2H), 6.96 (dd, *J=* 8.5, 1.7 Hz, 1H), 5.14 (s, 2H), 4.31 (t, *J =* 7.1 Hz, 2H), 3.09 - 3.00 (m, 2H), 2.53 (s, 3H), 2.41 (s, 3H), 2.24 - 2.12 (m, 2H), 1.89 (q, *J=* 7.0 Hz, 2H), 1.84 - 1.78 (m, 2H), 1.63 - 1.52 (m, 2H), 1.38 - 1.31 (m, 1H). FAB-MS [M+H]⁺ 483.

### Example 8.3. Preparation of 4-((4-fluorobenzyl)oxy)-N-(3-methyl-1-(2-(1-methylpiperidin-4-yl)ethyl)-1H-indazole-6-yl)benzamide (formula 1-44, 57c)

Using procedure C-method I, compound **57c** was prepared from compound **56c** and compound **9k** prepared according to Example 2.7.3 as a white solid at a 27% yield. mp 149-150°C. ¹H NMR (400 MHz, CDCl₃) δ 8.19 (s, 1H), 7.91 (s, 1H), 7.87 (d, *J =* 8.6 Hz, 2H), 7.56 (d, *J =* 8.5 Hz, 1H), 7.41 (dd, *J =* 8.4, 5.3 Hz, 2H), 7.12 - 7.02 (m, 4H), 6.90 (dd, *J=* 8.5, 1.7 Hz, 1H), 5.09 (s, 2H), 4.31 (t, *J =* 7.5 Hz, 2H), 2.90 - 2.80 (m, 2H), 2.53 (s, 3H), 2.24 (s, 3H), 1.90 - 1.80 (m, 4H), 1.77 - 1.70 (m, 2H), 1.38 - 1.29 (m, 3H). FAB-MS [M+H]⁺ 501.

### Example 8.4. Preparation of 4-((3-fluorobenzyl)oxy)-N-(3-methyl-1-(2-(1-methylpiperidin-4-yl)ethyl)-1H-indazol-6-yl)benzamide (formula 1-45, 57d)

Using procedure C-method I, compound **57d** was prepared from compound **56d** and compound **9k** prepared according to Example 2.7.3 as a white solid at a 38% yield. mp 144-145°C. ¹H NMR (400 MHz, CDCl₃) δ 8.19 (d, *J* = 1.7 Hz, 1H), 7.89 (s, 1H), 7.87 (d, *J =* 8.7 Hz, 2H), 7.56 (d, *J =* 8.5 Hz, 1H), 7.36 (ddd, *J =* 7.9, 7.9, 5.7 Hz, 1H), 7.20 (ddd, *J* = 7.5, 1.1, 1.1 Hz, 1H), 7.18 - 7.14 (m, 1H), 7.08 - 7.00 (m, 3H), 6.90 (dd, *J =* 8.6, 1.8 Hz, 1H), 5.13 (s, 2H), 4.31 (t, *J =* 7.4 Hz, 2H), 2.88 - 2.81 (m, 2H), 2.53 (s, 3H), 2.24 (s, 3H), 1.92 - 1.83 (m, 4H), 1.76 - 1.73 (m, 2H), 1.41 - 1.28 (m, 3H). FAB-MS [M+H]⁺ 501.

### Example 8.5. Preparation of 4-((4-chlorobenzyl)oxy)-N-(3-methyl-1-(2-(1-methylpiperidin-4-yl)ethyl)-1H-indazole-6-yl)benzamide (formula 1-46, 57e)

Using procedure C-method I, compound **57e** was prepared from compound **56e** and compound **9k** prepared according to Example 2.7.3 as a white solid at a 24% yield. mp 148-149°C. ¹H NMR (400 MHz, CDCl₃) δ 8.18 (d, *J =* 1.7 Hz, 1H), 7.95 (s, 1H), 7.86 (d, *J =* 8.6 Hz, 2H), 7.55 (d, *J =* 8.5 Hz, 1H), 7.40 - 7.34 (m, 4H), 7.03 (d, *J =* 8.7 Hz, 2H), 6.91 (dd, *J =* 8.5*,* 1.7 Hz, 1H), 5.09 (s, 2H), 4.30 (t, *J =* 7.3 Hz, 2H), 2.93 - 2.78 (m, 2H), 2.53 (s, 3H), 2.24 (s, 3H), 1.93 - 1.80 (m, 4H), 1.79 - 1.71 (m, 2H), 1.41 - 1.28 (m, 3H). FAB-MS [M+H]⁺ 517.

### Example 8.6. Preparation of 4-((3-chlorobenzyl)oxy)-N-(3-methyl-1-(2-(1-methylpiperidin-4-yl)ethyl)-1H-indazole-6-yl)benzamide (formula 1-47, 57f)

Using procedure C-method I, compound **57f** was prepared from compound **56f** and compound **9k** prepared according to Example 2.7.3 as a white solid at a 53% yield. mp 136-137°C. ¹H NMR (400 MHz, CDCl₃) δ 8.19 (d, *J* = 1.7 Hz, 1H), 7.91 (s, 1H), 7.87 (d, *J* = 8.8 Hz, 2H), 7.56 (d, *J=* 8.5 Hz, 1H), 7.44 (t, *J=* 2.1 Hz, 1H), 7.34 - 7.29 (m, 3H), 7.05 (d, *J =* 8.8 Hz, 2H), 6.90 (dd, *J =* 8.5, 1.7 Hz, 1H), 5.11 (s, 2H), 4.31 (t, *J* = 7.4 Hz, 2H), 2.88 - 2.78 (m, 2H), 2.53 (s, 3H), 2.24 (s, 3H), 1.92 - 1.84 (m, 4H), 1.77 - 1.72 (m, 2H), 1.40 - 1.27 (m, 3H). FAB-MS [M+H]⁺ 517.

### Example 8.7. Preparation of 2-methoxy-N-(3-methyl-1-(2-(1-methylpiperidin-4-yl)ethyl)-1H-indazole-6-yl)-4-phenoxybenzamide (formula 1-48, 57g)

### Example 8.7.1. Preparation of 2-methoxy-4-phenoxybenzonitrile (53)

A mixture of compound **51** (1.0 eq), phenol (1.5 eq) and K₂CO₃ (2.0 eq) in DMF (4 mL) was heated at 140°C for 6 hours. The reaction mixture was cooled to room temperature, quenched with water, and extracted with EA. The organic layer was dried over anhydrous sodium sulfate and concentrated. The residue was purified by column chromatography on silica (Hex:EA = 10:1) to obtain compound **53** as a white solid at a 95% yield. ¹H NMR (400 MHz, CDCl₃) δ 7.45 (d, *J =* 8.6 Hz, 1H), 7.40 (dd, *J =* 8.5, 7.4 Hz, 2H), 7.24 - 7.19 (m, 1H), 7.06 (dd, *J=* 8.6, 1.2 Hz, 2H), 6.56 (d, *J=* 2.2 Hz, 1H), 6.48 (dd, *J=* 8.6, 2.2 Hz, 1H), 3.85 (s, 3H).

### Example 8.7.2. Preparation of 2-methoxy-4-phenoxybenzoic acid (56g)

Using procedure K, compound **56g** was prepared from compound **53** as a white solid at an 88% yield. ¹H NMR (400 MHz, CDCl₃) δ 10.49 (br s, 1H), 8.10 (d, *J =* 8.7 Hz, 1H), 7.47 - 7.37 (m, 2H), 7.24 - 7.20 (m, 1H), 7.12 - 7.03 (m, 2H), 6.65 (d, *J=* 2.2 Hz, 1H), 6.60 (dd, *J =* 8.8, 2.2 Hz, 1H), 4.00 (s, 3H).

### Example 8.7.3. Preparation of 2-methoxy-N-(3-methyl-1-(2-(1-methylpiperidin-4-yl)ethyl)-1H-indazole-6-yl)-4-phenoxybenzamide (57g)

Using procedure C-method I, compound **57g** was prepared from compound **56g** and compound **9k** prepared according to Example 2.7.3 as a white solid at a 57% yield. mp 84-85°C. ¹H NMR (400 MHz, CDCl₃) δ 9.91 (s, 1H), 8.34 (d, *J =* 1.6 Hz, 1H), 8.24 (d, *J =* 8.8 Hz, 1H), 7.53 (d, *J =* 8.5 Hz, 1H), 7.40 (dd, *J =* 8.5, 7.4 Hz, 2H), 7.24 - 7.17 (m, 1H), 7.09 (dd, *J=* 8.6*,* 1.1 Hz, 2H), 6.82 (dd, *J=* 8.5, 1.7 Hz, 1H), 6.70 (d, *J= 2.2* Hz, 1H), 6.65 (dd, *J=* 8.7, 2.2 Hz, 1H), 4.31 (t, *J =* 7.5 Hz, 2H), 4.03 (s, 3H), 2.86 - 2.79 (m, 2H), 2.53 (s, 3H), 2.23 (s, 3H), 1.91 - 1.82 (m, 4H), 1.79 - 1.75 (m, 2H), 1.40 - 1.27 (m, 3H). FAB-MS [M+H]⁺ 499.

### Example 8.8. Preparation of 4-(benzyloxy)-2-methoxy-N-(3-methyl-1-(2-(1-methylpiperidin-4-yl)ethyl)-1H-indazole-6-yl)benzamide (formula 1-49, 57h)

### Example 8.8.1. Preparation of 4-(benzyloxy)-2-methoxybenzaldehyde (55h)

Using procedure H-method I, compound **55h** was prepared from compound **54** and benzyl bromide as a white solid at an 85% yield. ¹H NMR (400 MHz, CDCl₃) δ 10.28 (d, *J =* 0.7 Hz, 1H), 7.80 (d, *J =* 8.6 Hz, 1H), 7.44 - 7.32 (m, 5H), 6.61 (ddd, *J* = 8.7, 2.3, 0.8 Hz, 1H), 6.52 (d, *J=* 2.2 Hz, 1H), 5.12 (s, 2H), 3.87 (s, 3H).

### Example 8.8.2. Preparation of 4-(benzyloxy)-2-methoxybenzoic acid (56h)

Using procedure J, compound **56h** was prepared from compound **55h** as a white solid at a 70% yield. ¹H NMR (400 MHz, CDCl₃) δ 10.50 (br s, 1H), 8.13 (d, *J =* 8.8 Hz, 1H), 7.44 - 7.34 (m, 5H), 6.71 (dd, *J =* 8.8, 2.3 Hz, 1H), 6.61 (d, *J =* 2.3 Hz, 1H), 5.12 (s, 2H), 4.01 (s, 3H).

### Example 8.8.3. Preparation of 4-(benzyloxy)-2-methoxy-N-(3-methyl-1-(2-(1-methylpiperidin-4-yl)ethyl)-1H-indazole-6-yl)benzamide (57h)

Using procedure C-method I, compound **57h** was prepared from compound **56h** and compound **9k** prepared according to Example 2.7.3 as a white solid at a 66% yield. mp 123-124°C. ¹H NMR (400 MHz, CDCl₃) δ 9.92 (s, 1H), 8.35 (s, 1H), 8.26 (d, *J =* 8.7 Hz, 1H), 7.53 (d, *J=* 8.5 Hz, 1H), 7.46 - 7.33 (m, 5H), 6.81 (dd, *J=* 8.6, 1.7 Hz, 1H), 6.75 (dd, *J=* 8.8, 2.3 Hz, 1H), 6.63 (d, *J=* 2.2 Hz, 1H), 5.13 (s, 2H), 4.32 (t, *J =* 7.4 Hz, 2H), 4.04 (s, 3H), 2.98 - 2.88 (m, 2H), 2.53 (s, 3H), 2.31 (s, 3H), 2.05 - 1.95 (m, 2H), 1.87 (q, *J =* 7.1 Hz, 2H), 1.83 - 1.76 (m, 2H), 1.50 - 1.39 (m, 2H), 1.36 - 1.26 (m, 1H). FAB-MS [M+H]⁺ 513.

### Example 8.9. Preparation of 4-((3-fluorobenzyl)oxy)-2-methoxy-N-(3-methyl-1-(2-(1-methylpiperidin-4-yl)ethyl)-1H-indazole-6-yl)benzamide (formula 1-50, 57i)

### Example 8.9.1. Preparation of 4-((3-fluorobenzyl)oxy)-2-methoxybenzaldehyde (55i)

Using procedure H-method I, compound **55i** was prepared from compound **54** and 3-fluorobenzyl bromide as a white solid at an 82% yield. ¹H NMR (400 MHz, DMSO-*D*₆) δ 10.14 (d, *J =* 0.7 Hz, 1H), 7.63 (d, *J =* 8.7 Hz, 1H), 7.48 - 7.37 (m, 1H), 7.32 - 7.26 (m, 2H), 7.20 - 7.11 (m, 1H), 6.77 (d, *J=* 2.3 Hz, 1H), 6.70 (ddd, *J* = 8.7, 2.2, 0.8 Hz, 1H), 5.22 (s, 2H), 3.86 (s, 3H).

### Example 8.9.2. Preparation of 4-((3-fluorobenzyl)oxy)-2-methoxybenzoic acid (56i)

Using procedure J, compound **56i** was prepared from compound **55i** as a white solid at a 90% yield. ¹H NMR (400 MHz, CDCl₃) δ 10.46 (br s, 1H), 8.14 (d, *J =* 8.8 Hz, 1H), 7.37 (td, *J =* 8.0*,* 5.8 Hz, 1H), 7.18 (d, *J =* 7.6 Hz, 1H), 7.14 (dt, *J =* 9.5, 2.1 Hz, 1H), 7.04 (td, *J =* 8.3, 2.3 Hz, 1H), 6.69 (dd, *J =* 8.8, 2.3 Hz, 1H), 6.61 (d, *J =* 2.3 Hz, 1H), 5.12 (s, 2H), 4.03 (s, 3H).

### Example 8.9.3. Preparation of 4-((3-fluorobenzyl)oxy)-2-methoxy-N-(3-methyl-1-(2-(1-methylpiperidin-4-yl)ethyl)-1H-indazole-6-yl)benzamide (57i)

Using procedure C-method I, compound **57i** was prepared from compound **56i** and compound **9k** prepared according to Example 2.7.3 as a white solid at a 49% yield. mp 145-146°C. ¹H NMR (400 MHz, CDCl₃) δ 9.91 (s, 1H), 8.34 (d, *J =* 1.3 Hz, 1H), 8.26 (d, *J =* 8.8 Hz, 1H), 7.53 (d, *J =* 8.5 Hz, 1H), 7.37 (td, *J =* 8.0, 5.8 Hz, 1H), 7.20 (dt, *J =* 7.6, 1.2 Hz, 1H), 7.17 (dt, *J =* 9.6, 2.2 Hz, 1H), 7.04 (ddt, *J =* 8.7, 7.9, 1.6 Hz, 1H), 6.81 (dd, *J=* 8.5, 1.7 Hz, 1H), 6.72 (dd, *J=* 8.8, 2.3 Hz, 1H), 6.64 (d, *J =* 2.3 Hz, 1H), 5.13 (s, 2H), 4.32 (t, *J=* 7.3 Hz, 2H), 4.06 (s, 3H), 3.02 - 2.88 (m, 2H), 2.53 (s, 3H), 2.35 (s, 3H), 2.14 - 1.99 (m, 2H), 1.87 (q, *J=* 7.1 Hz, 2H), 1.84 - 1.78 (m, 2H), 1.56 - 1.45 (m, 2H), 1.38 - 1.29 (m, 1H). FAB-MS [M+H]⁺ 531.

### Example 8.10. Preparation of 4-((4-fluorobenzyl)oxy)-2-methoxy-N-(3-methyl-1-(2-(1-methylpiperidin-4-yl)ethyl)-1H-indazole-6-yl)benzamide (formula 1-51, 57j)

### Example 8.10.1. Preparation of 4-((4-fluorobenzyl)oxy)-2-methoxybenzaldehyde (55j)

Using procedure H-method I, compound **55j** was prepared from compound **54** and 4-fluorobenzyl bromide as a white solid at a 75% yield. ¹H NMR (400 MHz, CDCl₃) δ 10.28 (s, 1H), 7.80 (d, *J =* 8.7 Hz, 1H), 7.40 (dd, *J =* 8.2, 5.3 Hz, 2H), 7.08 (t, *J =* 8.7 Hz, 2H), 6.60 (dd, *J =* 8.7, 2.1 Hz, 1H), 6.51 (d, *J=* 2.2 Hz, 1H), 5.07 (s, 2H), 3.88 (s, 3H).

### Example 8.10.2. Preparation of 4-((4-fluorobenzyl)oxy)-2-methoxybenzoic acid (56j)

Using procedure J, compound **56j** was prepared from compound **55j** as a white solid at a 90% yield. ¹H NMR (400 MHz, CDCl₃) δ 10.46 (br s, 1H), 8.14 (d, *J =* 8.8 Hz, 1H), 7.40 (dd, *J =* 8.5, 5.4 Hz, 2H), 7.09 (t, *J =* 8.6 Hz, 2H), 6.70 (dd, *J =* 8.8, 2.3 Hz, 1H), 6.60 (d, *J* = 2.3 Hz, 1H), 5.08 (s, 2H), 4.02 (s, 3H).

### Example 8.10.3. Preparation of 4-((4-fluorobenzyl)oxy)-2-methoxy-N-(3-methyl-1-(2-(1-methylpiperidin-4-yl)ethyl)-1H-indazole-6-yl)benzamide (57j)

Using procedure C-method I, compound **57j** was prepared from compound **56j** and compound **9k** prepared according to Example 2.7.3 as a white solid at a 41% yield. mp 126-127°C. ¹H NMR (400 MHz, CDCl₃) δ 9.91 (s, 1H), 8.34 (d, *J =* 1.0 Hz, 1H), 8.26 (d, *J =* 8.8 Hz, 1H), 7.53 (dd, *J=* 8.5, 0.7 Hz, 1H), 7.42 (dd, *J =* 8.7, 5.3 Hz, 2H), 7.09 (t, *J =* 8.7 Hz, 2H), 6.81 (dd, *J =* 8.5, 1.7 Hz, 1H), 6.73 (dd, *J =* 8.8, 2.3 Hz, 1H), 6.62 (d, *J* = 2.3 Hz, 1H), 5.09 (s, 2H), 4.33 (t, *J =* 7.2 Hz, 2H), 4.05 (s, 3H), 3.10 - 2.92 (m, 2H), 2.53 (s, 3H), 2.39 (s, 3H), 2.21 - 2.08 (m, 2H), 1.92 - 1.80 (m, 4H), 1.61 - 1.50 (m, 2H), 1.39 - 1.28 (m, 1H). FAB-MS [M+H]⁺ 531.

### Example 8.11. Preparation of 4-((3-chlorobenzyl)oxy)-2-methoxy-N-(3-methyl-1-(2-(1-methylpiperidin-4-yl)ethyl)-1H-indazole-6-yl)benzamide (formula 1-52, 57k)

### Example 8.11.1. Preparation of 4-((3-chlorobenzyl)oxy)-2-methoxybenzaldehyde (55k)

Using procedure H-method I, compound **55k** was prepared from compound **54** and 3-chlorobenzyl bromide as a white solid at a 88% yield. ¹H NMR (400 MHz, CDCl₃) δ 10.29 (d, *J=* 0.7 Hz, 1H), 7.81 (d, *J=* 8.6 Hz, 1H), 7.46 - 7.41 (m, 1H), 7.34 - 7.27 (m, 3H), 6.59 (dd, *J=* 8.7, 2.2 Hz, 1H), 6.52 (d, *J =* 2.2 Hz, 1H), 5.09 (s, 2H), 3.89 (s, 3H).

### Example 8.11.2. Preparation of 4-((3-chlorobenzyl)oxy)-2-methoxybenzoic acid (56k)

Using procedure J, compound **56k** was prepared from compound **55k** as a white solid in 94% yield. ¹H NMR (400 MHz, CDCl₃) δ 10.44 (br s, 1H), 8.14 (d, *J =* 8.8 Hz, 1H), 7.44 - 7.41 (m, 1H), 7.35 - 7.27 (m, 3H), 6.69 (dd, *J = 8.8,* 2.3 Hz, 1H), 6.61 (d, *J* = 2.3 Hz, 1H), 5.09 (s, 2H), 4.03 (s, 3H).

### Example 8.11.3. Preparation of 4-((3-chlorobenzyl)oxy)-2-methoxy-N-(3-methyl-1-(2-(1-methylpiperidin-4-yl)ethyl)-1H-indazole-6-yl)benzamide (57k)

Using procedure C-method I, compound **57k** was prepared from compound **56k** and compound **9k** prepared according to Example 2.7.3 as a white solid at a 62% yield. mp 152-153°C. ¹H NMR (400 MHz, CDCl₃) δ 9.91 (s, 1H), 8.33 (d, *J =* 1.6 Hz, 1H), 8.26 (d, *J =* 8.8 Hz, 1H), 7.53 (dd, *J =* 8.5*,* 0.7 Hz, 1H), 7.47 - 7.44 (m, 1H), 7.34 - 7.30 (m, 3H), 6.81 (dd, *J=* 8.6, 1.7 Hz, 1H), 6.72 (dd, *J =* 8.8, 2.3 Hz, 1H), 6.64 (d, *J =* 2.3 Hz, 1H), 5.10 (s, 2H), 4.33 (t, *J* = 7.1 Hz, 2H), 4.06 (s, 3H), 3.10 - 2.94 (m, 2H), 2.53 (s, 3H), 2.40 (s, 3H), 2.26 - 2.09 (m, 2H), 1.91 - 1.81 (m, 4H), 1.63 - 1.50 (m, 2H), 1.38 - 1.29 (m, 1H). FAB-MS [M+H]⁺ 547.

### Example 8.12. Preparation of 4-((4-chlorobenzyl)oxy)-2-methoxy-N-(3-methyl-1-(2-(1-methylpiperidin-4-yl)ethyl)-1H-indazole-6-yl)benzamide (formula 1-53, 57l)

### Example 8.12.1. Preparation of 4-((4-chlorobenzyl)oxy)-2-methoxybenzaldehyde (55l)

Using procedure H-method I, compound **55l** was prepared from compound **54** and 4-chlorobenzyl bromide as a white solid at a 90% yield. ¹H NMR (400 MHz, CDCl₃) δ 10.28 (d, *J=* 0.7 Hz, 1H), 7.80 (d, *J =* 8.6 Hz, 1H), 7.41 - 7.32 (m, 4H), 6.58 (dd, *J* = 8.7, 2.2 Hz, 1H), 6.51 (d, *J=* 2.2 Hz, 1H), 5.08 (s, 2H), 3.88 (s, 3H).

### Example 8.12.2. Preparation of 4-((4-chlorobenzyl)oxy)-2-methoxybenzoic acid (56l)

Using procedure J, compound **56l** was prepared from compound **55l** as a white solid at a 90% yield. ¹H NMR (400 MHz, CDCl₃) δ 10.45 (br s, 1H), 8.13 (d, *J =* 8.8 Hz, 1H), 7.43 - 7.30 (m, 4H), 6.69 (dd, *J =* 8.8, 2.3 Hz, 1H), 6.59 (d, *J =* 2.3 Hz, 1H), 5.08 (s, 2H), 4.02 (s, 3H).

### Example 8.12.3. Preparation of 4-((4-chlorobenzyl)oxy)-2-methoxy-N-(3-methyl-1-(2-(1-methylpiperidin-4-yl)ethyl)-1H-indazole-6-yl)benzamide (57l)

Using procedure C-method I, compound **57l** was prepared from compound **56k** and compound **9k** prepared according to Example 2.7.3 as a white solid at a 55% yield. mp 134-135°C. ¹H NMR (400 MHz, CDCl₃) δ 9.90 (s, 1H), 8.33 (d, *J=* 1.6 Hz, 1H), 8.25 (d, *J =* 8.8 Hz, 1H), 7.53 (d, *J=* 8.5 Hz, 1H), 7.41 - 7.34 (m, 4H), 6.81 (dd, *J=* 8.5, 1.7 Hz, 1H), 6.72 (dd, *J =* 8.8, 2.3 Hz, 1H), 6.62 (d, *J =* 2.3 Hz, 1H), 5.09 (s, 2H), 4.33 (t, *J* = 7.0 Hz, 2H), 4.05 (s, 3H), 3.21 - 2.96 (m, 2H), 2.52 (s, 3H), 2.44 (s, 3H), 2.34 - 2.11 (m, 2H), 1.93 - 1.81 (m, 4H), 1.71 - 1.52 (m, 2H), 1.41 - 1.29 (m, 1H). FAB-MS [M+H]⁺ 547.

### Experimental Example 1. Screening for cell viability in breast cancer cells

A HER2 positive breast cancer (HER2+ BC) cell line, JIMT-1 (cell seeding numbers: 1.0 (JIMT) x 10⁴ cells/wells (confluency≤30%)) and a triple-negative breast cancer (TNBC) cell line, MDA-MB-231 (cell seeding numbers: 1.0 (M231) x 10⁴ cells/wells (confluency≤30%) were used in the experiment.

The above cell lines were cultured in Dulbecco's Modified Eagle's Medium (DMEM) or Eagle's minimum Essential Medium (MEM) each containing 10% fetal bovine serum (FBS), streptomycin-penicillin (100 U/ml), and Fungizone (0.625 µg/ml), respectively under 5% CO₂, 37°C environment.

The JIMT-1 cell line and MDA-MB-231 cell line were treated with the indazole derivatives (formulas 1-1 to 1-53) of the present disclosure at a concentration of 10 µM each for 72 hours, and then cell viability was measured using the MTS assay technique. The control group was treated with DMSO as an untreated group (0 µM) of the indazole derivative of the present disclosure.

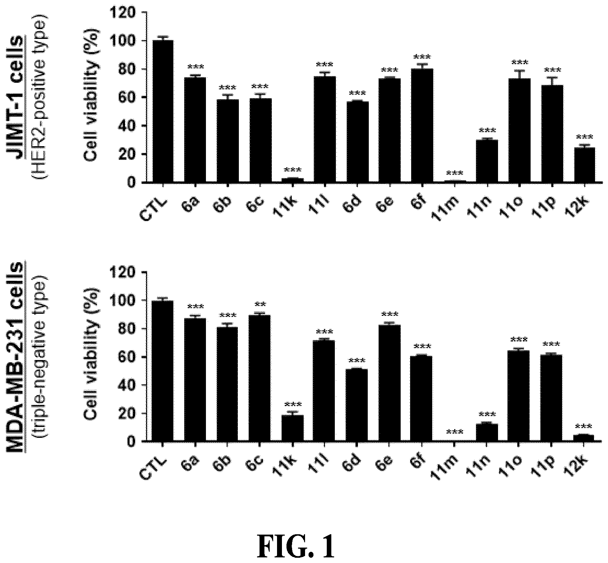

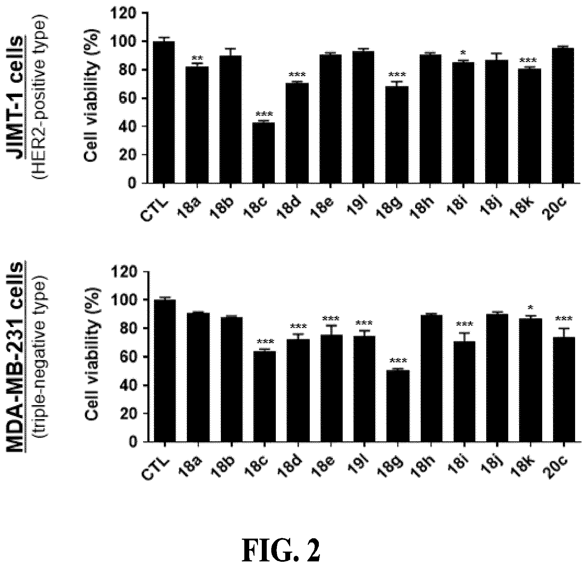

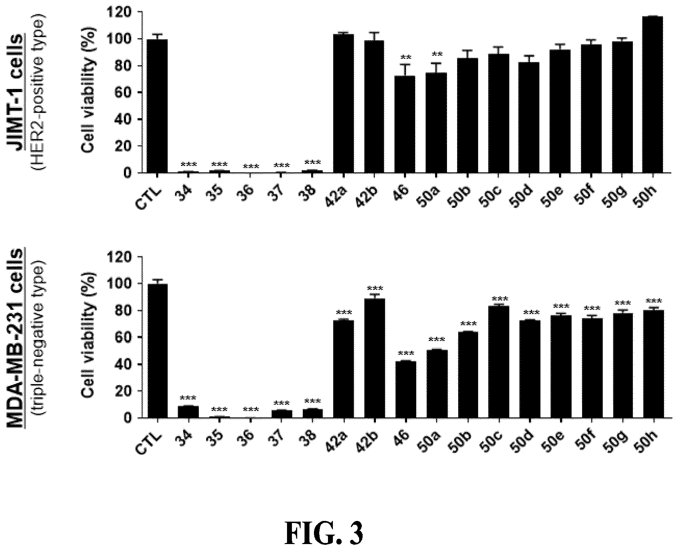

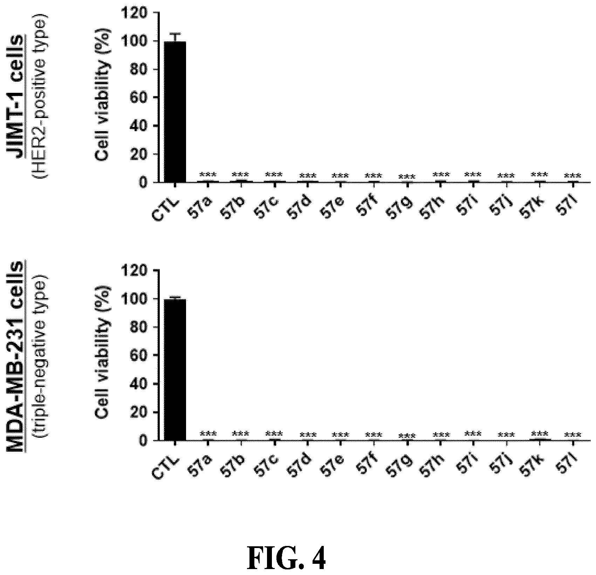

Specifically, cells were attached to 96 well plates for 24 hours, and then treated with the indazole derivatives for 72 hours. Thereafter, the cells were chromogenized with MTS for 4 hours, and the absorbance was measured at 490 nm using a Spectramax Plus384 microplate analyzer. The results are shown in FIGS. 1 to 4. The significance between the control group and the experimental group was verified using an unpaired Students t-test (*p<0.01; DMSO control vs indazole series).

The results show that most of the indazole derivatives inhibited cell viability in the JIMT-1 cell line and MDA-MB-231 cell line compared to the control group (FIGS. 1 to 4).

### Experimental Example 2. Evaluation for in vitro anticancer efficacy

### Experimental Example 2.1. Confirming on cell viability by concentration

HER2-positive breast cancer cell lines, BT474 (cell seeding numbers: 2.0 (JIMT) x 10⁴ cells/wells (confluency≤30%)) sensitive to trastuzumab, and JIMT-1 (cell seeding numbers: 1.0 (JIMT) x 10⁴ cells/wells (confluency≤30%)) resistant to trastuzumab, and a triple-negative breast cancer cell line, MDA-MB-231 (cell seeding numbers: 1.0 (M231) x 10⁴ cells/wells (confluency≤30%)) were used.

The BT474, JIMT-1, and MDA-MB-231 cell lines were treated with compound 18c (formula 1-16) and compound 11k (formula 1-7) at concentrations of 0 (DMSO), 0.1, 0.5, 1, 5, 10, and 20 µM, respectively, for 72 hours, and then cell viability was measured using the MTS assay technique as explained by the description of Experimental Example 1. The cell viability experiment was performed independently three times, and the significance was verified by a One-Way Anova, Bonferroni post-hoc test (*p<0.01; DMSO control vs. compound 18c or compound 11k).

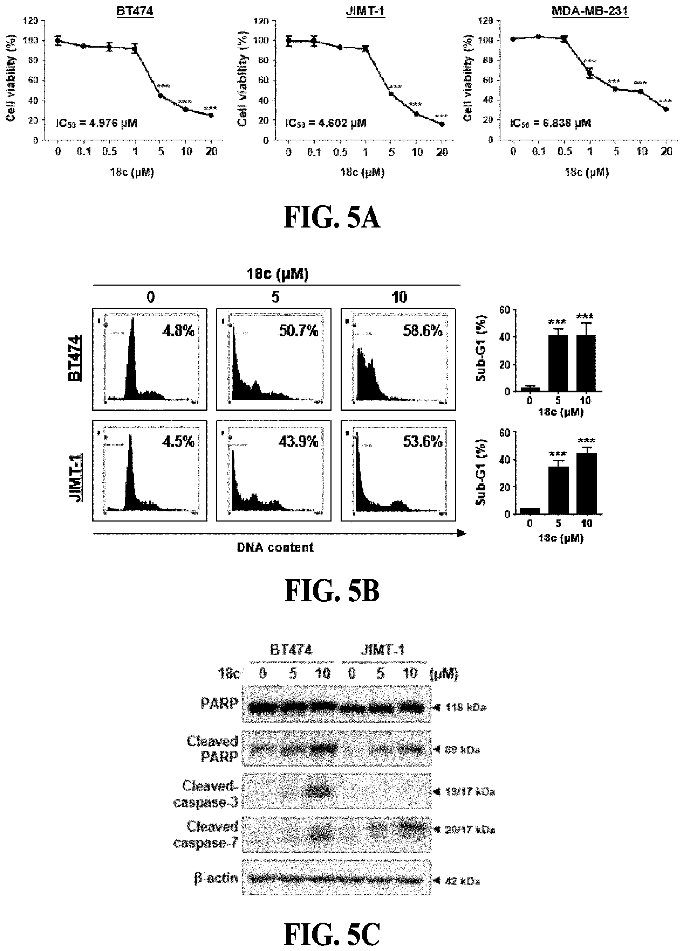

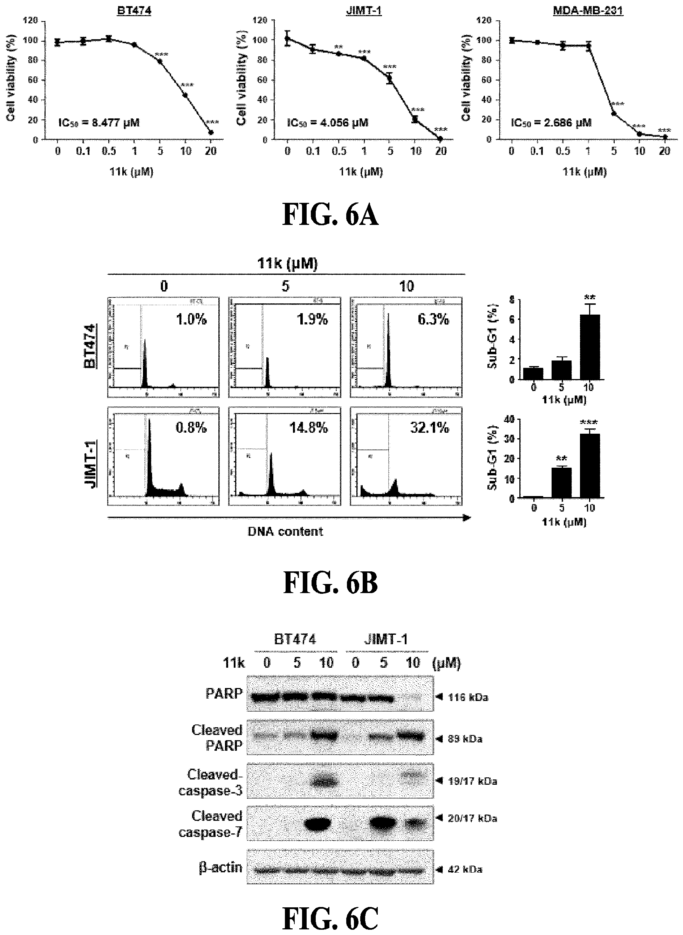

The result shows that cell viability was significantly reduced in a concentration-dependent manner in all three breast cancer cell lines. Specifically, the IC₅₀ values of compound 18c were 4.976 µM (BT474), 4.602 µM (JIMT-1), and 6.838 µM (MDA-MB-231), respectively (FIG. 5A), and the IC₅₀ values of compound 11k were 8.477 µM (BT474), 4.056 µM (JIMT-1), and 2.686 µM (MDA-MB-231), respectively (FIG. 6A), these values are very low.

In addition, the JIMT-1 and MDA-MB-231 cell lines were treated with compound 11m (formula 1-9), 11n (formula 1-10) and compound 12k (formula 1-13) at concentrations of 0 (DMSO), 0.1, 1, 5, 10, and 20 µM, respectively, for 72 hours, and then cell viability was measured.

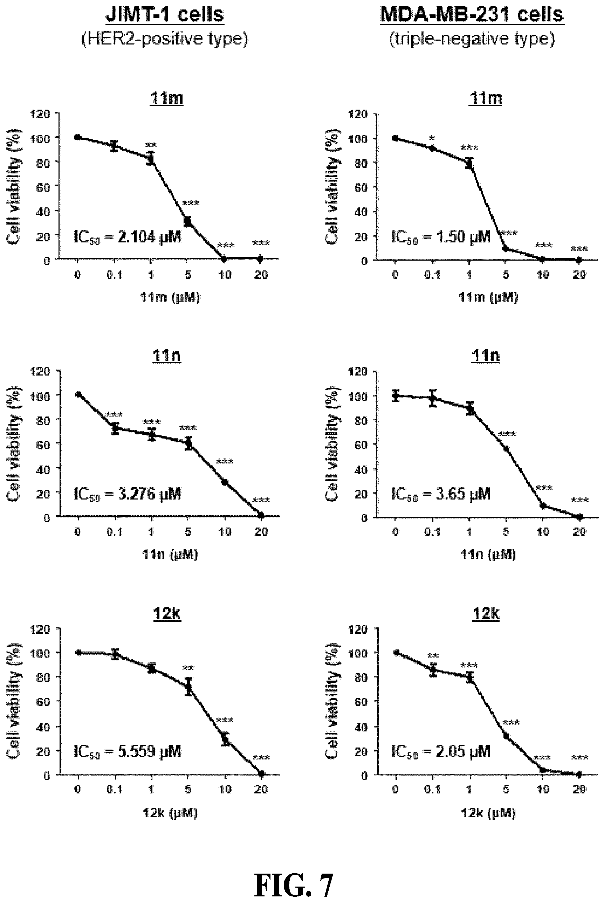

The result shows that all of the three compounds decreased cell viability in a concentration-dependent manner. Specifically, the IC₅₀ values of compound 11m were 2.104 (JIMT-1) and 1.50 (MDA-MB-231), respectively, the IC₅₀ values of compound 11n were 3.276 µM (JIMT-1) and 3.65 µM (MDA-MB-231), respectively, and the IC₅₀ values of compound 12k were 5.559 µM (JIMT-1) and 2.05 µM (MDA-MB-231), respectively (FIG. 7).

Meanwhile, triple-negative breast cancer cell lines MDA-MB-231 and BT549, and a mouse breast cancer cell line 4T1 were treated with compound 38 (formula 1-30) at concentrations of 0 (DMSO), 0.1, 0.5, 1, 2, 5, 10, and 20 µM for 72 hours, and then cell viability was measured.

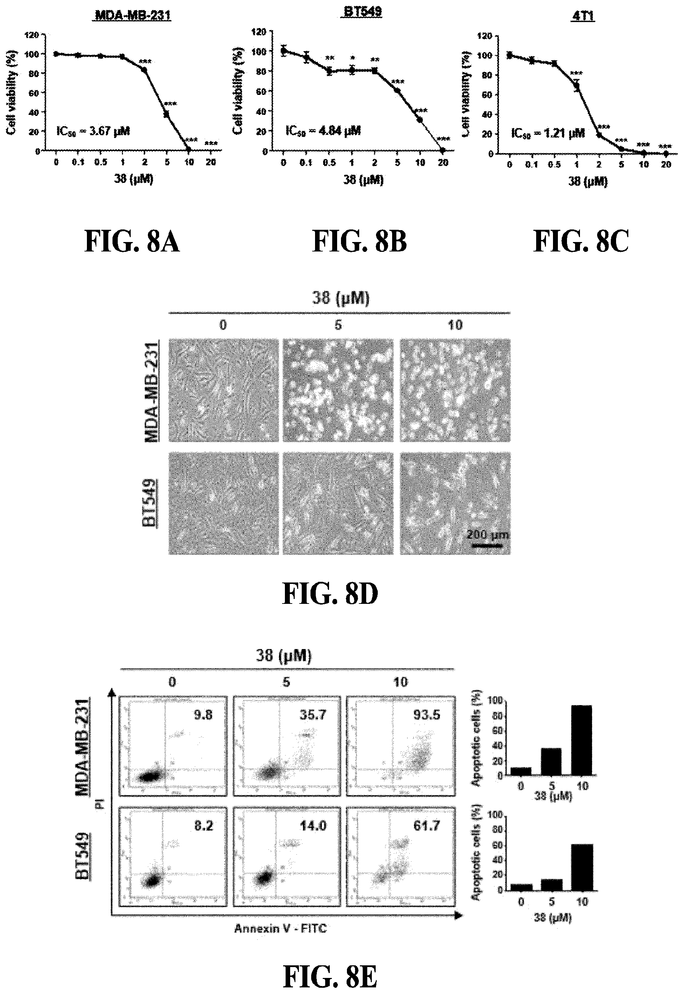

The result shows that compound 38 (formula 1-30) also decreased cell viability in a concentration-dependent manner, similar to compound 18c (formula 1-16) and compound 11k (formula 1-7), and the IC₅₀ values of compound 38 also showed very low values of 3.67 µM (MDA-MB-231), 4.84 µM (BT549), and 1.21 µM (4T1), respectively (FIG. 8A-8C).

### Experimental Example 2.2. Confirmation on cell cycle arrest and cell death

The degree of cancer cell death by the indazole derivative of the present disclosure was measured by analyzing DNA content using a flow cytometer. Cells were treated with compound 18c (formula 1-16) and compound 11k (formula 1-7) at concentrations of 0 (DMSO), 5, and 10 µM for 72 hours, and then the cells were harvested. Thereafter, the cells were fixed with 95% ethanol containing 0.5% Tween-20 for 24 hours, and stained with propidium iodide (PI, 50 µg/mL) and RNase (50 µg/mL) for 30 minutes. Afterwards, the degree of cancer cell death was analyzed using a flow cytometer.

In general, the cell cycle is divided into G1 (cell growth phase) - S (cell replication phase) - G2/M (cell division phase) according to the content of intracellular DNA. When cell death is induced, DNA fragmentation occurs, so that the content of DNA in each cell becomes significantly less than it is in the G1 phase. The results of this cell death within the cell cycle are manifested by the Sub-G1 region, and the percentage of Sub-G1 induced by the indazole derivative of the present disclosure is shown numerically in FIGS. 5B and 6B.

The result shows that the compound 18c (formula 1-16) and compound 11k (formula 1-7) significantly induced cell death (Sub-G1 population) and arrested the G2/M phase of the cell cycle in BT474 and JIMT-1 breast cancer cell lines.

Meanwhile, triple-negative breast cancer cell lines, MDA-MB-231 and BT549 were treated with compound 38 (formula 1-30), and then the type of cell death was determined by Annexin V/PI staining. The result shows that early and late apoptosis of cancer cells was induced (FIG. 8E).

### Experimental Example 2.3. Confirmation on expression of proteins involved in apoptosis

To investigate the mechanism of cell death by compound 18c (formula 1-16) and compound 11k (formula 1-7), the activation of apoptosis-related factor caspase family members was determined through western blotting.

To perform western blotting, BT-474 and JIMT-1 cell lines were treated with control (DMSO), compound 18c, and compound 11k at concentrations of 10 µM each for 72 hours, and then lysed in lysis buffer (30 mM NaCl, 0.5% Triton X-100, 50 mM Tris-HCl; pH 7.4) containing phosphatase and protease inhibitors. The proteins present in the supernatant were harvested. The protein concentration was measured using a Bradford protein assay kit, and 30 µg of protein was taken equally from each experimental group. These proteins were electrophoresed on SDS-Polyacrylamide gels (8-15%), and electrically transferred to a nitrocellulose membrane. The membrane was reacted with primary antibodies [pro PARP (1:1000), cleaved PARP (1:2000), cleaved caspase-3 (1:1000), cleaved caspase-7 (1:1000), β-actin (1:5000)] diluted in 5% bovine serum albumin (BSA) at 4°C for 24 hours, followed by a secondary antibody, horseradish peroxidase (HRP)-conjugated rabbit IgG (1:3000) for 2 hours at room temperature. The signal intensity of the above proteins was checked by color development using an Enhanced Chemiluminescence Kit and x-ray film.

As shown in FIG. 5C and FIG. 6C, the amounts of cleaved caspase-3 and cleaved caspase-7 in BT474 and JIMT-1 cell lines were determined by western blotting, and it was found that the protein amounts were significantly increased in the compound 18c and compound 11k treatment groups compared to the control group. Poly (ADP-ribose) polymerase (PARP), a DNA repair factor, is a substrate of caspase-3, and the increase in cleaved-PARP (89 kDa) suggests that it was cleaved by the activation of caspase-3 induced by treatment with compounds 18c and 11k. This demonstrates that the cell death induced by the indazole derivatives of the present disclosure is associated with caspase activation.

### Experimental Example 2.4. Confirmation on changes in cell morphology

MDA-MB-231 and BT549 cell lines were treated with compound 38 (formula 1-30) at concentrations of 0 (DMSO), 5, and 10 µM for 72 hours. Then, the drug-induced cytotoxicity was assessed by observing changes in cell morphology using phase contrast microscopy. The photographs were taken at a magnification of x200 using phase contrast microscopy. The photographs are shown in FIG. 8D.

Upon being treated with compound 38 (formula 1-30), both MDA-MB-231 and BT549 cell lines exhibited morphological changes in which the cytoplasm shrank, and most cells were observed in a floating state due to cytotoxicity (FIG. 8D).

### Experimental Example 2.5. Investigation on the expression of Hsp90 clients

Western blotting was used to investigate whether Hsp90 inhibitors, compound 18c (formula 1-16) and compound 11k (formula 1-7), could downregulate the expression and activity (phosphorylation) of HER2 and HER3, which are representative clients of Hsp90, in BT474 and JIMT-1 cell lines. The expression of Akt, MEK, ERK, Bcl-2, Cyclin D1, and Survivin, which are key factors for cell survival as Hsp90 clients, was also investigated. The western blotting was performed by the method that was used in Experimental Example 2.3.

The primary antibodies used for the western blotting were [HER2 (1:5000), phospho-HER2 (Tyr1221/1222, 1:1000), HER3 (1:2000), phospho-HER3 (Tyr1289, 1:2000), Akt (1:2000), MEK (1:2000), ERK (1:2000), phospho-ERK (Thr202/Tyr204, 1:2000), Bcl-2 (1:2000), Cyclin D1 (1:3000), Survivin (1:1000)] and were used after being diluted with 5% bovine serum albumin (BSA).

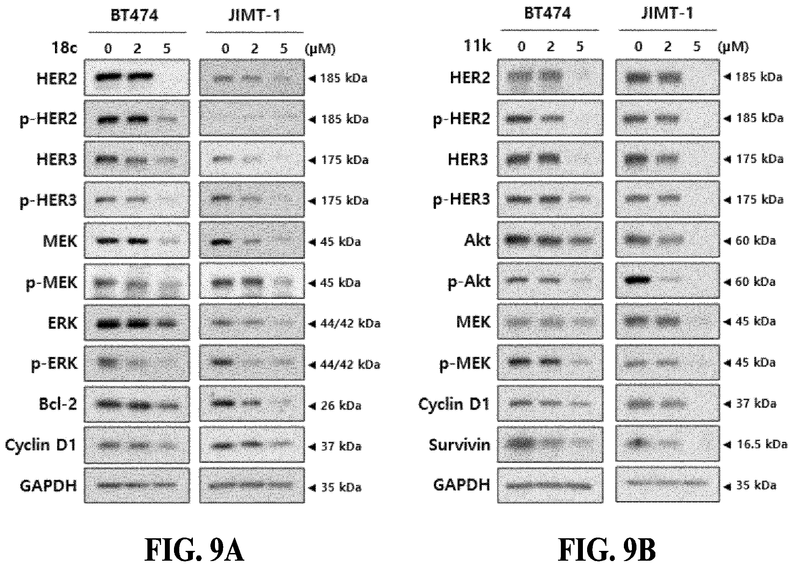

The BT474 and JIMT-1 cell lines were treated with compound 18c and compound 11k at a concentration of 10 µM for 72 hours. It was found that the expression of HER2 and HER3 was decreased, and that the expression of activated forms phospho-HER2 and phospho-HER3 was also significantly reduced, and that both compounds inhibited the expression of cell proliferation factors Akt, MEK, ERK, Bcl-2, Cyclin D1, and Survivin (FIG. 9).

### Experimental Example 3. Evaluation for in vivo anticancer efficacy

### Experimental Example 3.1. Confirmation on tumor growth

This experiment was performed in an in vivo environment using a xenograft mouse model transplanted with the JIMT-1 breast cancer cell line.

6-week-old mice (Balb/c nude mice) were purchased and raised in an environment where they could freely access food and water. The mice were acclimated for more than 7 days, and then were transplanted with tumor cells. Tumor cells were injected subcutaneously into the mammary gland tissue of JIMT-1 breast cancer cells at a density of 3 × 10⁶ cells/mL per individual, and when the tumor reached a size of 50 to 100 mm³, the indazole derivative of the present disclosure was administered intraperitoneally, and the tumor growth was measured. The experimental groups were treated with either 30 mg/kg of compound 18c (formula 1-16) or 20 mg/kg of compound 11k (formula 1-7), and tumor volume, tumor weight, body weight, and the like were measured every 3 to 4 days from the time of administration until the end of the study.

The result shows that the tumor volume (FIG. 10A and FIG. 11A) and tumor weight (FIG. 10B and FIG. 11B) in the compound 18c and compound 11k treatment groups were significantly reduced at a statistically significant level, compared to the control group. In particular, through visual inspection it was found that in the control group the tumor size continuously increased, but in the indazole derivative treatment group of the present disclosure, the tumor size was reduced by more than half and the body weight was maintained. The fact that the body weight was maintained, showed that only tumor growth was specifically inhibited and drug administration produced no serious toxic effects (FIG. 10C and FIG. 11C).

### Experimental Example 3.2. Confirmation on toxicity

To determine whether the treatment with the indazole derivatives of the present disclosure would affect normal organs, a toxicity analysis was performed.

The mouse model was sacrificed. The liver, kidney, and lung tissues were fixed in 4% paraformaldehyde for 24 hours, washed in PBS buffer, and then embedded in paraffin. Tissue samples were sectioned and stained with H&E (hematoxylin & eosin).

Additionally, blood was collected from the mouse model to determine the levels of AST (Aspartate aminotransferase) and ALT (Alanine aminotransferase) proteins, which can predict liver toxicity, and a BUN assay (Blood Urea Nitrogen) was performed to determine renal toxicity.

The result shows that there was no functional abnormality in the liver, kidney, and lung tissues at a level similar to that of the control group (FIG. 10D, FIG. 10E, FIG. 11D, and FIG. 11E). This suggests that the indazole derivative of the present disclosure only affects tumors and does not affect normal cells.

### Experimental Example 4. Confirmation on anticancer activity against other cancer cell lines

In addition to the breast cancer cell line, a leukemia cell line HL-60, a liver cancer cell line HepG2, a colorectal cancer cell line HCT116, a prostate cancer cell line Du145, an ovarian cancer cell line SKOV3, and non-small cell lung cancer cell lines NCI-H1299 and A549 were treated with compound 11k (formula 1-7) at concentrations of 0 (DMSO), 0.1, 0.5, 1, 2, 5, 10, and 20 µM, respectively, for 72 hours, and then cell viability was measured using the MTS assay technique as explained by the description of Experimental Example 1. The significance between the control group and the experimental group was verified using an unpaired Students t-test (*p<0.01; DMSO control vs compound 11k).

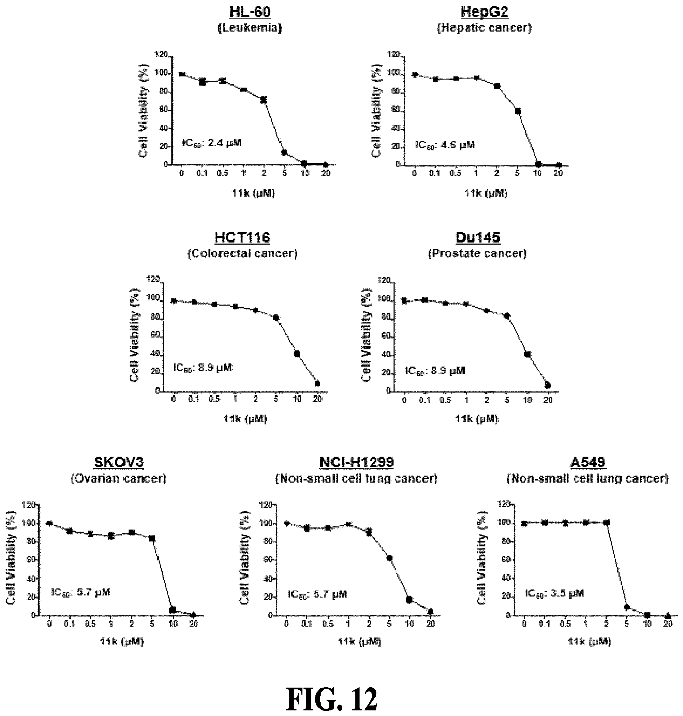

The result shows that the indazole derivative of the present disclosure is effective in inhibiting cell viability in breast cancer cell lines as well as other cancer cell lines (FIG. 12).

### Experimental Example 5. Administration in combination with paclitaxel

### Experimental Example 5.1. Confirmation on in vitro synergistic effect

HER2 positive breast cancer (HER2+ BC) cell lines, BT474 (cell seeding numbers: 2.4(BT474) x 10⁴ cells/wells (confluency≥50%)) sensitive to trastuzumab and JIMT-1 (cell seeding numbers: 1.0 (JIMT-1) x 10⁴ cells/wells (confluency≥30%)) resistant to trastuzumab were used in the experiment.

Compound 11k (formula 1-7) and paclitaxel, a representative anticancer drug were co-administered to the BT474 and JIMT-1 cell lines. The cell lines were treated with compound 11k at concentrations of 0 (DMSO), 2, and 5 µM, and with paclitaxel at concentrations of 0 (DMSO), 0.01, 0.05, and 0.1 µM for 72 hours, and then cell viability was measured. In addition, the synergistic effect from the compound 11k and paclitaxel combination treatment was calculated using the combination index (CI) as a function of the concentration of the compound 11k and paclitaxel combination treatment.

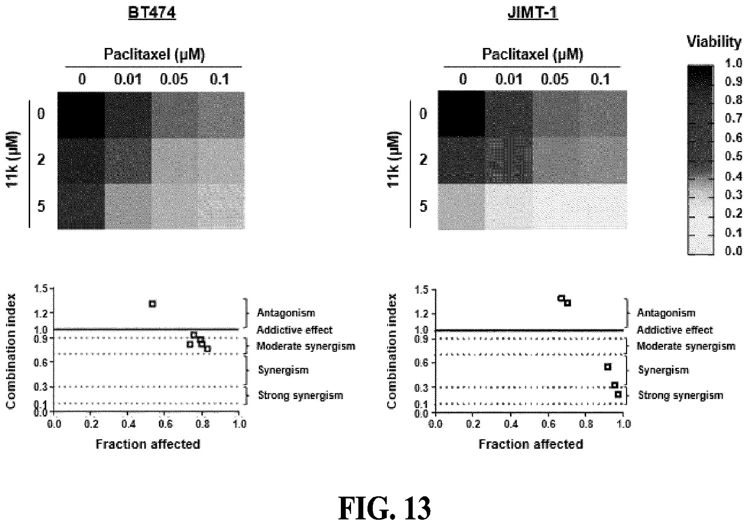

The result shows that the compound 11k and paclitaxel combination treatment had a very strong synergistic effect that further reduced cell viability, especially in the JIMT-1 cell lines (FIG. 13).

### Experimental Example 5.2. Confirmation on in vivo synergistic effect

A xenograft mouse model transplanted with the JIMT-1 breast cancer cell lines was prepared as explained by the description of Experimental Example 3, and the tumor volume, tumor weight, body weight, and the like were measured for the compound 11k alone treatment, the paclitaxel alone treatment, and the compound 11k and paclitaxel combination treatment.

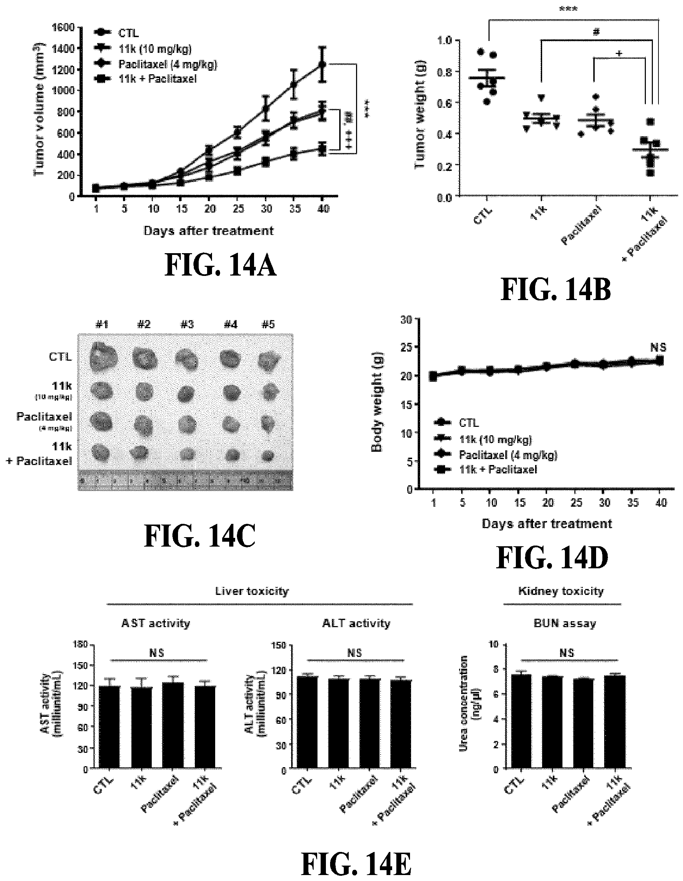

The result shows that the tumor volume (FIG. 14A) and tumor weight (FIG. 14B) were significantly reduced at a statistically significant level in the compound 11k and paclitaxel combination treatment group, compared to the control group, compared to the compound 11k alone treatment group, and compared to the paclitaxel alone treatment group. In addition, it was found that the tumor size was significantly reduced (FIG. 14C). The tumor volume of each group measured at 40 days after drug administration was 1246.91 mm³ for the control group; 786.392 and 813.356 mm³, respectively for compound 11k and paclitaxel alone treatment groups; and 451.915 mm³ for the compound 11k and paclitaxel combination treatment group, for which the combination treatment showed excellent synergistic effects. Similarly, the tumor weights measured after tumor resection were 761.667 mg for the control group; 500 mg for the compound 11k alone treatment group; 488.333 mg for the paclitaxel alone treatment group; and 298.333 mg for the compound 11k and paclitaxel combination treatment group. In these cases, the body weights all remained unchanged in the compound 11k alone treatment group, the paclitaxel alone treatment group, and the compound 11k and paclitaxel combination treatment group (FIG. 14D).

On the other hand, blood was collected from the mouse model to measure AST and ALT levels, a BUN assay was performed, and the result shows that the compound 11k alone treatment group, the paclitaxel alone treatment group, and the compound 11k and paclitaxel combination treatment group all exhibited levels similar to those of the control group (FIG. 14E).

This means that when the indazole derivative of the present disclosure is administered in combination with paclitaxel, a representative anticancer agent, the combination specifically inhibits tumor growth and affects only tumors and not normal cells.

### Experimental Example 6. Comparison with related art (KR 10-2304532)

The indazole derivative of the present disclosure was compared with the benzopyran derivative of the related art (KR 10-2304532) in terms of cell viability of the BT474, JIMT-1, and MDA-MB-231 cell lines (Table 1). In this case, the cell lines were treated with the benzopyran derivative at a concentration of 10 µM for 72 hours and the cell lines were treated with the indazole derivative at a concentration of 10 µM for 72 hours, and then the cell viability was measured.

**[Table 1]**

| **Present invention** | **compound** | **Prior Art** | **compound** |
|---|---|---|---|
| compound 6b (formula 1-2) | 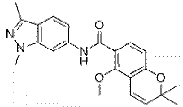 | NCT-88 (formula 2) | 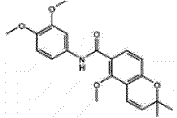 |
| compound 6c (formula 1-3) | 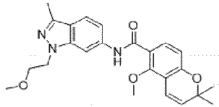 | NCT-368 (formula 3) | 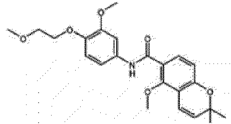 |
| compound 6d (formula 1-4) | 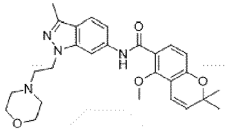 | NCT-21 (formula 14) | 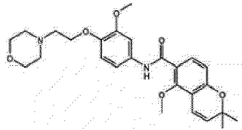 |
| compound 11k (formula 1-7) | 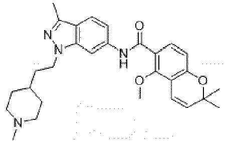 | NCT-44 (formula 18) | 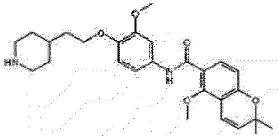 |
| compound 11l (formula 1-8) | 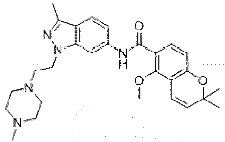 | NCT-25 (formula 16) | 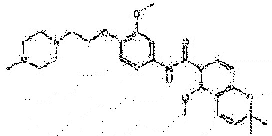 |
| compound 11m (formula 1-9) | 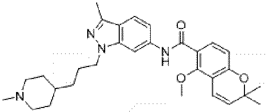 | NCT-394 (formula 23) | 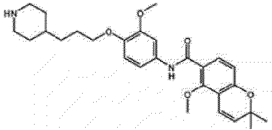 |
| compound 11n (formula 1-10) | 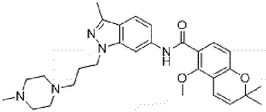 | NCT-364 (formula 21) | 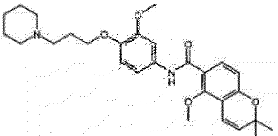 |

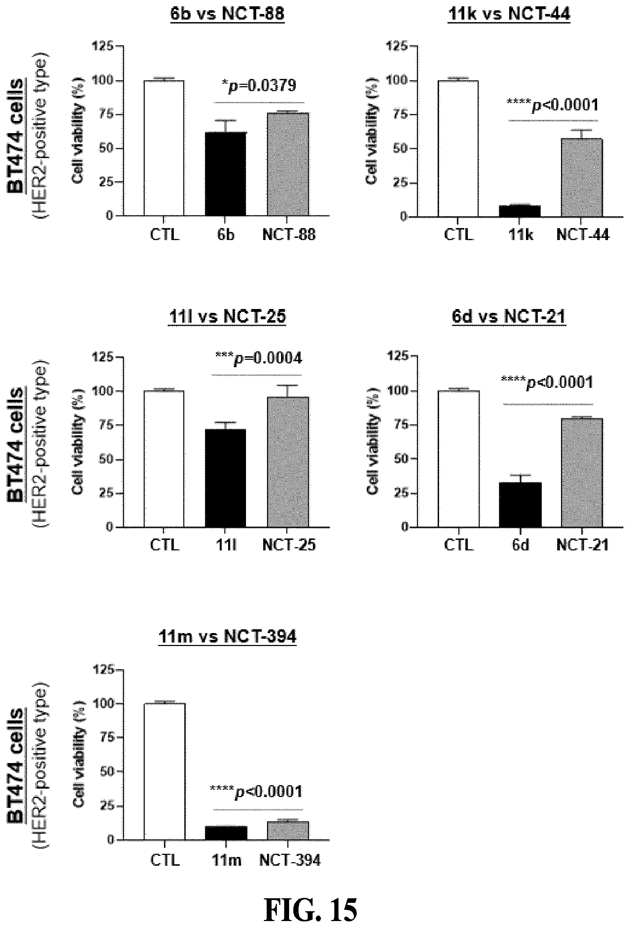

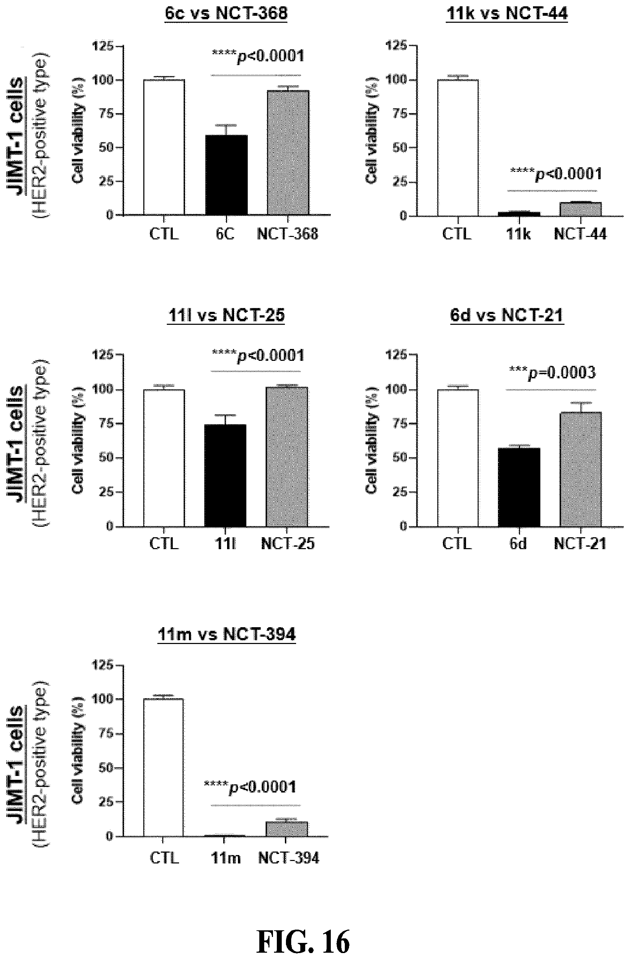

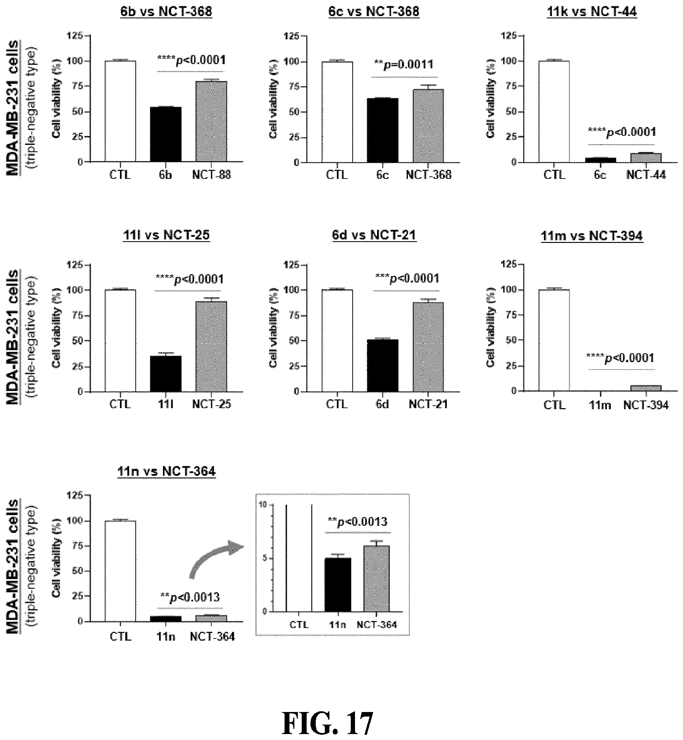

The result shows that even in the case of having similar substituents, the indazole derivatives of the present disclosure exhibited a superior cell viability inhibition effect (FIGS. 15 to 17).

## Claims

1. An indazole derivative represented by the following [Formula 1] or a pharmaceutically acceptable salt thereof: 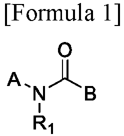 wherein, in Formula 1,
Ais 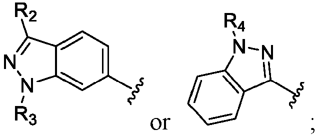
B is 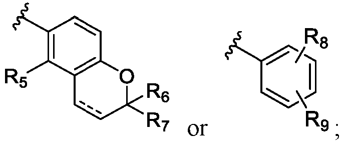
R₁ and R₂ are identical or different from each other, and each represents hydrogen or a C₁-C₆ chained alkyl group;
R₃ and R₄ are identical or different from each other, and each represents hydrogen or a C₁-C₆ chained alkyl group, wherein at least one hydrogen of the alkyl group is unsubstituted or substituted with a C₁-C₆ alkoxy group, a C₅-C₁₀ heterocycloalkyl group, or a C₅-C₁₀ heteroaryl group, wherein the alkoxy group is unsubstituted or substituted with a C₁-C₆ alkoxy group, and the heterocycloalkyl group is unsubstituted or substituted with a C₁-C₆ chained alkyl group, 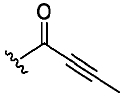 or 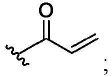
R₅ is a halogen group or a C₁-C₆ alkoxy group;
R₆ and R₇ are identical or different from each other, and each represents a C₁-C₆ chained alkyl group;
R₈ is NR₁₀C(O)R₁₁, OR₁₂, a C₅-C₁₀ heteroaryl group, or a tetrahydropyranyl amine group;
R₉ is hydrogen, a C₁-C₆ alkoxy group, or a C₅-C₁₀ heterocycloalkyl group, wherein the heterocycloalkyl group is unsubstituted or substituted with a C₁-C₆ chained alkyl group;
R₁₀ and R₁₁ are identical or different from each other, and each represents a C₁-C₆ chained alkyl group or C₅-C₁₀ aryl group, wherein the alkyl group is unsubstituted or substituted with a methylpiperazinyl group;
R₁₂ is a C₅-C₁₀ aryl group or a C₅-C₁₀ benzyl group, wherein the benzyl group is unsubstituted or substituted with a halogen group;
- - - is a single bond or a double bond.

2. The indazole derivative or a pharmaceutically acceptable salt thereof of claim 1, **characterized in that** the indazole derivative represented by the [Formula 1] is any one or more selected from the group consisting of the compounds represented by the following formulas: 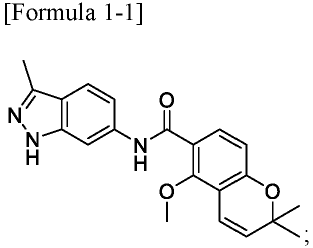 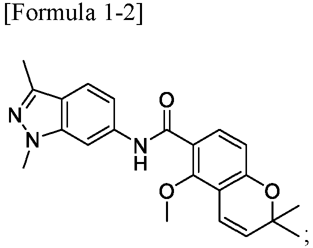 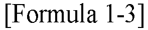 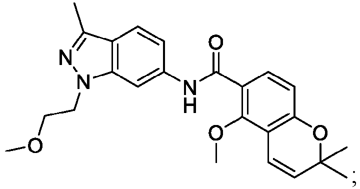 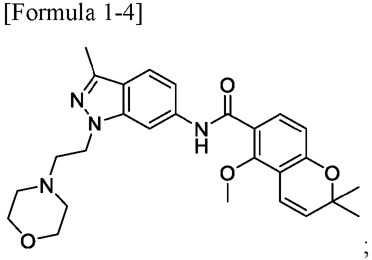 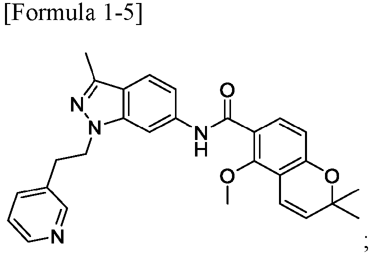 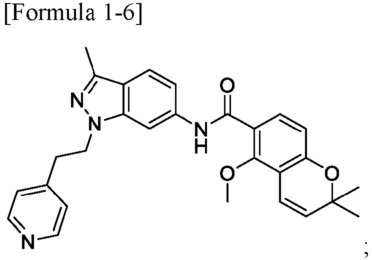 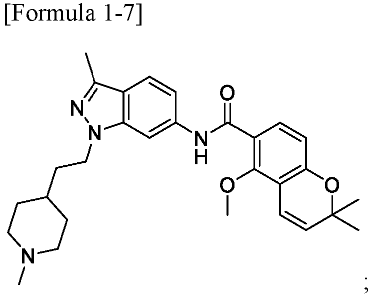 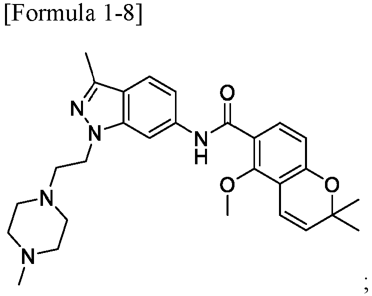 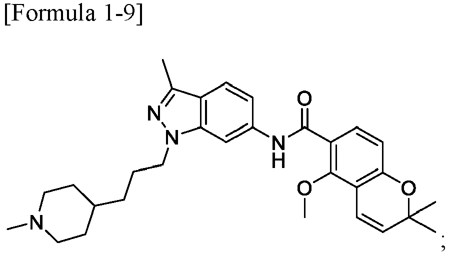 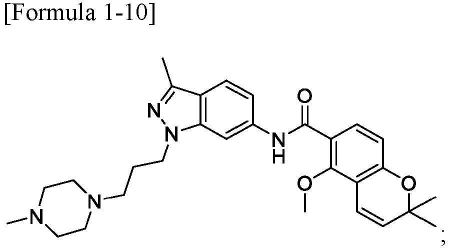 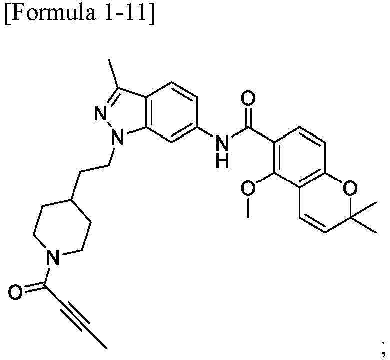 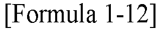 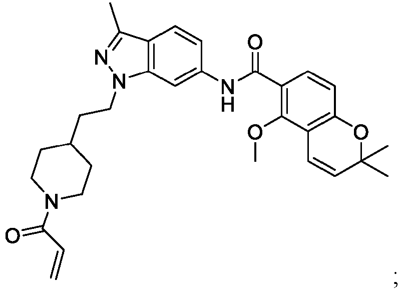 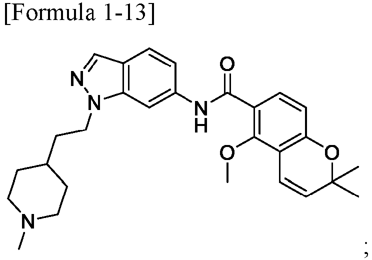 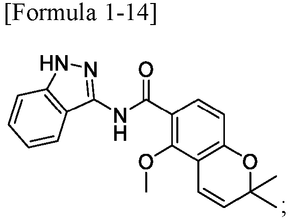 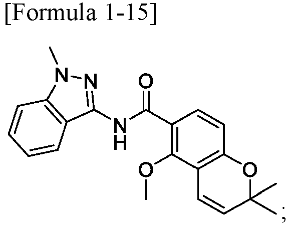 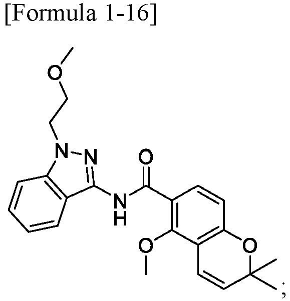 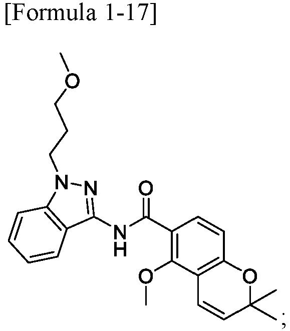 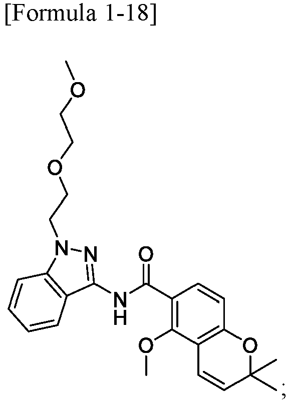 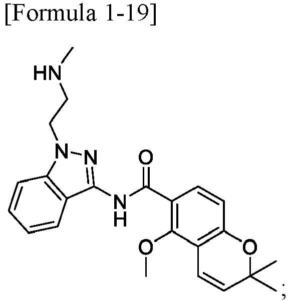 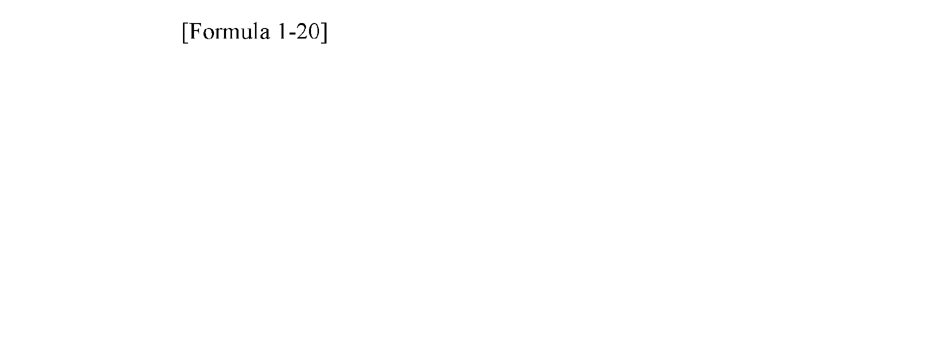 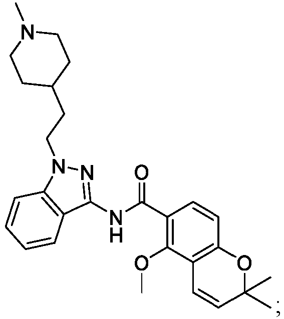 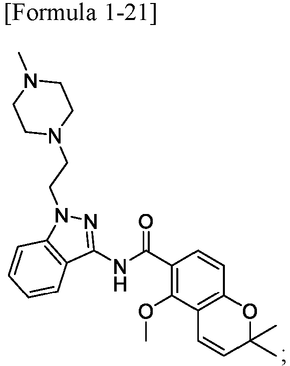 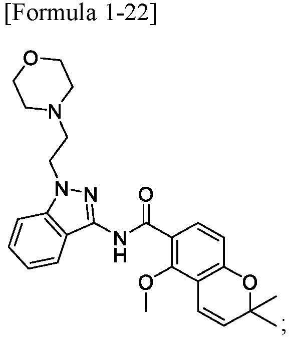 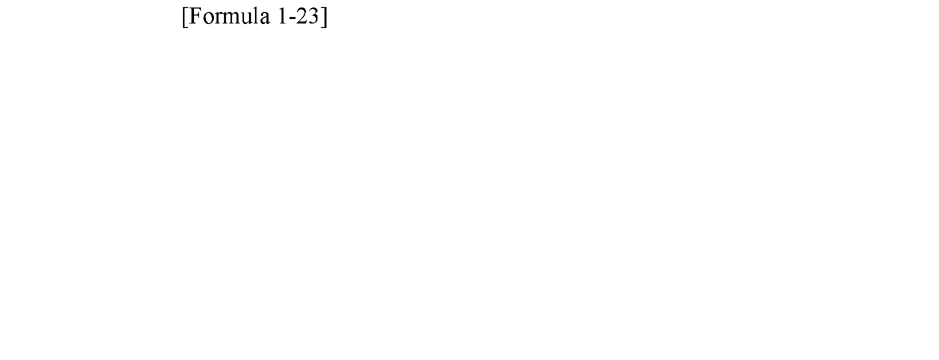 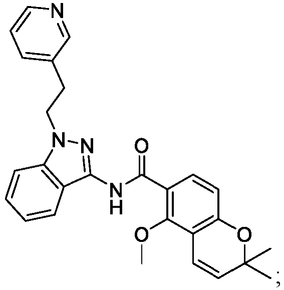 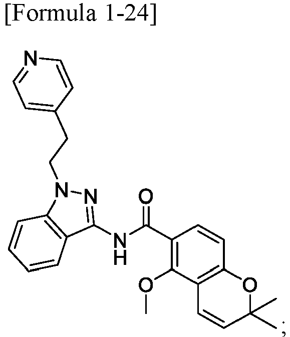 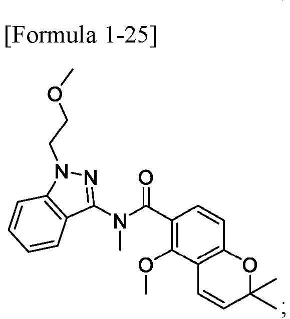 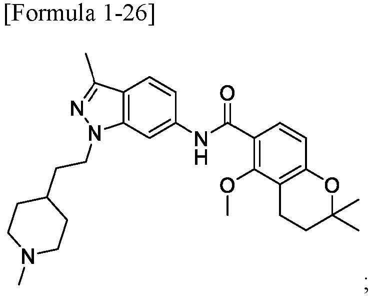 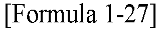 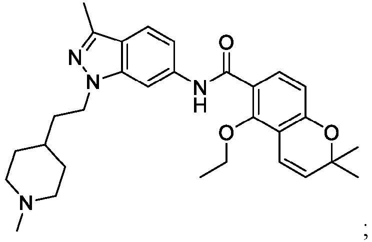 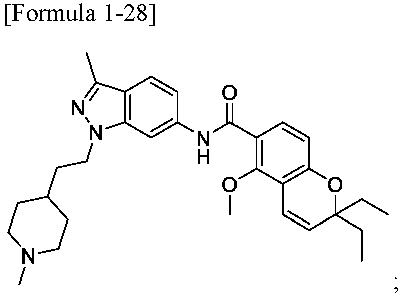 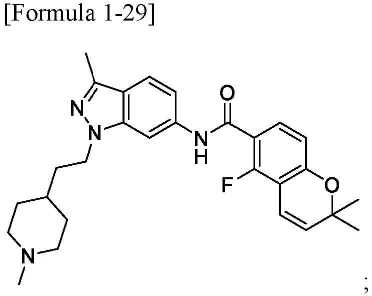 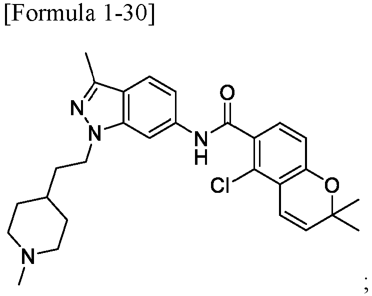 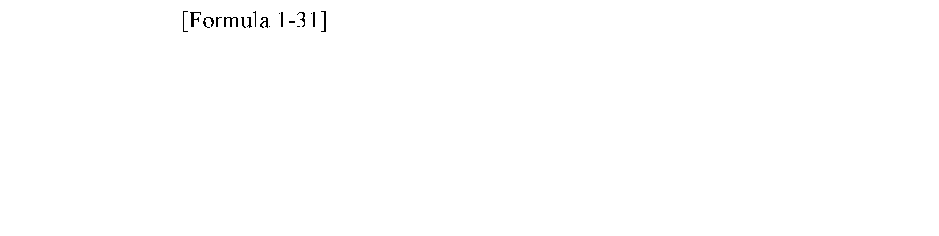 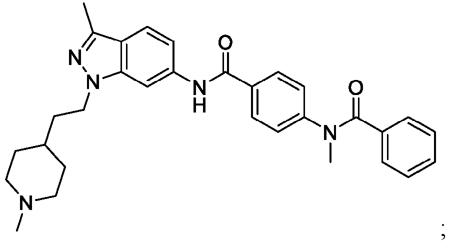 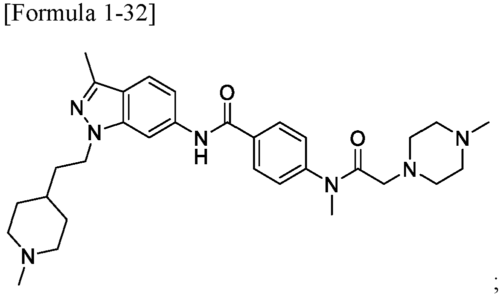 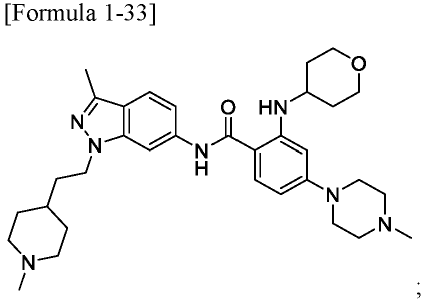 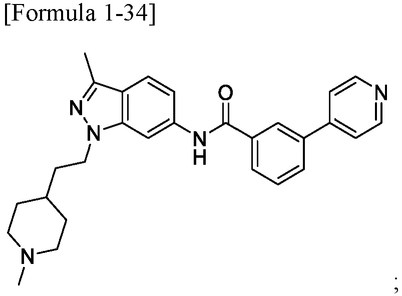 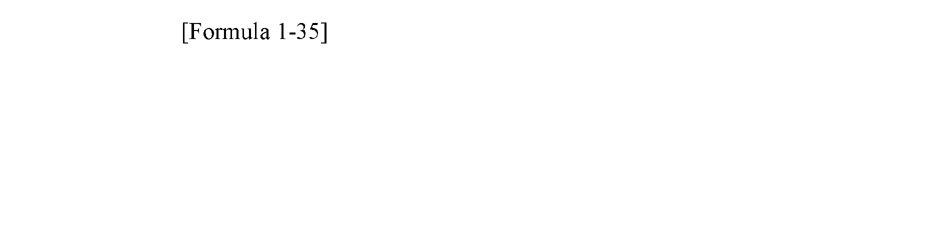 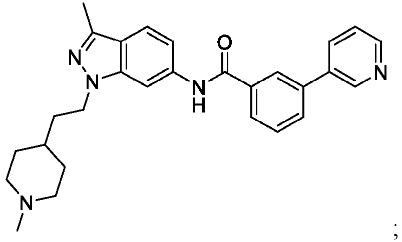 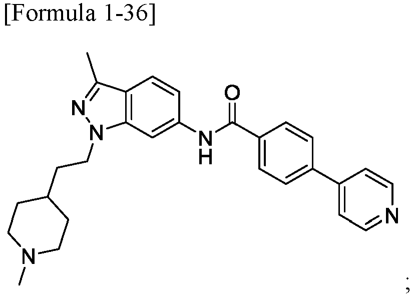 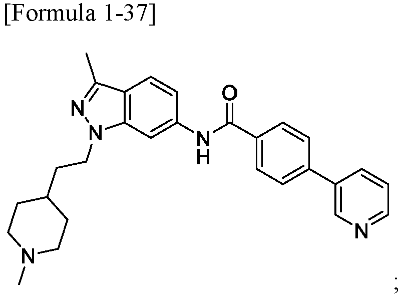 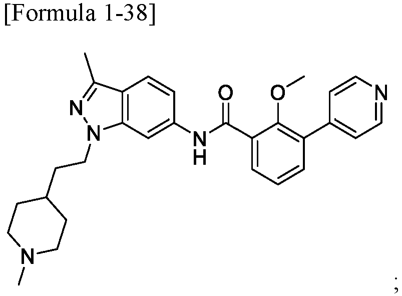 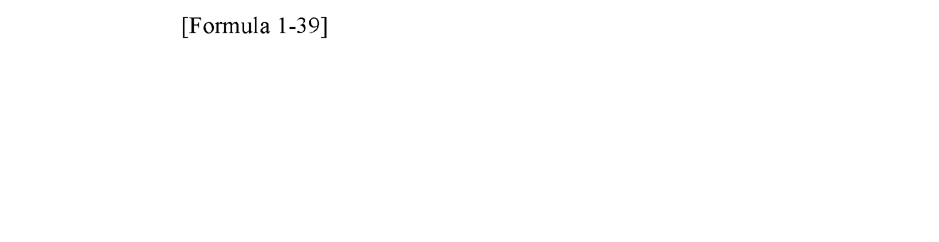 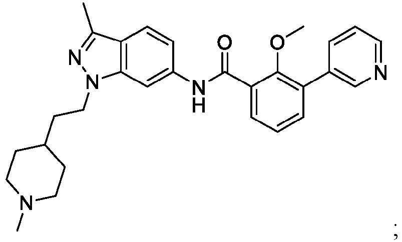 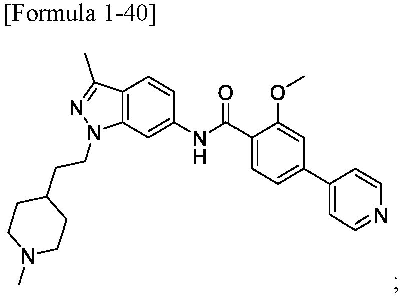 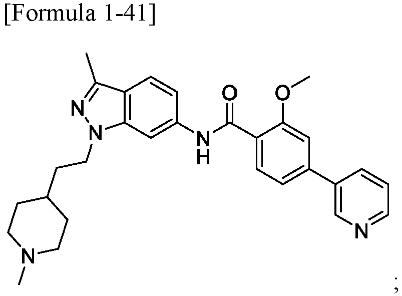 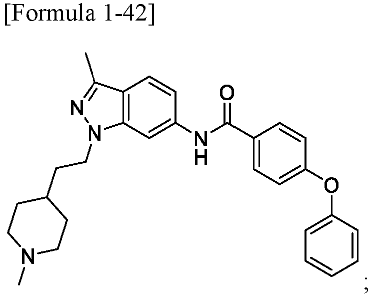 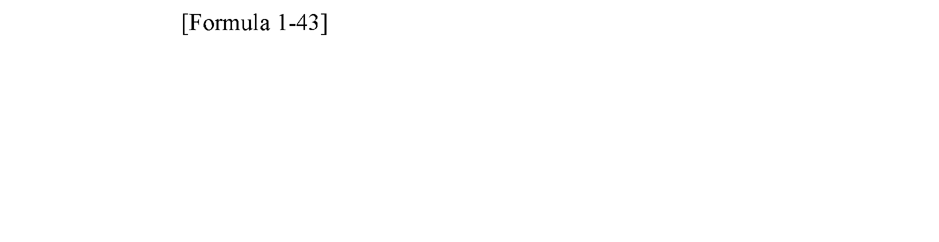 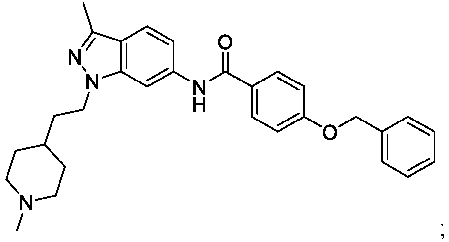 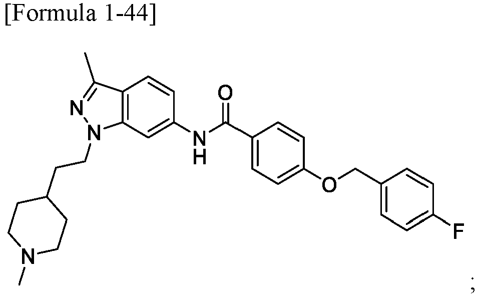 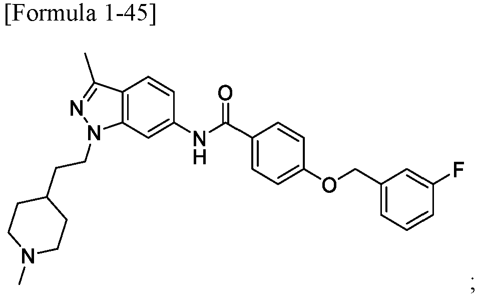 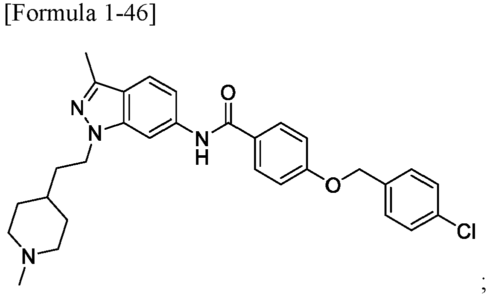 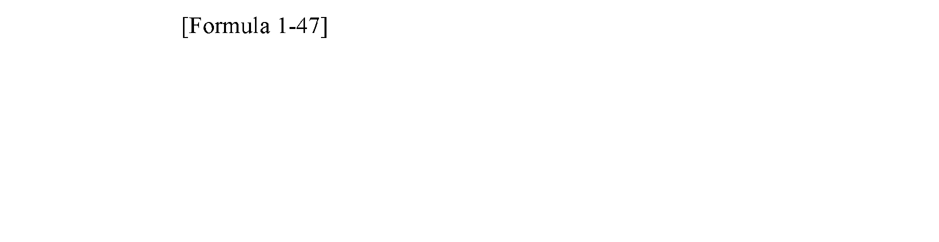 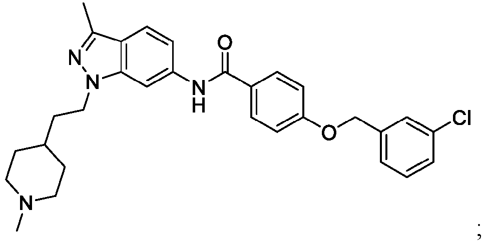 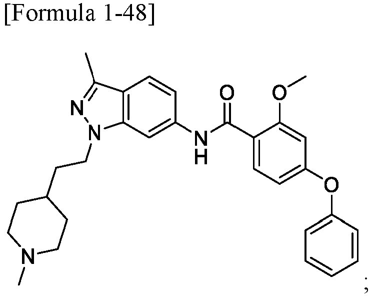 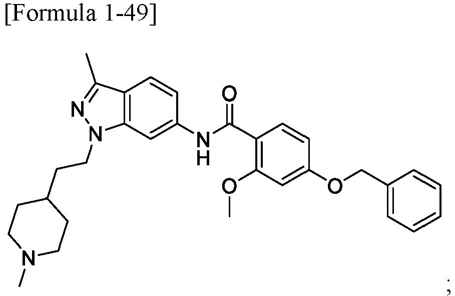 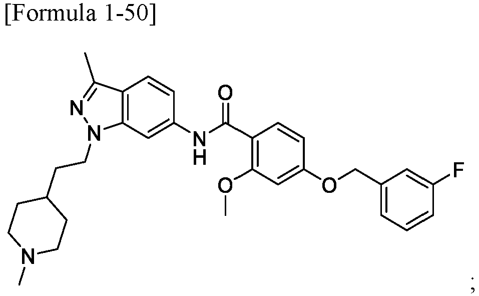 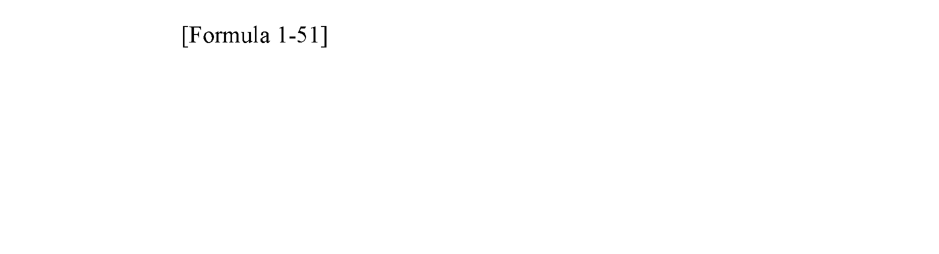 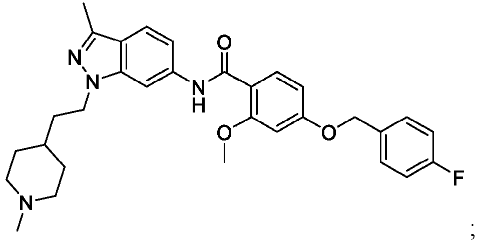 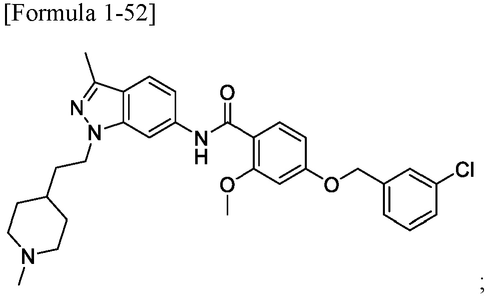 and

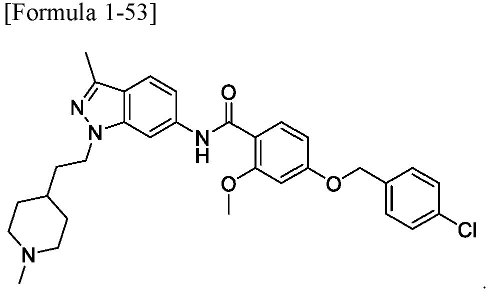

3. A pharmaceutical composition for use in a method for preventing or treating cancer, comprising the indazole derivative represented by [Formula 1] or a pharmaceutically acceptable salt thereof of claim 1 as an active ingredient.

4. The pharmaceutical composition for use of claim 3, **characterized in that** the indazole derivative induces cell death by inhibiting Hsp90.

5. The pharmaceutical composition for use of claim 3, wherein the cancer is any one or more selected from the group consisting of skin cancer, breast cancer, uterine cancer, esophageal cancer, stomach cancer, a brain tumor, colon cancer, rectal cancer, colorectal cancer, lung cancer, ovarian cancer, cervical cancer, endometrial cancer, vulvar cancer, kidney cancer, blood cancer, pancreatic cancer, prostate cancer, testicular cancer, laryngeal cancer, head and neck cancer, thyroid cancer, liver cancer, bladder cancer, osteosarcoma, lymphoma, leukemia, thymic cancer, urethral cancer, bronchial cancer, and combinations thereof.

6. The pharmaceutical composition for use of claim 3, **characterized in that** the pharmaceutical composition comprises the indazole derivative or a pharmaceutically acceptable salt thereof; and further comprises any one or more additional component selected from the group consisting of a pharmaceutically acceptable carrier, excipient, diluent, stabilizer, and preservative.

7. The pharmaceutical composition for use of claim 3, **characterized in that** the pharmaceutical composition comprises the indazole derivative or a pharmaceutically acceptable salt thereof; and further comprises an anticancer agent.

8. The pharmaceutical composition for use of claim 7, wherein the anticancer agent is any one or more selected from the group consisting of paclitaxel, docetaxel, doxorubicin, sorafenib, vemurafenib, irinotecan, cisplatin, alpharadin, mitoxantrone, cyclophosphamide, vinblastine, carboplatin, actinomycin-D, etoposide, teniposide, bisantrene, homoharringtonine, Gleevec (STI-571), 5-fluorouracil, busulfan, chlorambucil, melphalan, nitrogen mustard, nitrosoureas, and combinations thereof.

9. The pharmaceutical composition for use of claim 3, **characterized in that** the pharmaceutical composition has any one or more dosage form selected from the group consisting of powders, granules, tablets, capsules, and injections.

10. A method for producing an indazole derivative represented by [Formula 1] or a pharmaceutically acceptable salt thereof, comprising reacting a compound represented by [Formula 2] or [Formula 3] with a compound represented by [Formula 4] or [Formula 5]: 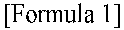 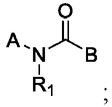 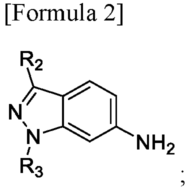 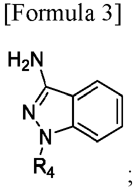 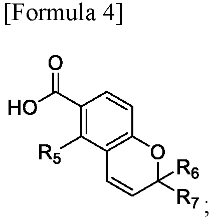 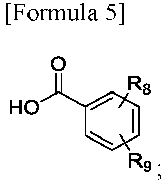 wherein, in Formula 1,
Ais 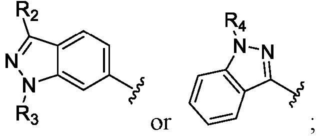
B is 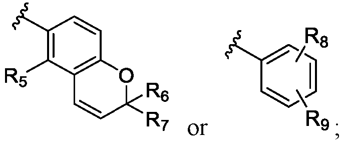
R₁ is hydrogen or a C₁-C₆ chained alkyl group;
wherein, in Formulas 1 and 2,
R₂ is hydrogen or a C₁-C₆ chained alkyl group;
R₃ is hydrogen or a C₁-C₆ chained alkyl group, wherein at least one hydrogen of the alkyl group is unsubstituted or substituted with a C₁-C₆ alkoxy group, a C₅-C₁₀ heterocycloalkyl group, or a C₅-C₁₀ heteroaryl group, wherein the alkoxy group is unsubstituted or substituted with a C₁-C₆ alkoxy group, and the heterocycloalkyl group is unsubstituted or substituted with a C₁-C₆ chained alkyl group, 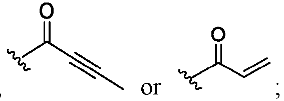
wherein, in Formulas 1 and 3,
R₄ is hydrogen or a chained C₁-C₆ alkyl group,
wherein at least one hydrogen of the alkyl group is unsubstituted or substituted with a C₁-C₆ alkoxy group, a C₅-C₁₀ heterocycloalkyl group, or a C₅-C₁₀ heteroaryl group, wherein the alkoxy group is unsubstituted or substituted with a C₁-C₆ alkoxy group, and the heterocycloalkyl group is unsubstituted or substituted with a C₁-C₆ chained alkyl group, 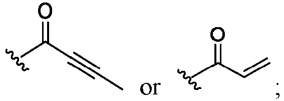
wherein, in Formulas 1 and 4,
R₅ is a halogen group or a C₁-C₆ alkoxy group;
R₆ and R₇ are identical or different from each other, and each represents a C₁-C₆ chained alkyl group;
wherein, in Formulas 1 and 5,
R₈ is NR₁₀C(O)R₁₁, OR₁₂, a C₅-C₁₀ heteroaryl group, or a tetrahydropyranyl amine group;
R₉ is hydrogen, a C₁-C₆ alkoxy group, or a C₅-C₁₀ heterocycloalkyl group, wherein the heterocycloalkyl group is unsubstituted or substituted with a C₁-C₆ chained alkyl group;
R₁₀ and R₁₁ are identical or different from each other, and each represents a C₁-C₆ chained alkyl group or C₅-C₁₀ aryl group, wherein the alkyl group is unsubstituted or substituted with a methylpiperazinyl group;
R₁₂ is a C₅-C₁₀ aryl group or a C₅-C₁₀ benzyl group, wherein the benzyl group is unsubstituted or substituted with a halogen group.

11. The method of claim 10, **characterized in that** the compound represented by the [Formula 2] is any one or more selected from the group consisting of the compounds represented by the following formulas: 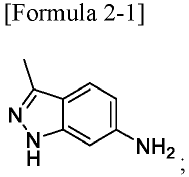 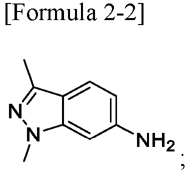 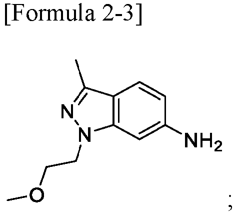 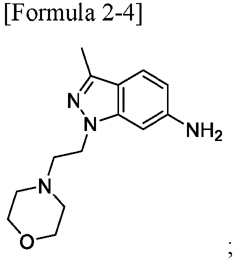 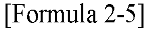 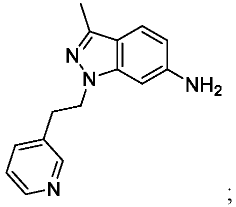 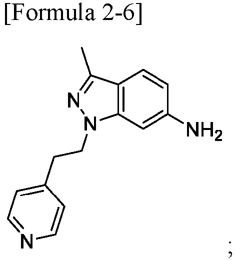 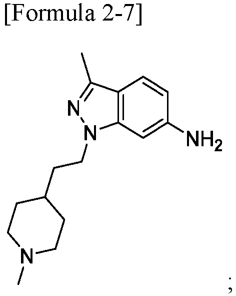 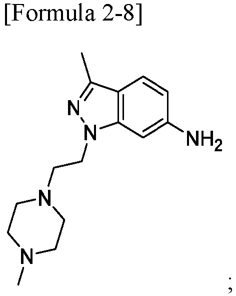 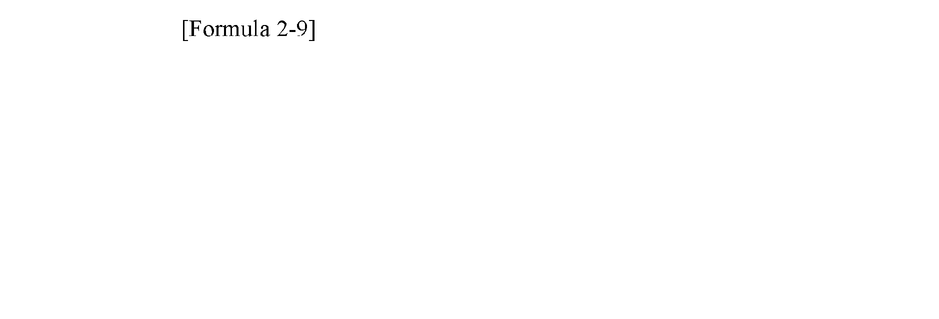 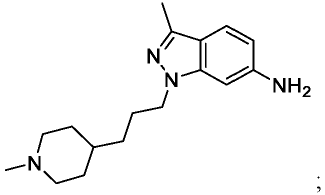 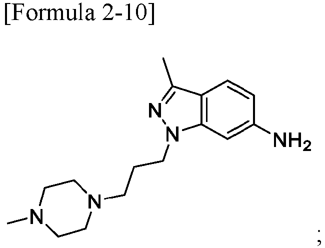 and

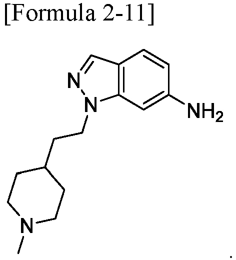

12. The method of claim 10, **characterized in that** the compound represented by the [Formula 3] is any one or more selected from the group consisting of the compounds represented by the following formulas: 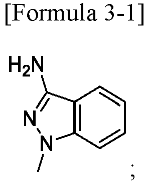 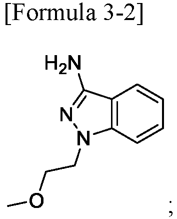 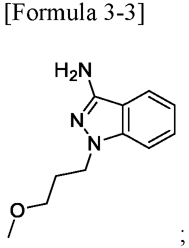 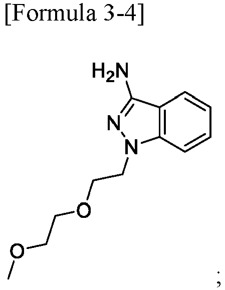 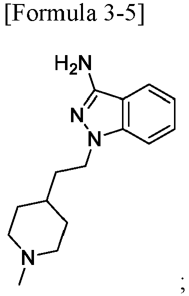 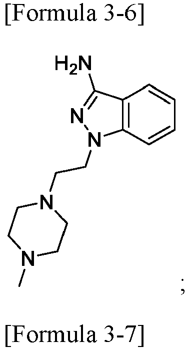 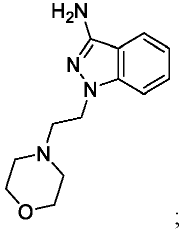 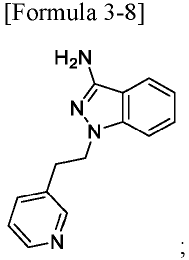 and

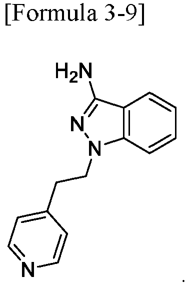

13. The method of claim 10, **characterized in that** the compound represented by the [Formula 4] is any one or more selected from the group consisting of the compounds represented by the following formulas: 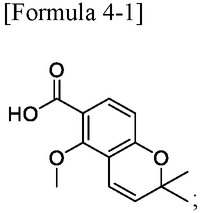 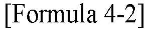 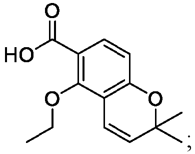 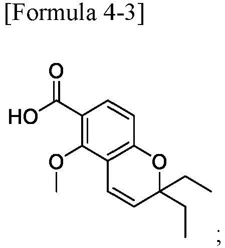 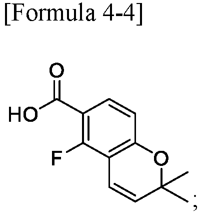 and

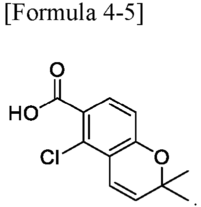

14. The method of claim 10, **characterized in that** the compound represented by the [Formula 5] is any one or more selected from the group consisting of the compounds represented by the following formulas: 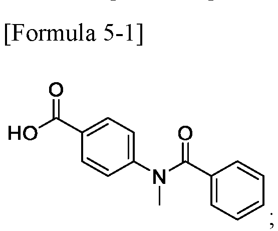 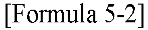 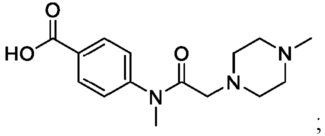 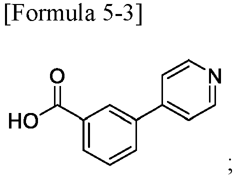 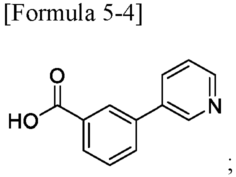 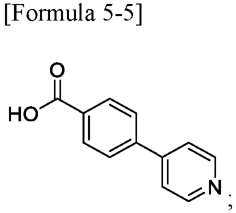 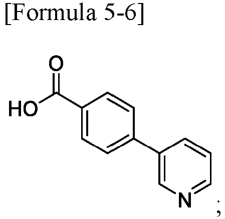 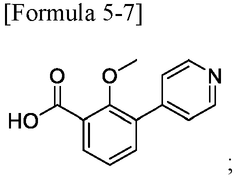 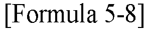 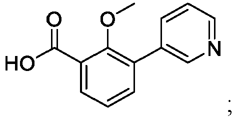 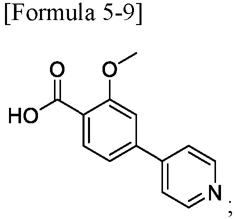 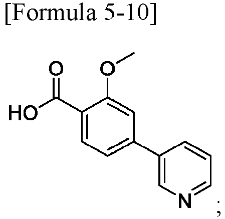 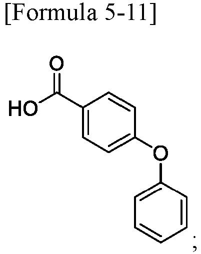 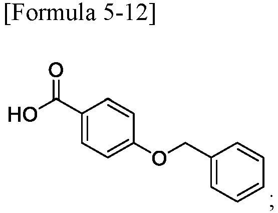 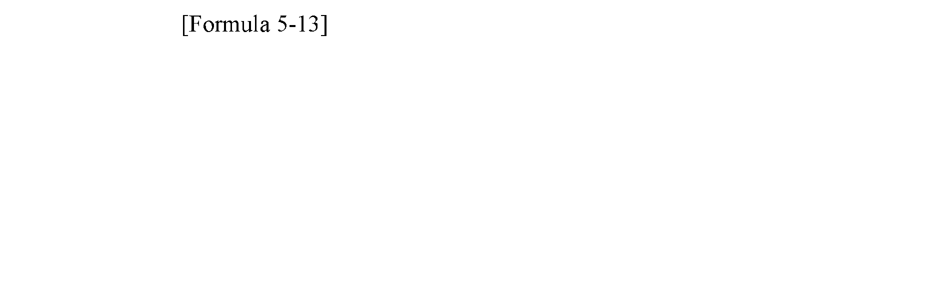 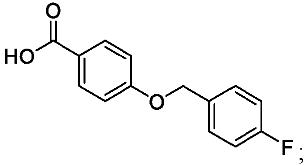 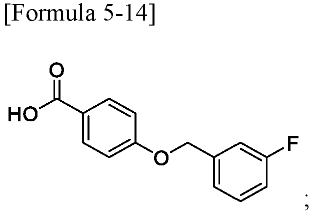 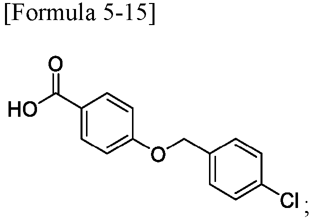 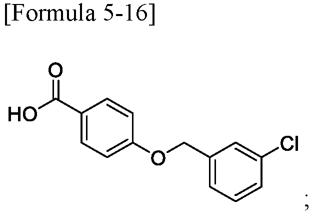 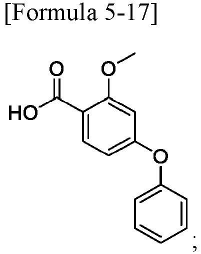 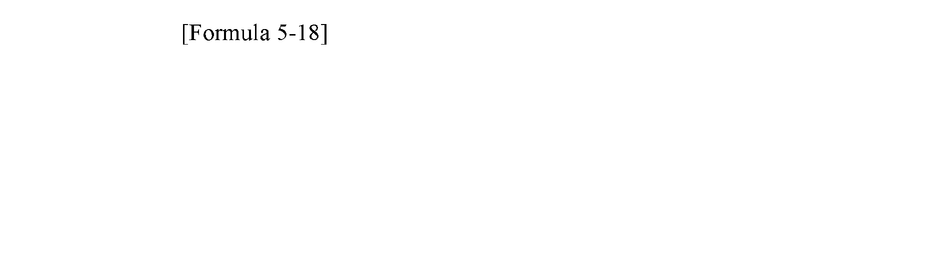 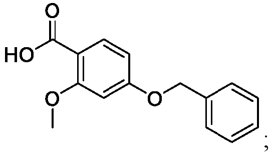 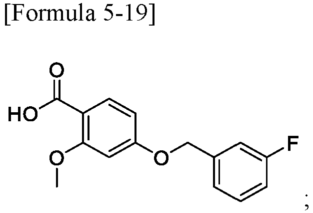 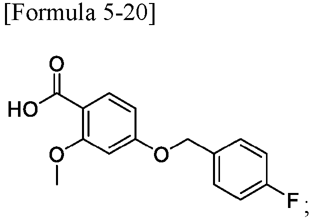 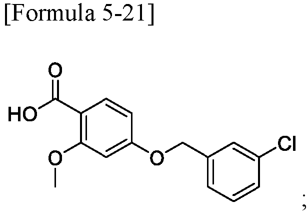 and

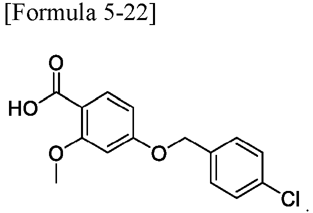

15. The method of claim 10, **characterized in that** the compound represented by the [Formula 2] or [Formula 3] is reacted by adding the compound represented by the [Formula 2] or [Formula 3] to a mixture of the compound represented by the [Formula 4] or [Formula 5], hydroxybenzotriazole (HOBt), triethylamine (Et₃N), and EDC·HCl.

## Patentansprüche

1. Indazol-Derivat, welches durch die folgende [Formel 1] dargestellt ist, oder ein pharmazeutisch annehmbares Salz davon: 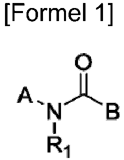 wobei in Formel 1,
A 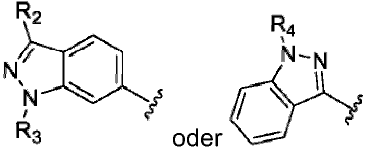 ist;
B 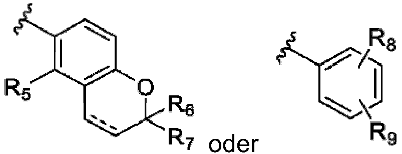 ist;
R₁ und R₂ identisch oder verschieden voneinander sind, und jeweils Wasserstoff oder eine kettenförmige C₁-C₆-Alkylgruppe darstellen;
R₃ und R₄ identisch oder verschieden voneinander sind, und jeweils Wasserstoff oder eine kettenförmige C₁-C₆-Alkylgruppe darstellen, wobei mindestens ein Wasserstoff der Alkylgruppe unsubstituiert oder mit einer C₁-C₆-Alkoxygruppe, einer C₅-C₁₀-Heterocycloalkylgruppe oder einer C₅-C₁₀-Heteroarylgruppe substituiert ist, wobei die Alkoxygruppe unsubstituiert oder mit einer C₁-C₆-Alkoxygruppe substituiert ist und die Heterocycloalkylgruppe unsubstituiert oder mit einer kettenförmigen C₁-C₆-Alkylgruppe, 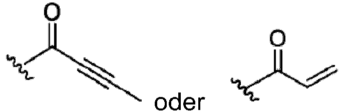 substituiert ist;
R₅ eine Halogengruppe oder eine C₁-C₆-Alkoxygruppe ist;
R₆ und R₇ identisch oder verschieden voneinander sind und jeweils eine kettenförmige C₁-C₆-Alkylgruppe darstellen;
R₈ NR₁₀C(O)R₁₁, OR₁₂, eine C₅-C₁₀-Heteroarylgruppe oder eine Tetrahydropyranylamingruppe ist;
R₉ Wasserstoff, eine C₁-C₆-Alkoxygruppe oder eine C₅-C₁₀-Heterocycloalkylgruppe ist, wobei die Heterocycloalkylgruppe unsubstituiert oder mit einer kettenförmigen C₁-C₆-Alkylgruppe substituiert ist;
R₁₀ und R₁₁ identisch oder verschieden voneinander sind und jeweils eine kettenförmige C₁-C₆-Alkylgruppe oder C₅-C₁₀-Arylgruppe darstellen, wobei die Alkylgruppe unsubstituiert oder mit einer Methylpiperazinylgruppe substituiert ist;
R₁₂ eine C₅-C₁₀-Arylgruppe oder eine C₅-C₁₀-Benzylgruppe ist, wobei die Benzylgruppe unsubstituiert oder mit einer Halogengruppe substituiert ist;
- - - eine Einfachbindung oder eine Doppelbindung ist.

2. Indazol-Derivat oder ein pharmazeutisch annehmbares Salz davon nach Anspruch 1, **dadurch gekennzeichnet, dass** das Indazol-Derivat, welches durch die [Formel 1] dargestellt ist, eines oder mehrere ist, ausgewählt aus der Gruppe bestehend aus den Verbindungen, welche durch die folgenden Formeln dargestellt sind: 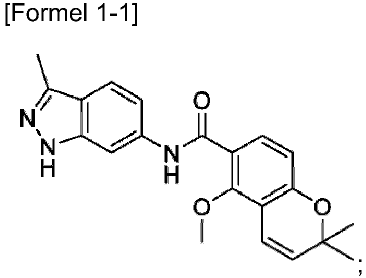 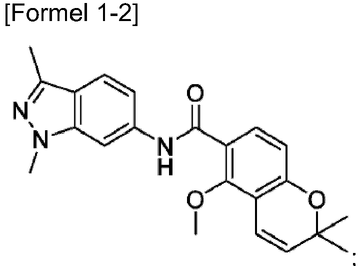 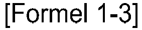 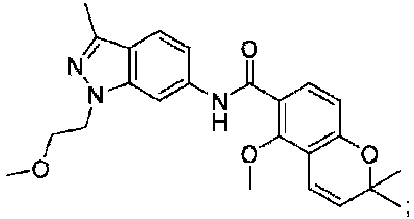 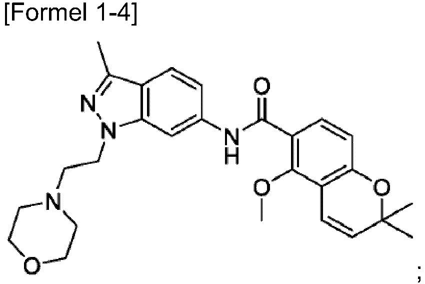 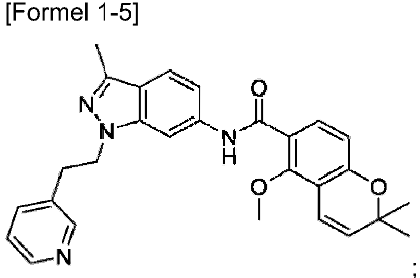 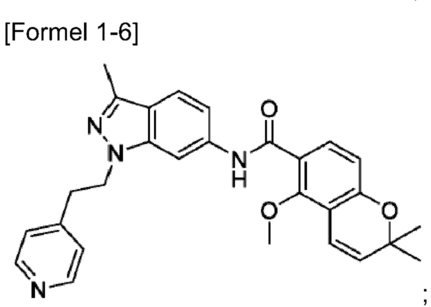 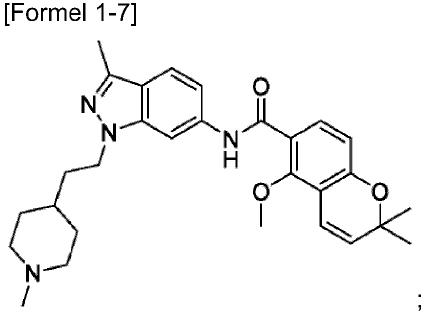 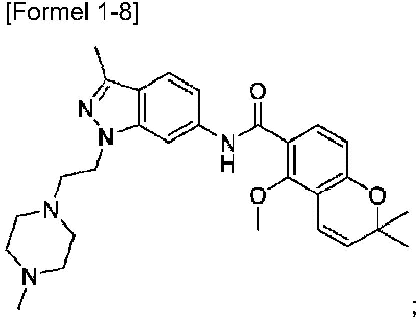 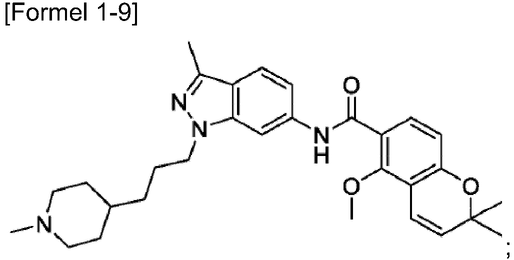 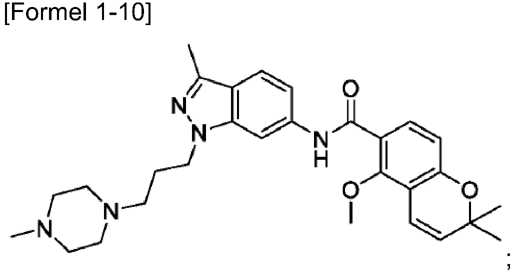 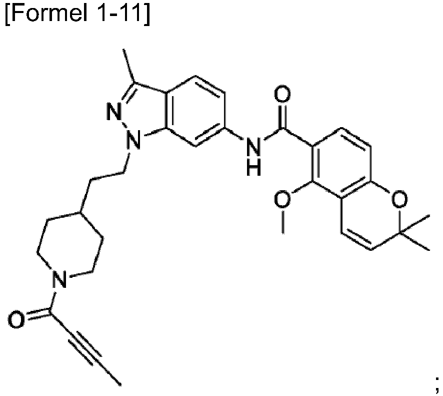 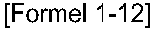 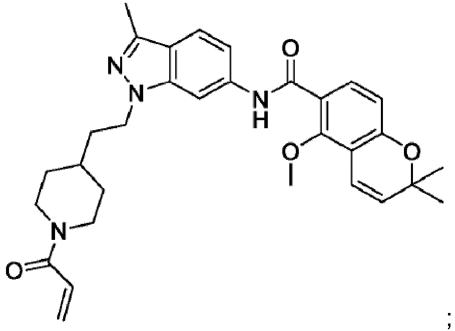 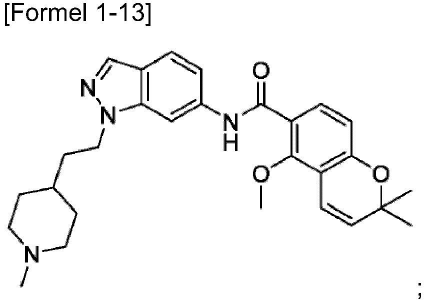 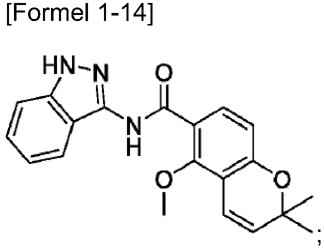 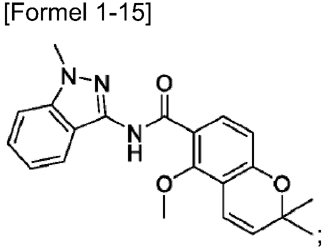 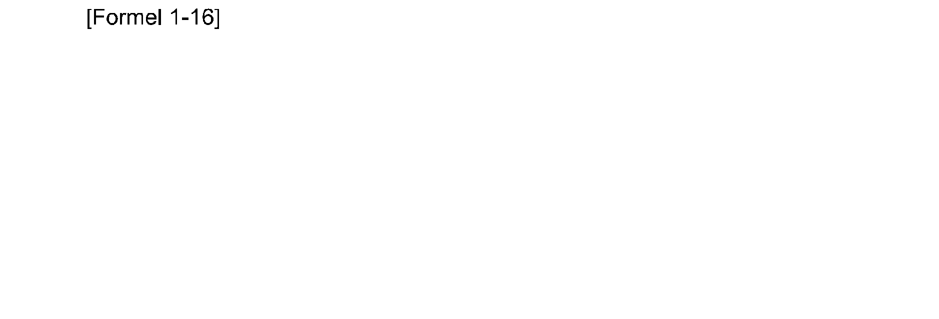 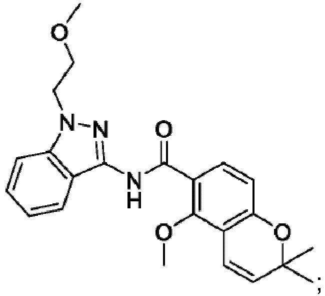 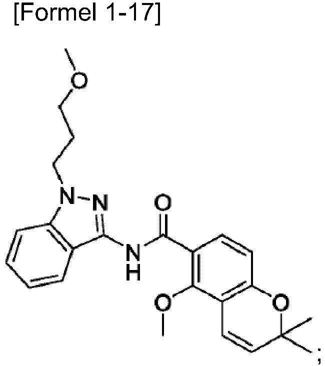 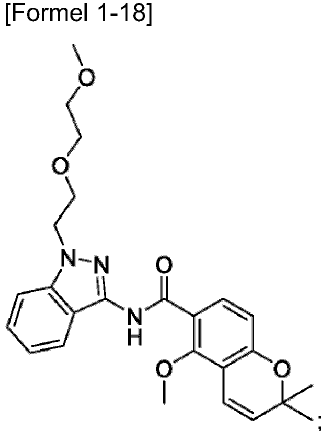 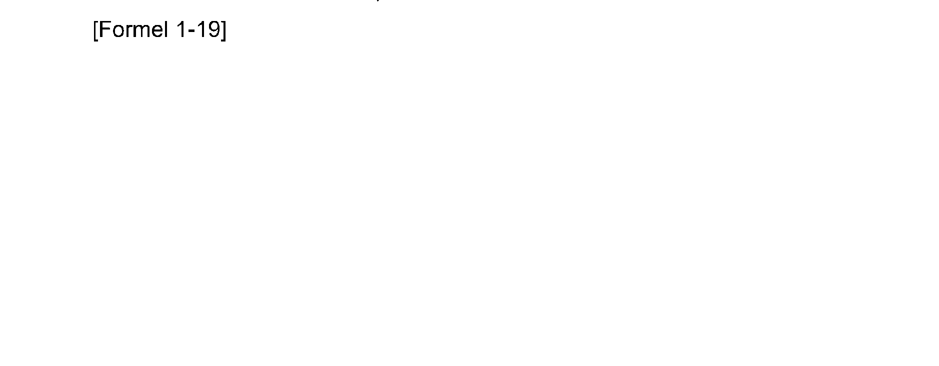 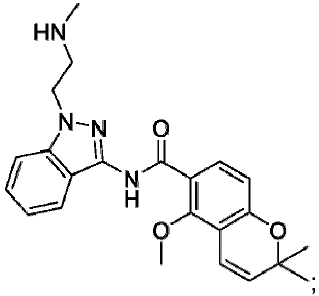 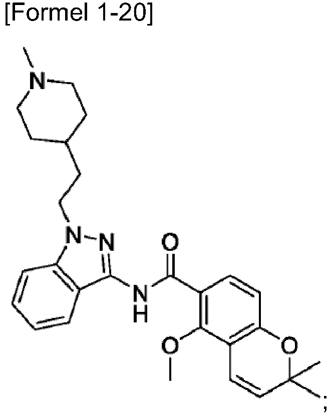 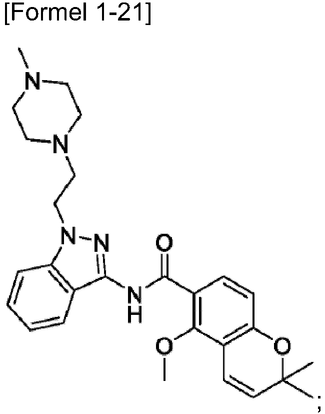 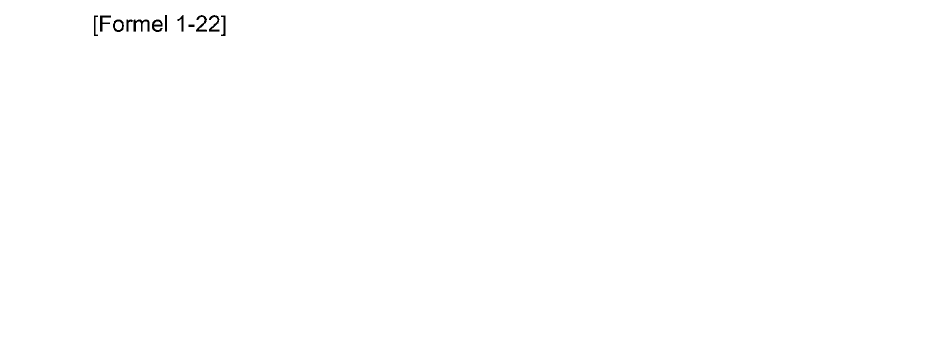 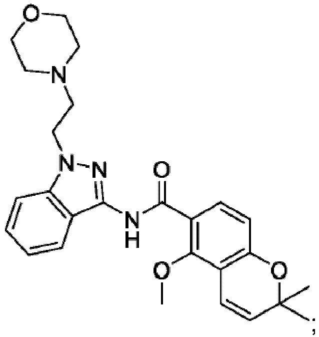 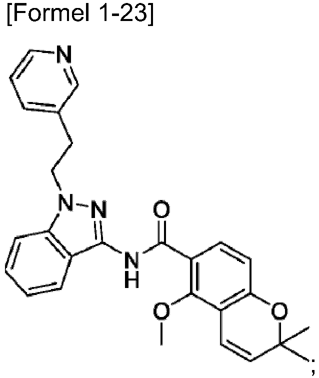 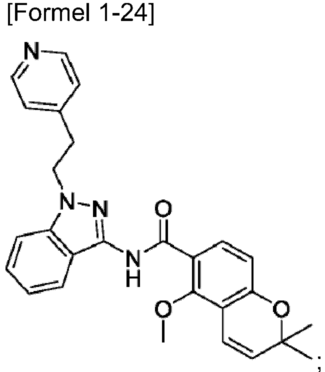 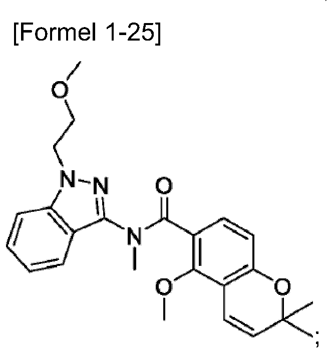 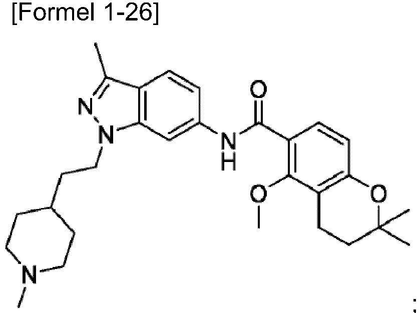 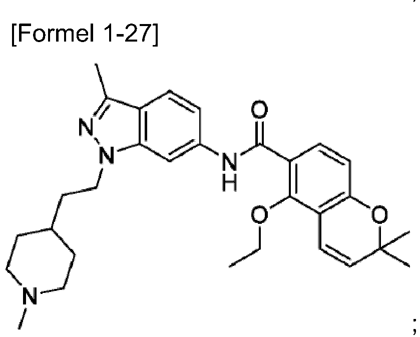 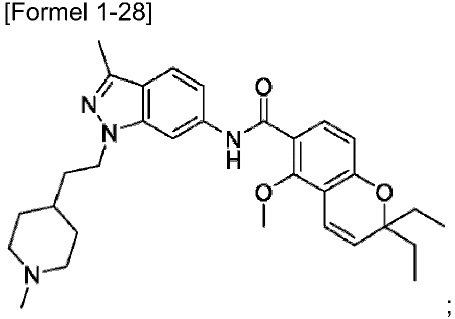 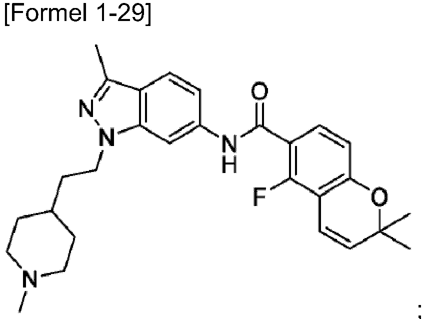 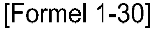 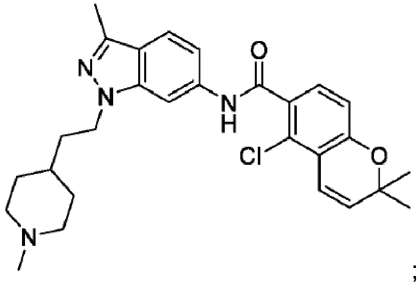 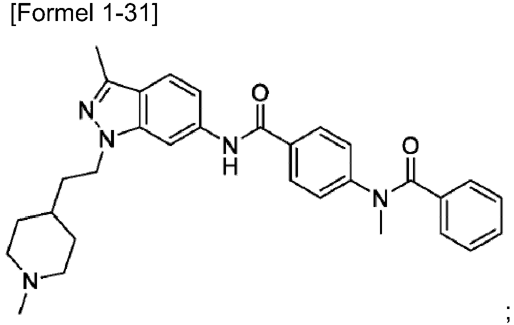 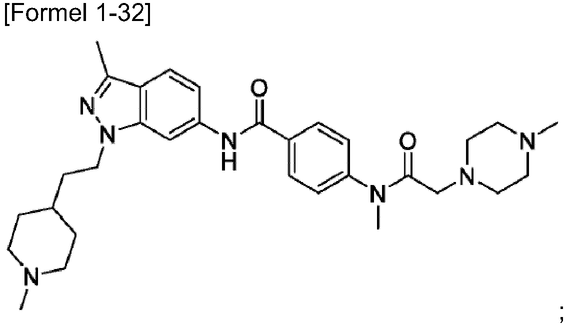 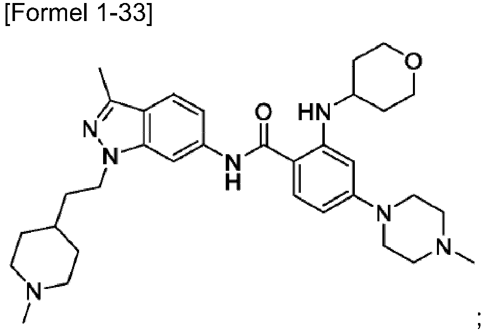 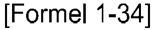 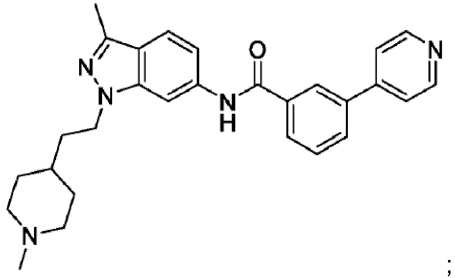 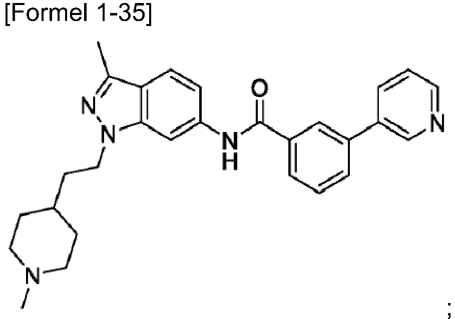 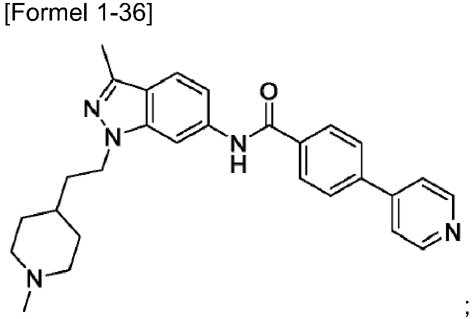 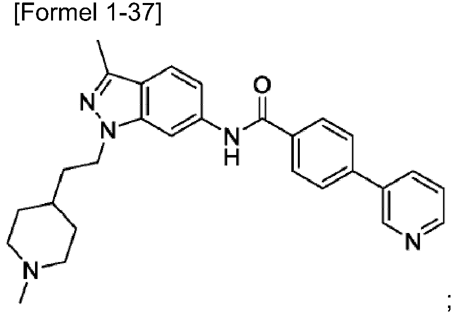 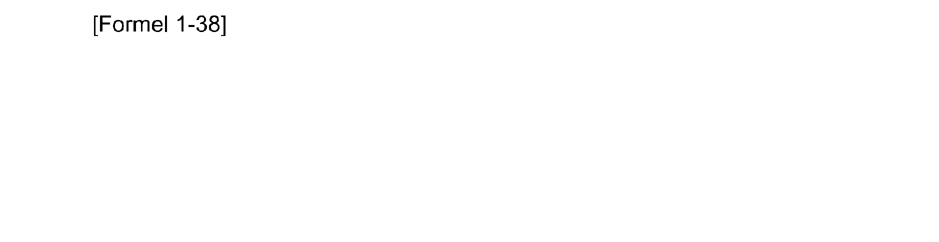 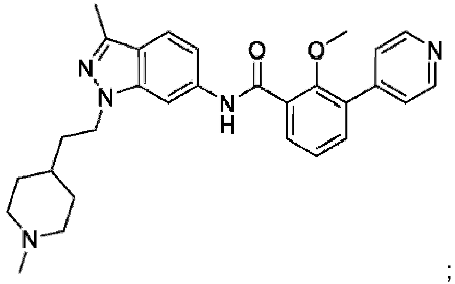 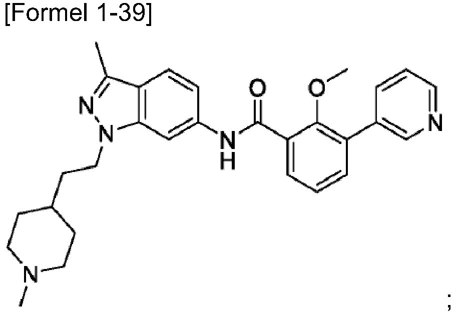 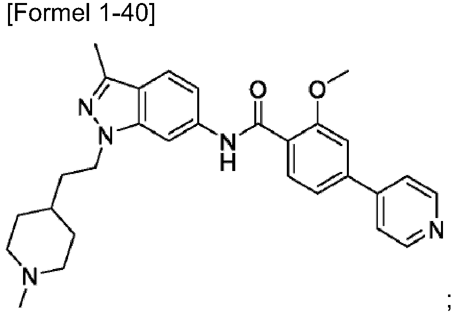 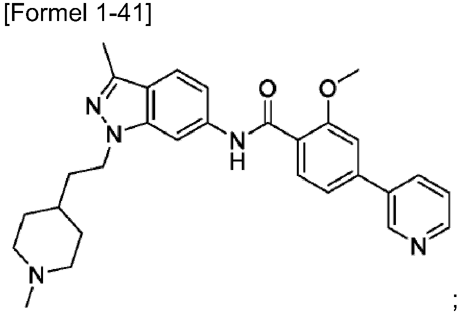 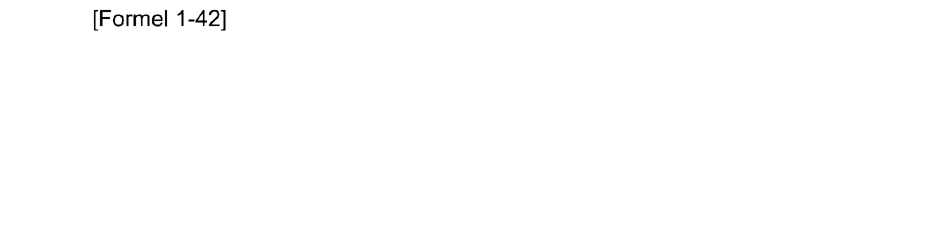 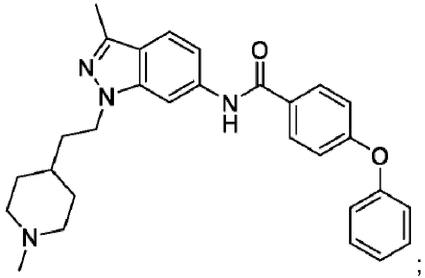 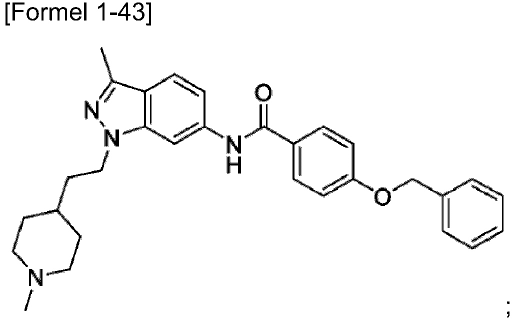 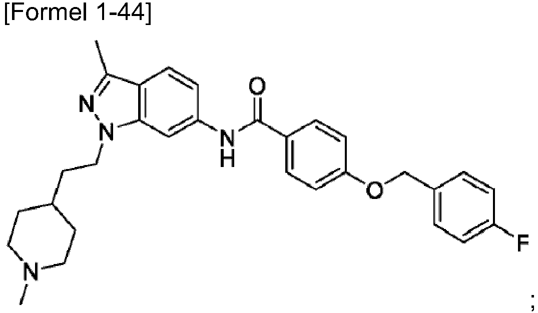 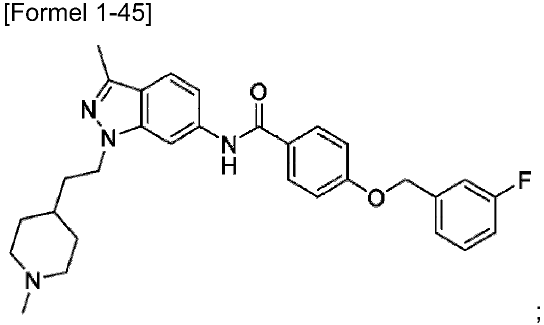 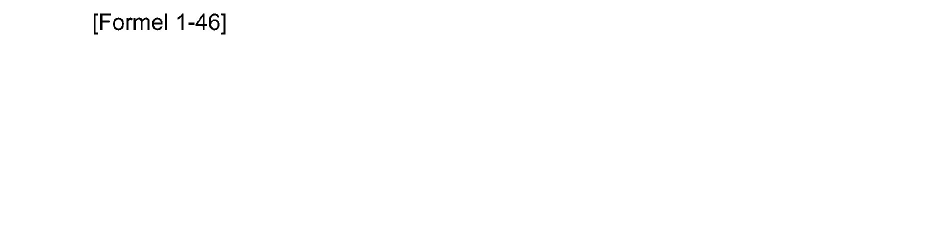 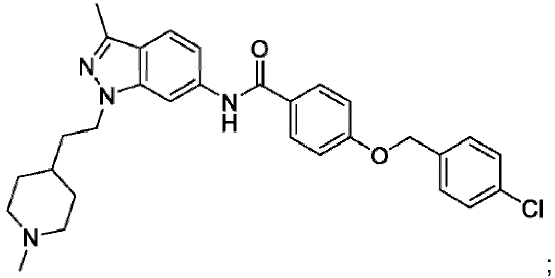 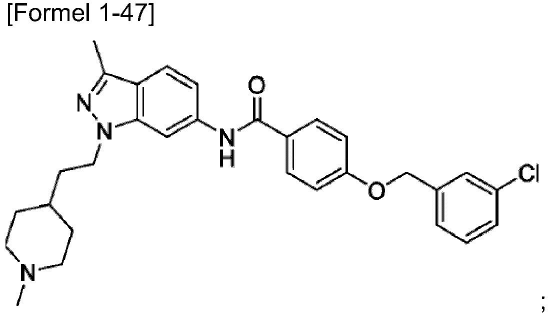 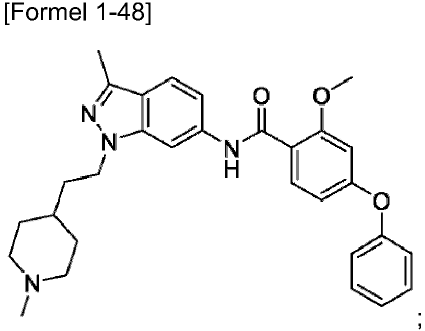 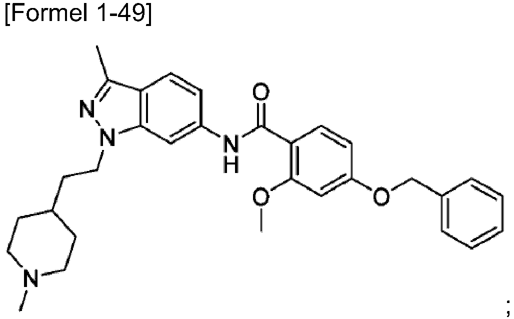 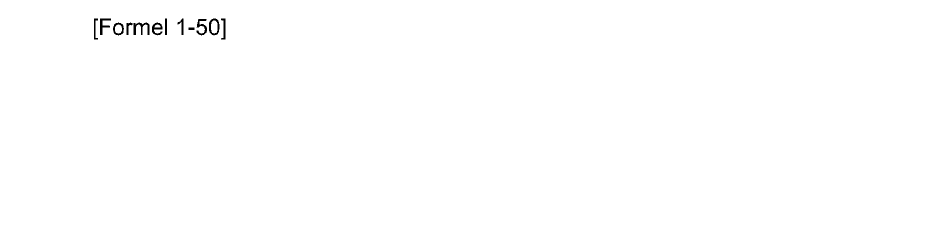 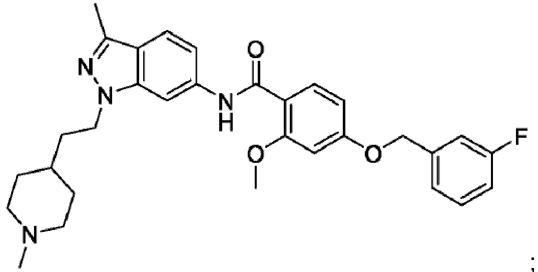 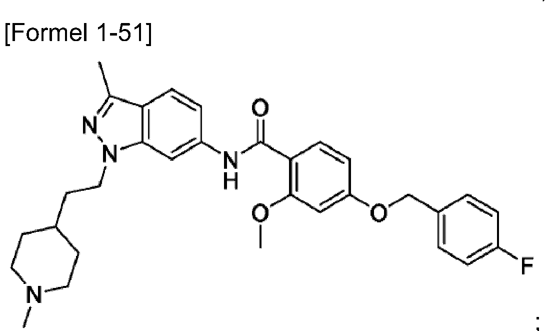 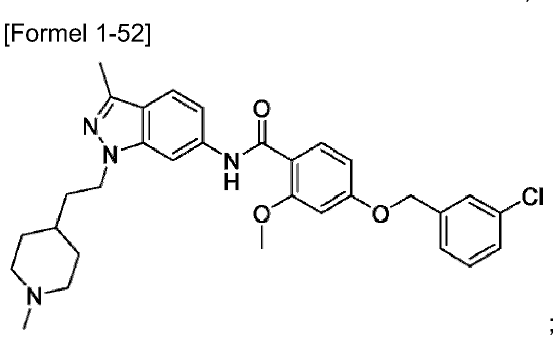 und

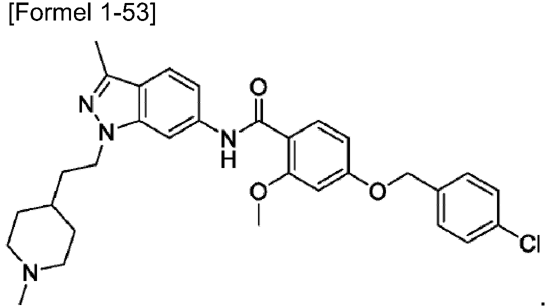

3. Pharmazeutische Zusammensetzung zur Verwendung in einem Verfahren zur Vorbeugung oder Behandlung von Krebs, umfassend das Indazol-Derivat, welches durch [Formel 1] dargestellt ist, oder ein pharmazeutisch annehmbares Salz davon nach Anspruch 1 als einen Wirkstoff.

4. Pharmazeutische Zusammensetzung zur Verwendung nach Anspruch 3, **dadurch gekennzeichnet, dass** das Indazol-Derivat durch Hemmung von Hsp90 Zelltod induziert.

5. Pharmazeutische Zusammensetzung zur Verwendung nach Anspruch 3, wobei der Krebs einer oder mehrere ist, ausgewählt aus der Gruppe bestehend aus Hautkrebs, Brustkrebs, Gebärmutterkrebs, Speiseröhrenkrebs, Magenkrebs, einem Hirntumor, Dickdarmkrebs, Mastdarmkrebs, Kolorektalkrebs, Lungenkrebs, Eierstockkrebs, Gebärmutterhalskrebs, Endometriumkrebs, Vulvakrebs, Nierenkrebs, Blutkrebs, Bauchspeicheldrüsenkrebs, Prostatakrebs, Hodenkrebs, Kehlkopfkrebs, Kopf- und Hals-Krebs, Schilddrüsenkrebs, Leberkrebs, Blasenkrebs, Osteosarkom, Lymphom, Leukämie, Thymuskrebs, Harnröhrenkrebs, Bronchialkrebs und Kombinationen davon.

6. Pharmazeutische Zusammensetzung zur Verwendung nach Anspruch 3, **dadurch gekennzeichnet, dass** die pharmazeutische Zusammensetzung das Indazol-Derivat oder ein pharmazeutisch annehmbares Salz davon umfasst; und ferner eine oder mehrere zusätzliche Komponenten umfasst, welche ausgewählt sind aus der Gruppe bestehend aus einem pharmazeutisch annehmbaren Träger, Hilfsstoff, Verdünnungsmittel, Stabilisator und Konservierungsmittel.

7. Pharmazeutische Zusammensetzung zur Verwendung nach Anspruch 3, **dadurch gekennzeichnet, dass** die pharmazeutische Zusammensetzung das Indazol-Derivat oder ein pharmazeutisch annehmbares Salz davon umfasst; und ferner ein Antikrebsmittel umfasst.

8. Pharmazeutische Zusammensetzung zur Verwendung nach Anspruch 7, wobei das Antikrebsmittel eines oder mehrere ist, ausgewählt aus der Gruppe bestehend aus Paclitaxel, Docetaxel, Doxorubicin, Sorafenib, Vemurafenib, Irinotecan, Cisplatin, Alpharadin, Mitoxantron, Cyclophosphamid, Vinblastin, Carboplatin, Actinomycin-D, Etoposid, Teniposid, Bisantren, Homoharringtonin, Gleevec (STI-571), 5-Fluorouracil, Busulfan, Chlorambucil, Melphalan, Stickstofflost, Nitrosoharnstoffen und Kombinationen davon.

9. Pharmazeutische Zusammensetzung zur Verwendung nach Anspruch 3, **dadurch gekennzeichnet, dass** die pharmazeutische Zusammensetzung eine oder mehrere Darreichungsform aufweist, welche ausgewählt ist aus der Gruppe bestehend aus Pulvern, Granulaten, Tabletten, Kapseln und Injektionen.

10. Verfahren zur Herstellung eines Indazol-Derivats, welches durch [Formel 1] dargestellt ist, oder eines pharmazeutisch annehmbaren Salzes davon, umfassend Umsetzen einer Verbindung, welche durch [Formel 2] oder [Formel 3] dargestellt ist, mit einer Verbindung, welche durch [Formel 4] oder [Formel 5] dargestellt ist: 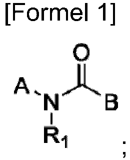 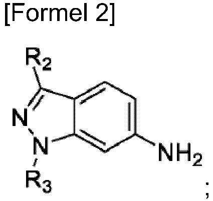 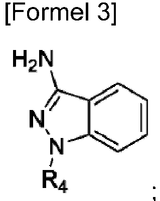 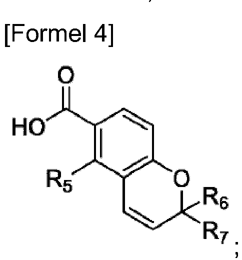 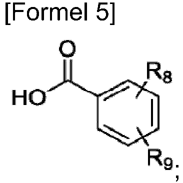 wobei in Formel 1,
A 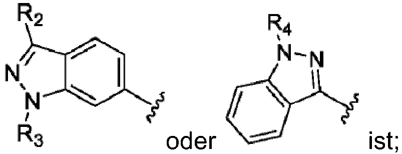
B 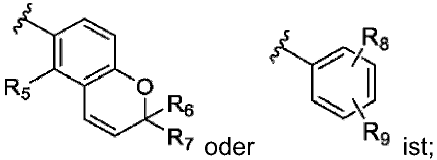
R₁ Wasserstoff oder eine kettenförmige C₁-C₆-Alkylgruppe ist;
wobei in Formeln 1 und 2,
R₂ Wasserstoff oder eine kettenförmige C₁-C₆-Alkylgruppe ist;
R₃ Wasserstoff oder eine kettenförmige C₁-C₆-Alkylgruppe ist, wobei mindestens ein Wasserstoff der Alkylgruppe unsubstituiert oder mit einer C₁-C₆-Alkoxygruppe, einer C₅-C₁₀-Heterocycloalkylgruppe oder einer C₅-C₁₀-Heteroarylgruppe substituiert ist, wobei die Alkoxygruppe unsubstituiert oder mit einer C₁-C₆-Alkoxygruppe substituiert ist und die Heterocycloalkylgruppe unsubstituiert oder mit einer kettenförmigen C₁-C₆-Alkylgruppe, 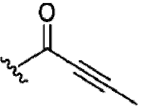 oder 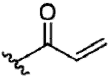 substituiert ist;
wobei in Formeln 1 und 3,
R₄ Wasserstoff oder eine kettenförmige C₁-C₆-Alkylgruppe ist, wobei mindestens ein Wasserstoff der Alkylgruppe unsubstituiert oder mit einer C₁-C₆-Alkoxygruppe, einer C₅-C₁₀-Heterocycloalkylgruppe oder einer C₅-C₁₀-Heteroarylgruppe substituiert ist, wobei die Alkoxygruppe unsubstituiert oder mit einer C₁-C₆-Alkoxygruppe substituiert ist und die Heterocycloalkylgruppe unsubstituiert oder mit einer kettenförmigen C₁-C₆-Alkylgruppe, 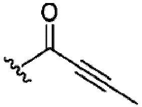
oder 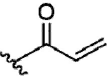 substituiert ist,
wobei in Formeln 1 und 4,
R₅ eine Halogengruppe oder eine C₁-C₆-Alkoxygruppe ist;
R₆ und R₇ identisch oder verschieden voneinander sind und jeweils eine kettenförmige C₁-C₆-Alkylgruppe darstellen;
wobei in Formeln 1 und 5,
R₈ NR₁₀C(O)R₁₁, OR₁₂, eine C₅-C₁₀-Heteroarylgruppe oder eine Tetrahydropyranylamingruppe ist;
R₉ Wasserstoff, eine C₁-C₆-Alkoxygruppe oder eine C₅-C₁₀-Heterocycloalkylgruppe ist, wobei die Heterocycloalkylgruppe unsubstituiert oder mit einer kettenförmigen C₁-C₆-Alkylgruppe substituiert ist;
R₁₀ und R₁₁ identisch oder verschieden voneinander sind und jeweils eine kettenförmige C₁-C₆-Alkylgruppe oder C₅-C₁₀-Arylgruppe darstellen, wobei die Alkylgruppe unsubstituiert oder mit einer Methylpiperazinylgruppe substituiert ist;
R₁₂ eine C₅-C₁₀-Arylgruppe oder eine C₅-C₁₀-Benzylgruppe ist, wobei die Benzylgruppe unsubstituiert oder mit einer Halogengruppe substituiert ist.

11. Verfahren nach Anspruch 10, **dadurch gekennzeichnet, dass** die Verbindung, welche durch die [Formel 2] dargestellt ist, eine oder mehrere ist, ausgewählt aus der Gruppe bestehend aus den Verbindungen, welche durch die folgenden Formeln dargestellt sind: 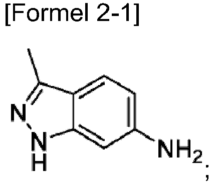 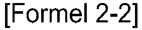 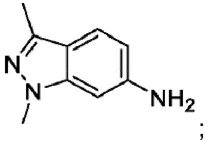 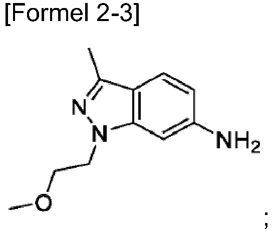 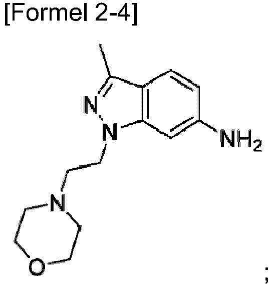 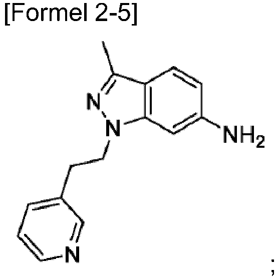 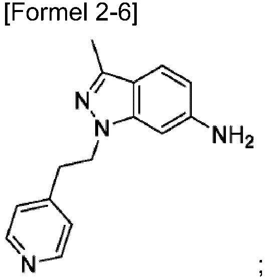 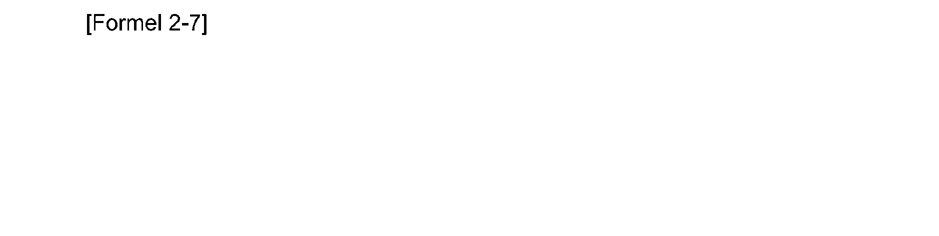 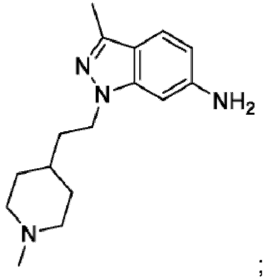 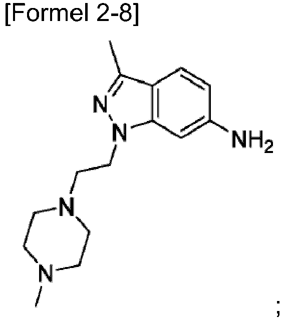 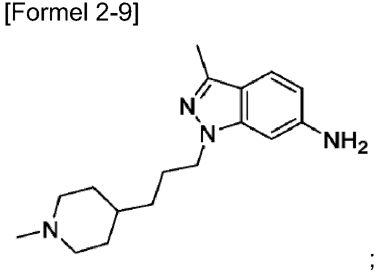 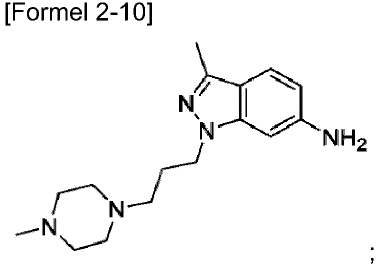 und

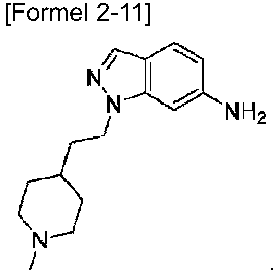

12. Verfahren nach Anspruch 10, **dadurch gekennzeichnet, dass** die Verbindung, welche durch die [Formel 3] dargestellt ist, eine oder mehrere ist, ausgewählt aus der Gruppe bestehend aus den Verbindungen, welche durch die folgenden Formeln dargestellt sind: 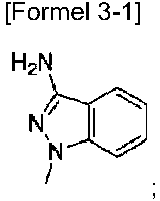 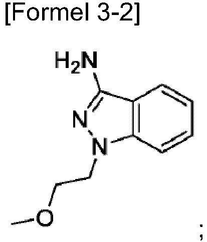 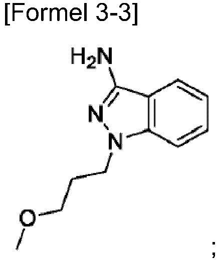 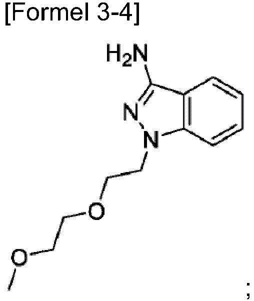 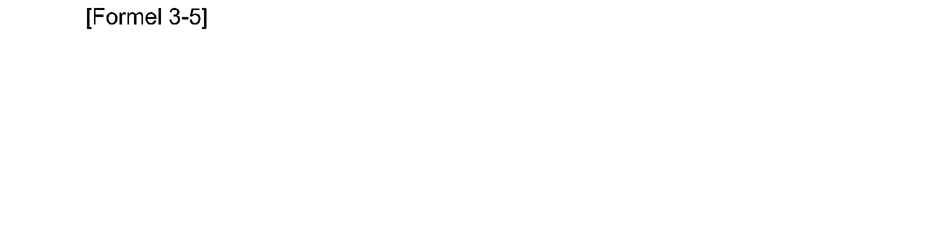 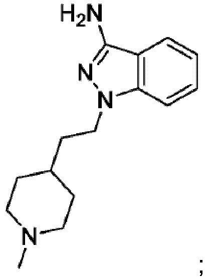 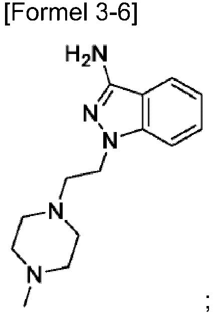 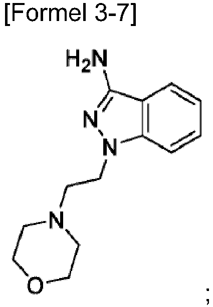 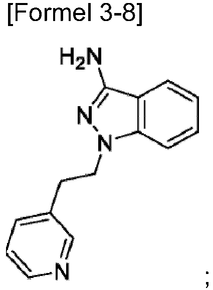 und

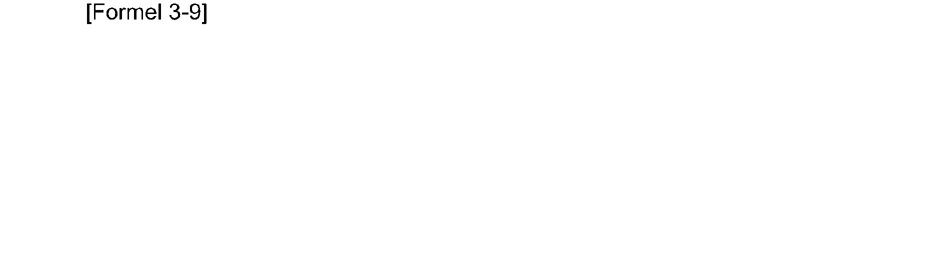

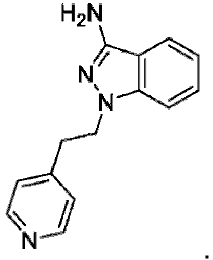

13. Verfahren nach Anspruch 10, **dadurch gekennzeichnet, dass** die Verbindung, welche durch die [Formel 4] dargestellt ist, eine oder mehrere ist, ausgewählt aus der Gruppe bestehend aus den Verbindungen, welche durch die folgenden Formeln dargestellt sind: 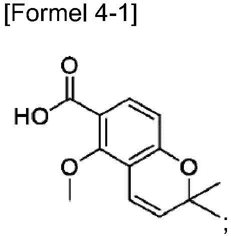 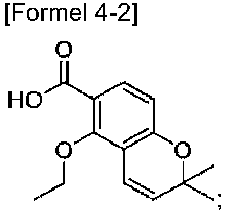 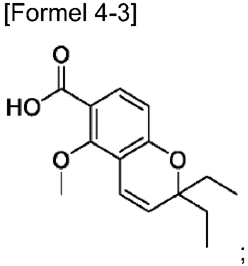 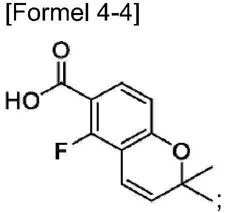 und

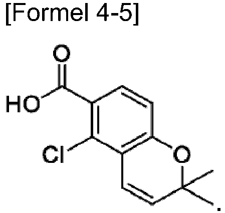

14. Verfahren nach Anspruch 10, **dadurch gekennzeichnet, dass** die Verbindung, welche durch die [Formel 5] dargestellt ist, eine oder mehrere ist, ausgewählt aus der Gruppe bestehend aus den Verbindungen, welche durch die folgenden Formeln dargestellt sind: 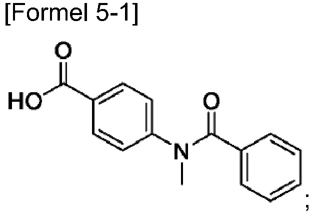 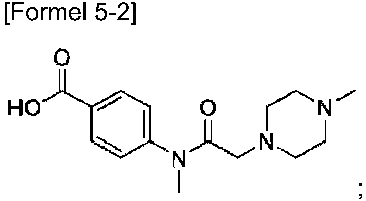 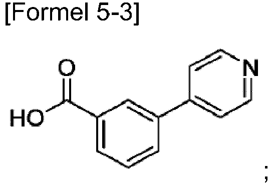 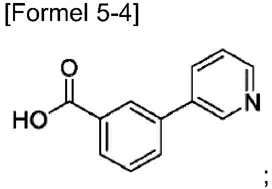 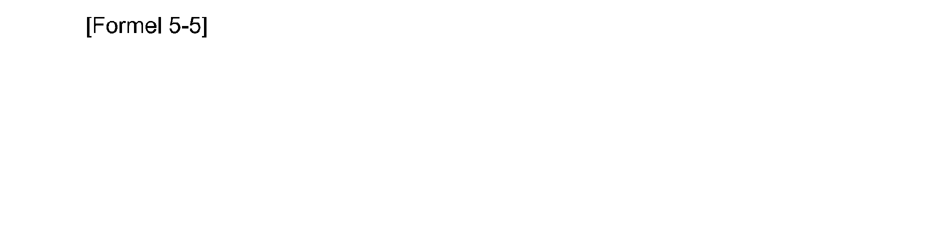 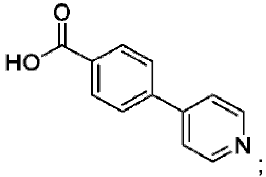 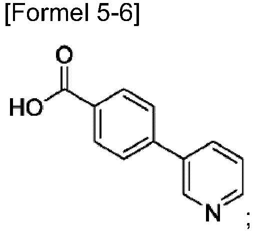 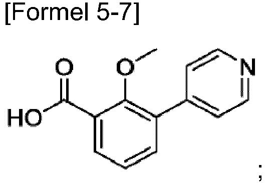 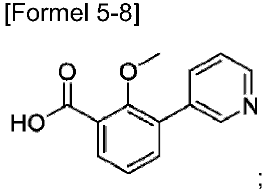 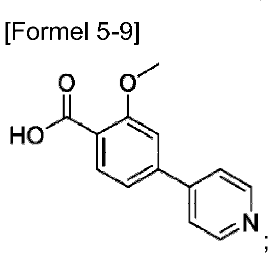 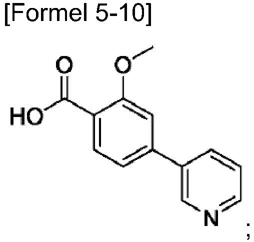 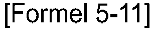 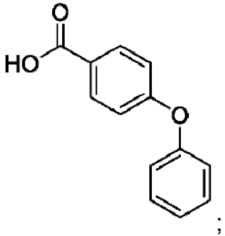 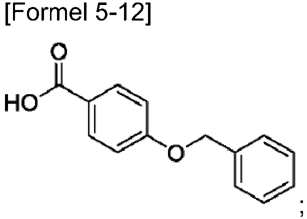 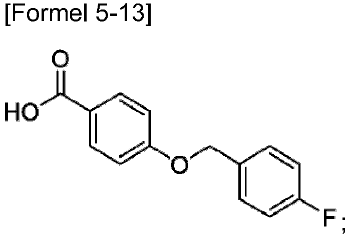 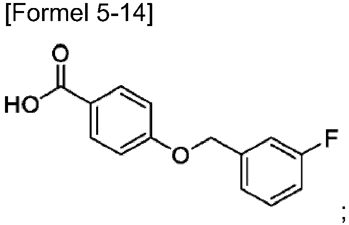 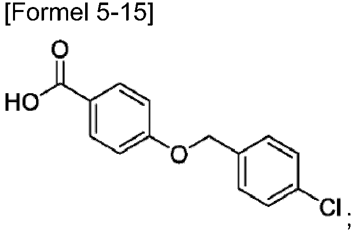 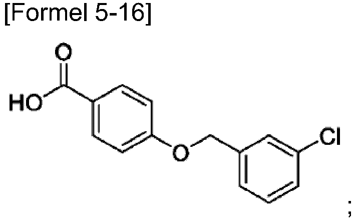 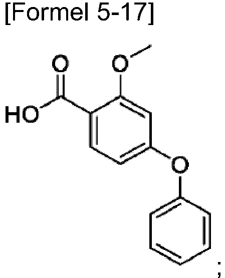 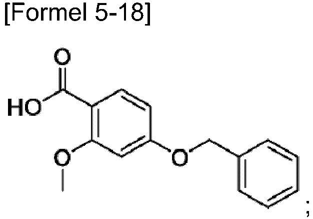 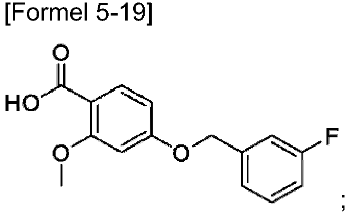 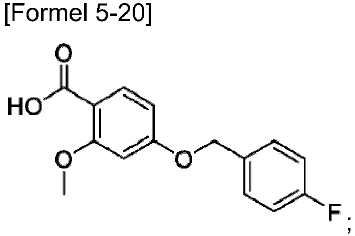 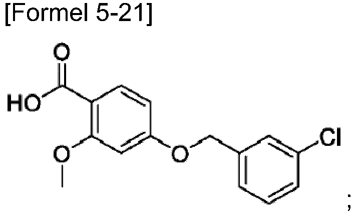 und

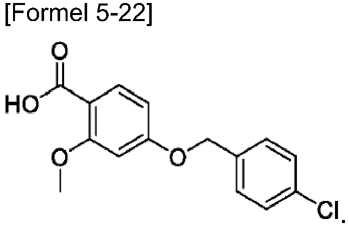

15. Verfahren nach Anspruch 10, **dadurch gekennzeichnet, dass** die Verbindung, welche durch die [Formel 2] oder [Formel 3] dargestellt ist, umgesetzt wird, indem die Verbindung, welche durch die [Formel 2] oder [Formel 3] dargestellt ist, zu einer Mischung aus der Verbindung, welche durch die [Formel 4] oder [Formel 5] dargestellt ist, Hydroxybenzotriazol (HOBt), Triethylamin (Et₃N) und EDC·HCl gegeben wird.

## Revendications

1. Dérivé d'indazole représenté par la [Formule 1] suivante ou sel pharmaceutiquement acceptable de celui-ci: 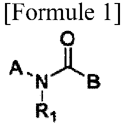 où, dans la Formule 1,
A est 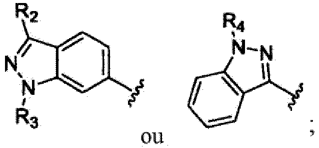
B est 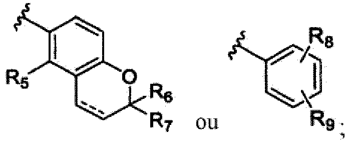
R₁ et R₂ sont identiques ou différents l'un de l'autre, et chacun représente un atome d'hydrogène ou un groupe alkyle à chaîne ouverte en C₁-C₆;
R₃ et R₄ sont identiques ou différents l'un de l'autre, et chacun représente un atome d'hydrogène ou un groupe alkyle à chaîne ouverte en C₁-C₆, où au moins un atome d'hydrogène du groupe alkyle est non substitué ou substitué par un groupe alcoxy en C₁-C₆, un groupe hétérocycloalkyle en C₅-C₁₀, ou un groupe hétéroaryle en C₅-C₁₀, où le groupe alcoxy est non substitué ou substitué par un groupe alcoxy en C₁-C₆, et le groupe hétérocycloalkyle est non substitué ou substitué par un groupe alkyle à chaîne ouverte en C₁-C₆, 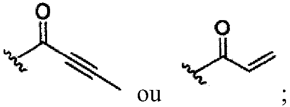
R₅ est un groupe halogène ou un groupe alcoxy en C₁-C₆;
R₆ et R₇ sont identiques ou différents l'un de l'autre, et chacun représente un groupe alkyle à chaîne ouverte en C₁-C₆;
R₈ est NR₁₀C(O)R₁₁, OR₁₂, un groupe hétéroaryle en C₅-C₁₀, ou un groupe tétrahydropyranyl-amine;
R₉ est un atome d'hydrogène, un groupe alcoxy en C₁-C₆, ou un groupe hétérocycloalkyle en C₅-C₁₀, où le groupe hétérocycloalkyle est non substitué ou substitué par un groupe alkyle à chaîne ouverte en C₁-C₆;
R₁₀ et R₁₁ sont identiques ou différents l'un de l'autre, et chacun représente un groupe alkyle à chaîne ouverte en C₁-C₆ ou un groupe aryle en C₅-C₁₀, où le groupe alkyle est non substitué ou substitué par un groupe méthylpipérazinyle;
R₁₂ est un groupe aryle en C₅-C₁₀ ou un groupe benzyle en C₅-C₁₀, où le groupe benzyle est non substitué ou substitué par un groupe halogène;
- - - est une simple liaison ou une double liaison.

2. Dérivé d'indazole ou sel pharmaceutiquement acceptable de celui-ci selon la revendication 1, **caractérisé en ce que** le dérivé d'indazole représenté par la [Formule 1] est l'un quelconque ou plusieurs choisis dans le groupe consistant en les composés représentés par les formules suivantes: 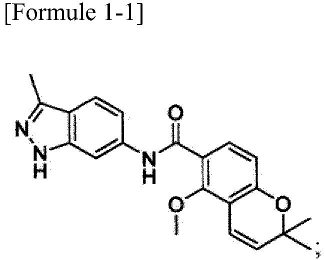 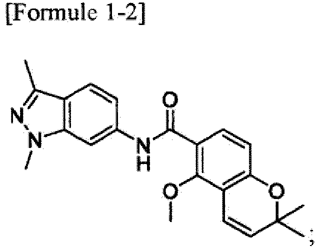 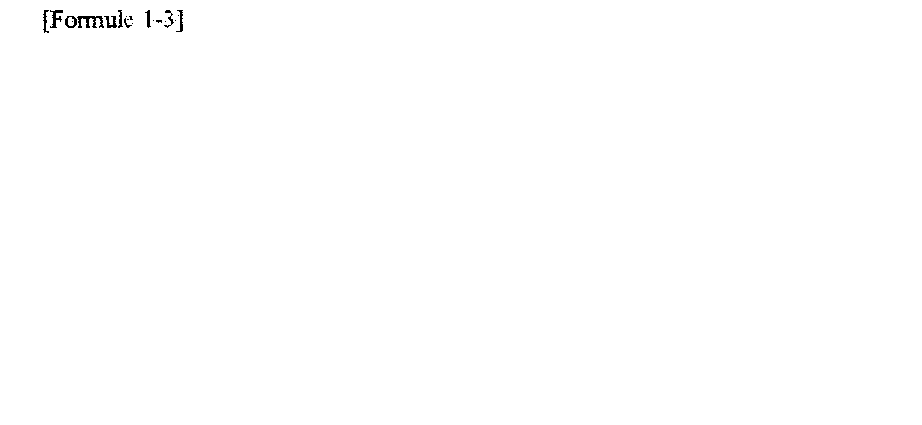 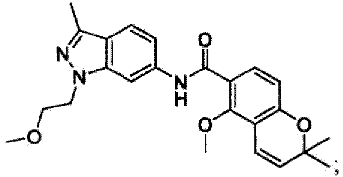 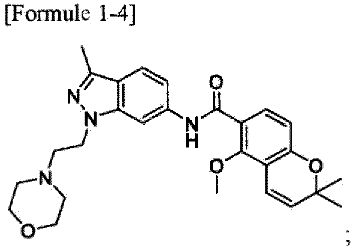 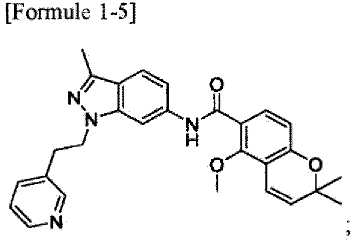 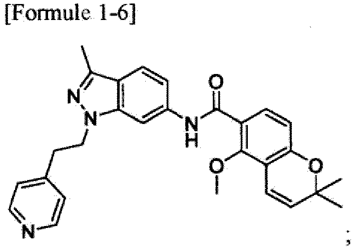 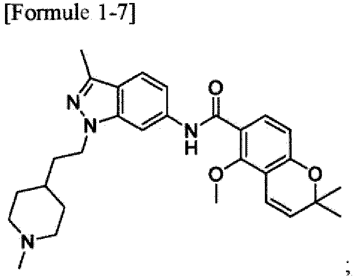 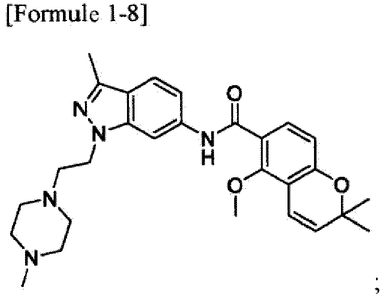 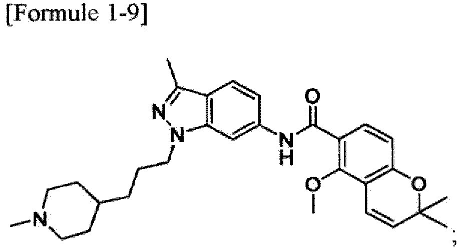 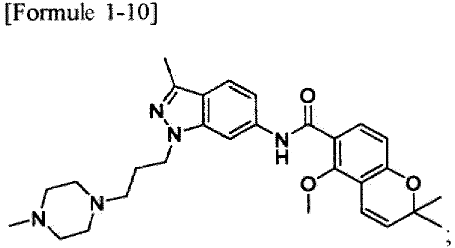 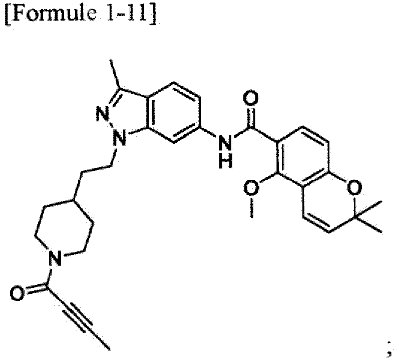 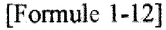 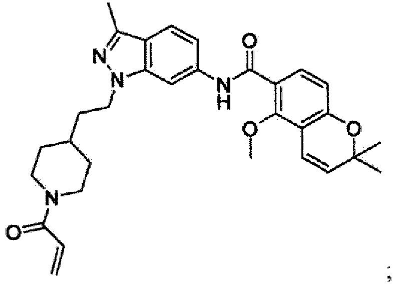 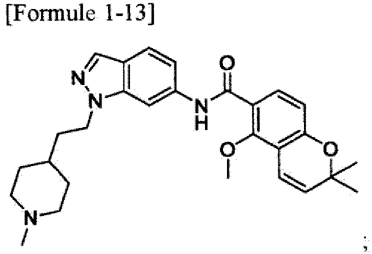 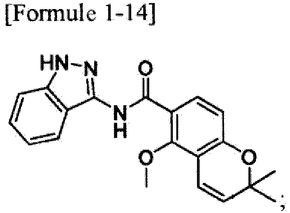 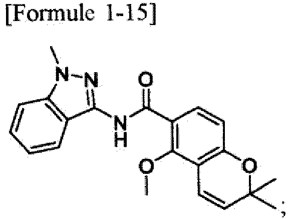 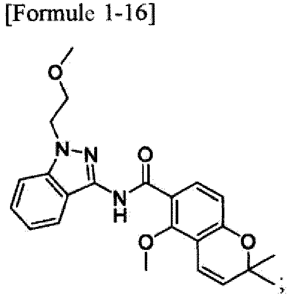 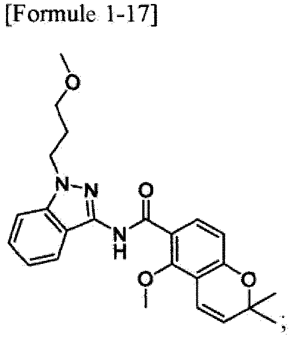 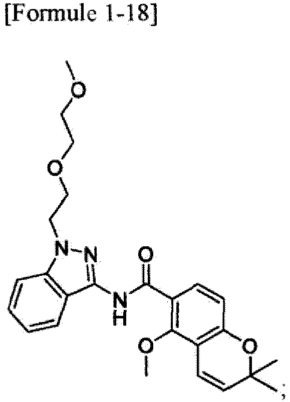 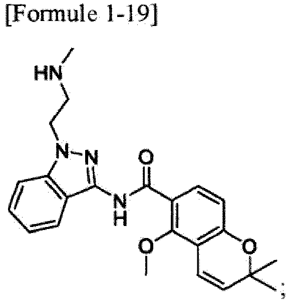 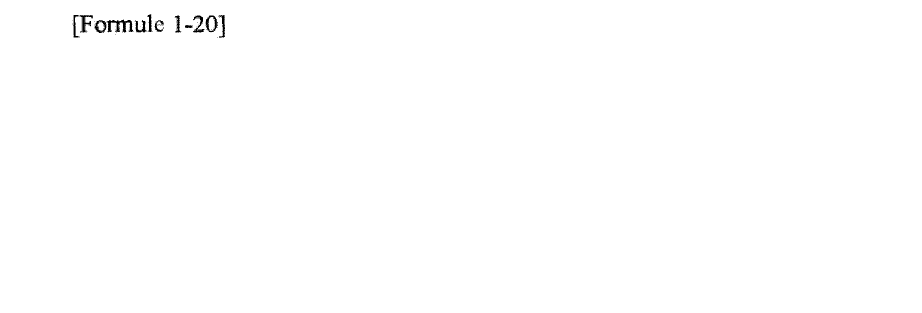 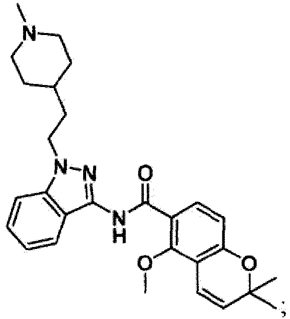 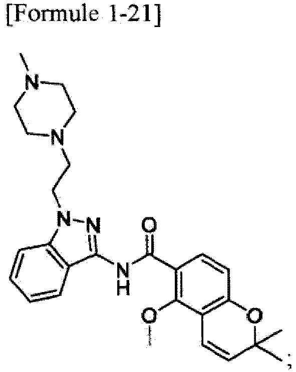 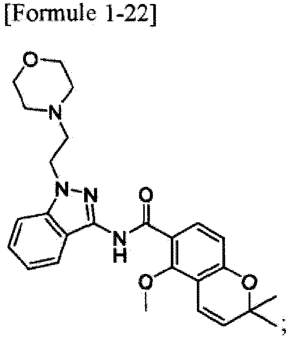 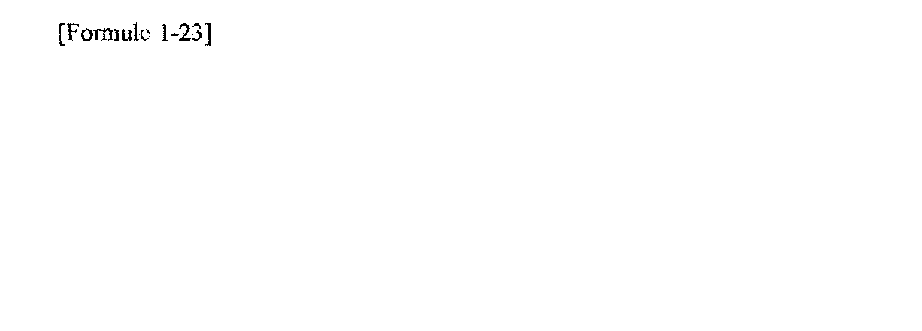 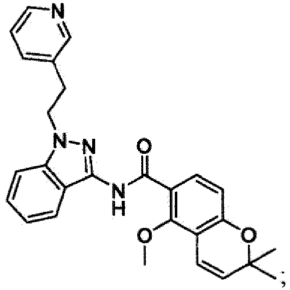 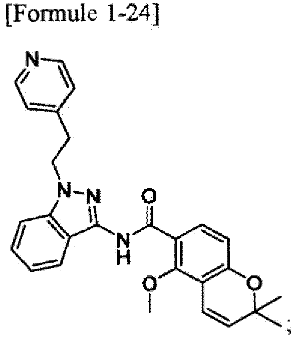 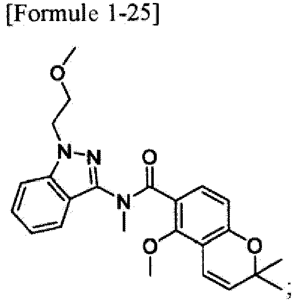 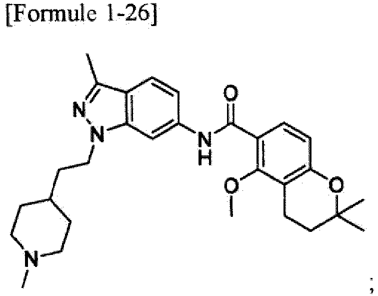 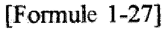 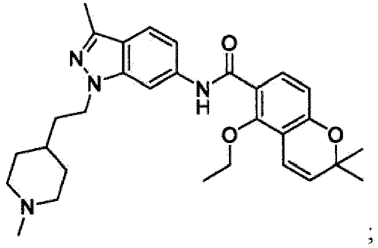 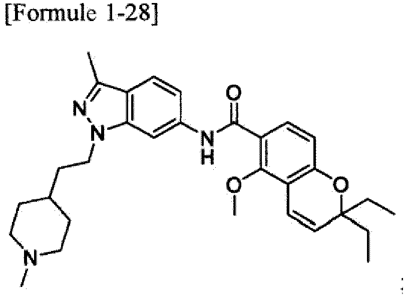 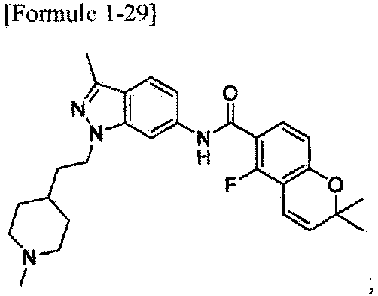 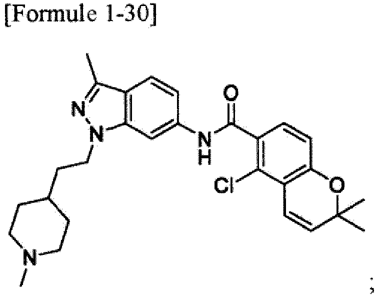 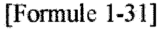 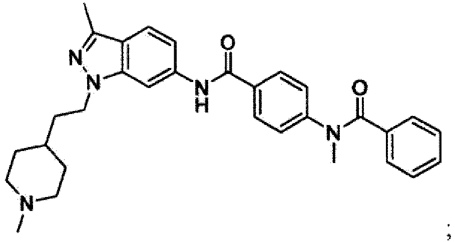 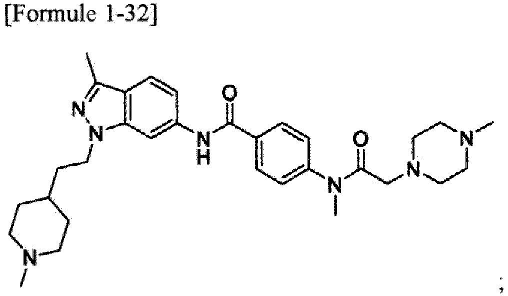 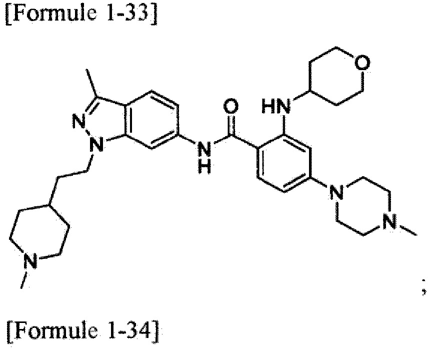 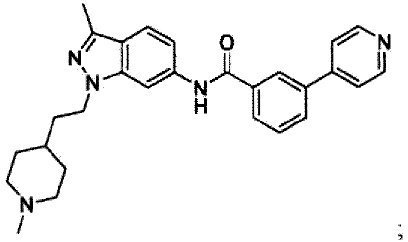 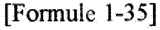 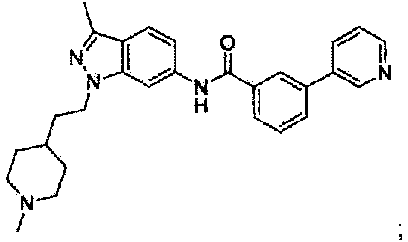 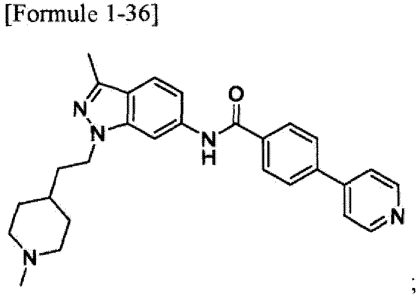 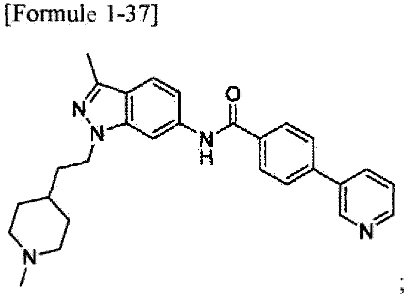 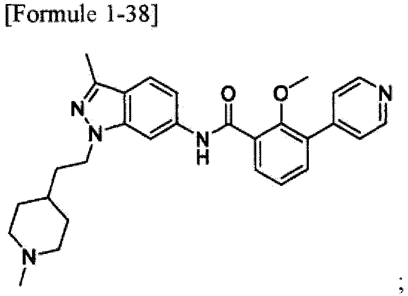 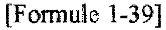 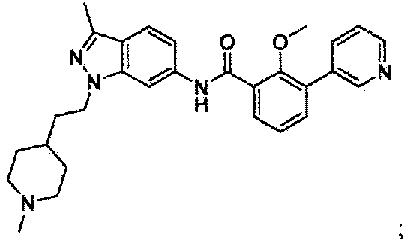 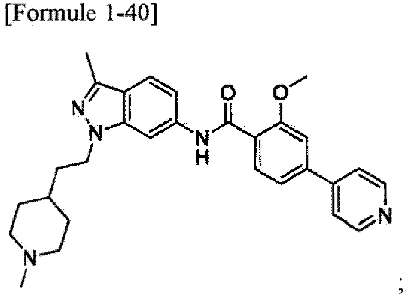 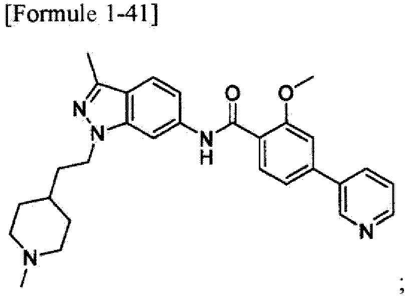 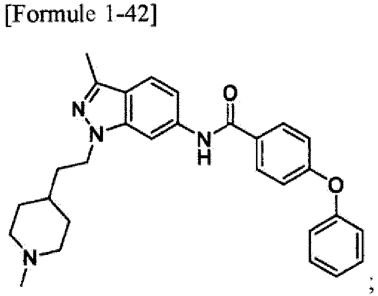 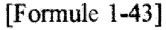 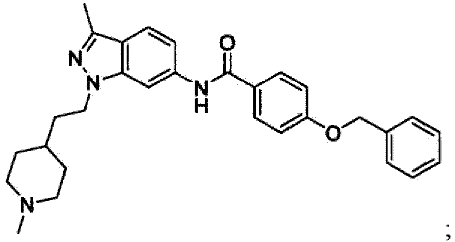 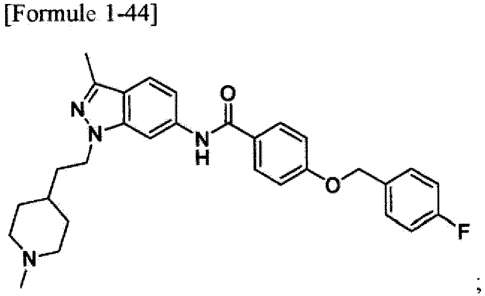 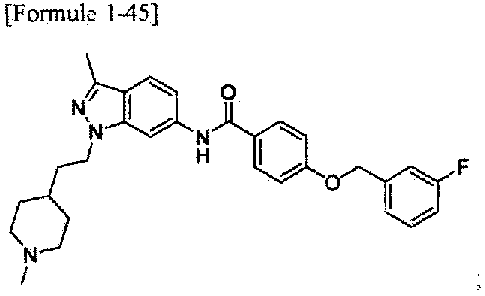 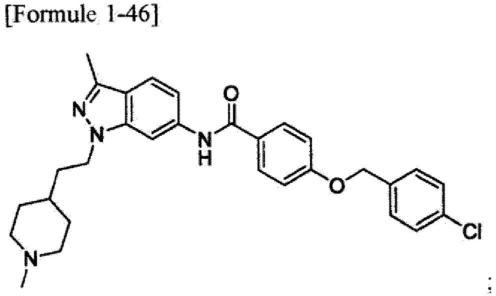 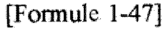 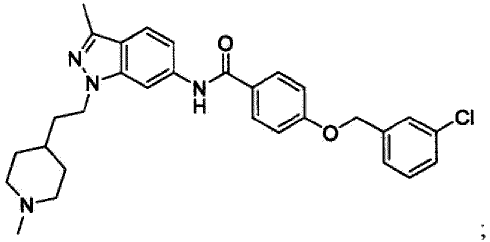 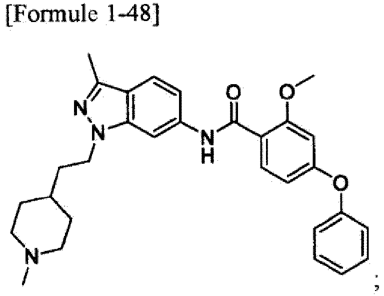 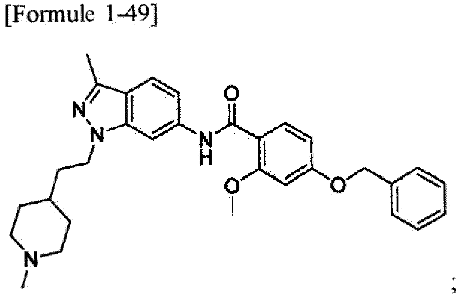 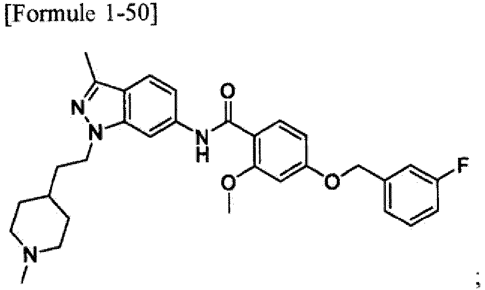 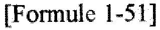 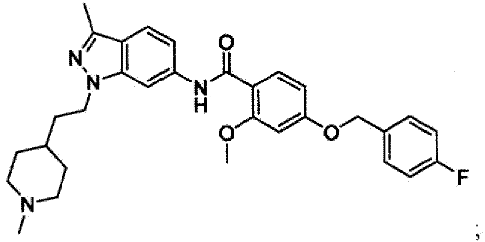 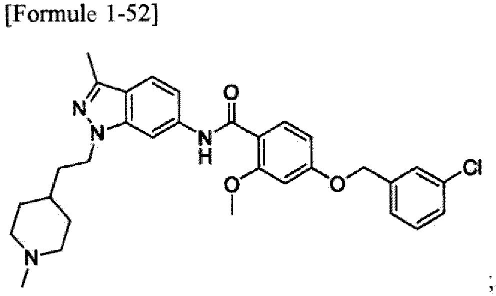 et

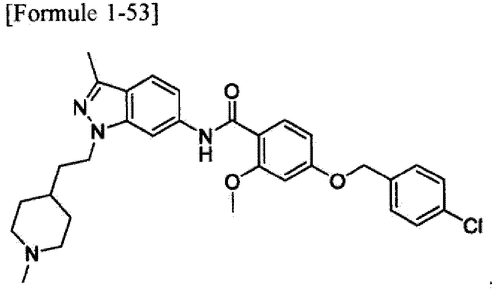

3. Composition pharmaceutique destinée à être utilisée dans un procédé de prévention ou de traitement du cancer, comprenant le dérivé d'indazole représenté par la [Formule 1] ou un sel pharmaceutiquement acceptable de celui-ci selon la revendication 1 en tant qu'ingrédient actif.

4. Composition pharmaceutique destinée à être utilisée selon la revendication 3, **caractérisée en ce que** le dérivé d'indazole induit la mort cellulaire en inhibant Hsp90.

5. Composition pharmaceutique destinée à être utilisée selon la revendication 3, où le cancer est l'un quelconque ou plusieurs choisis dans le groupe consistant en le cancer de la peau, le cancer du sein, le cancer de l'utérus, le cancer de l'œsophage, le cancer de l'estomac, une tumeur cérébrale, le cancer du côlon, le cancer du rectum, le cancer colorectal, le cancer du poumon, le cancer de l'ovaire, le cancer du col de l'utérus, le cancer de l'endomètre, le cancer de la vulve, le cancer du rein, le cancer du sang, le cancer du pancréas, le cancer de la prostate, le cancer du testicule, le cancer du larynx, le cancer de la tête et du cou, le cancer de la thyroïde, le cancer du foie, le cancer de la vessie, l'ostéosarcome, le lymphome, la leucémie, le cancer du thymus, le cancer de l'urètre, le cancer bronchique, et leurs combinaisons.

6. Composition pharmaceutique destinée à être utilisée selon la revendication 3, **caractérisée en ce que** la composition pharmaceutique comprend le dérivé d'indazole ou un sel pharmaceutiquement acceptable de celui-ci; et comprend en outre l'un quelconque ou plusieurs composants supplémentaires choisis dans le groupe consistant en un véhicule, un excipient, un diluant, un stabilisant et un conservateur pharmaceutiquement acceptables.

7. Composition pharmaceutique destinée à être utilisée selon la revendication 3, **caractérisée en ce que** la composition pharmaceutique comprend le dérivé d'indazole ou un sel pharmaceutiquement acceptable de celui-ci; et comprend en outre un agent anticancéreux.

8. Composition pharmaceutique destinée à être utilisée selon la revendication 7, où l'agent anticancéreux est l'un quelconque ou plusieurs choisis dans le groupe consistant en le paclitaxel, le docétaxel, la doxorubicine, le sorafénib, le vémurafénib, l'irinotécan, le cisplatine, l'alpharadine, la mitoxantrone, le cyclophosphamide, la vinblastine, le carboplatine, l'actinomycine D, l'étoposide, le téniposide, le bisantrène, l'homoharringtonine, le Gleevec (STI-571), le 5-fluorouracile, le busulfan, le chlorambucil, le melphalan, les moutardes azotées, les nitrosourées, et leurs combinaisons.

9. Composition pharmaceutique destinée à être utilisée selon la revendication 3, **caractérisée en ce que** la composition pharmaceutique a l'une quelconque ou plusieurs formes galéniques choisies dans le groupe consistant en les poudres, les granulés, les comprimés, les gélules et les préparations injectables.

10. Procédé de production d'un dérivé d'indazole représenté par la [Formule 1] ou d'un sel pharmaceutiquement acceptable de celui-ci, comprenant la réaction d'un composé représenté par la [Formule 2] ou la [Formule 3] avec un composé représenté par la [Formule 4] ou la [Formule 5]: 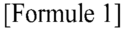 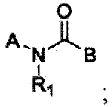 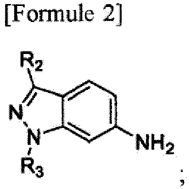 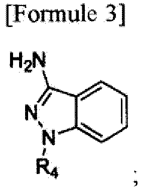 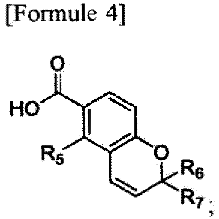 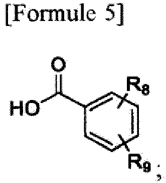 où, dans la Formule 1,
A est 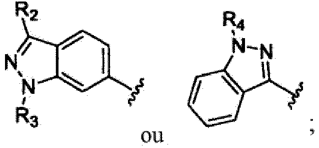
B est 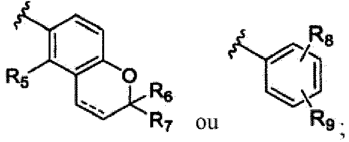
R₁ est un atome d'hydrogène ou un groupe alkyle à chaîne ouverte en C₁-C₆;
où, dans les Formules 1 et 2,
R₂ est un atome d'hydrogène ou un groupe alkyle à chaîne ouverte en C₁-C₆;
R₃ est un atome d'hydrogène ou un groupe alkyle à chaîne ouverte en C₁-C₆, où au moins un atome d'hydrogène du groupe alkyle est non substitué ou substitué par un groupe alcoxy en C₁-C₆, un groupe hétérocycloalkyle en C₅-C₁₀, ou un groupe hétéroaryle en C₅-C₁₀, où le groupe alcoxy est non substitué ou substitué par un groupe alcoxy en C₁-C₆, et le groupe hétérocycloalkyle est non substitué ou substitué par un groupe alkyle à chaîne ouverte en C₁-C₆, 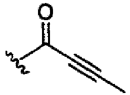
ou 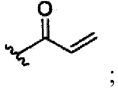
où, dans les Formules 1 et 3,
R₄ est un atome d'hydrogène ou un groupe alkyle à chaîne ouverte en C₁-C₆,
où au moins un atome d'hydrogène du groupe alkyle est non substitué ou substitué par un groupe alcoxy en C₁-C₆, un groupe hétérocycloalkyle en C₅-C₁₀, ou un groupe hétéroaryle en C₅-C₁₀, où le groupe alcoxy est non substitué ou substitué par un groupe alcoxy en C₁-C₆, et le groupe hétérocycloalkyle est non substitué ou substitué par un groupe alkyle à chaîne ouverte en C₁-C₆, 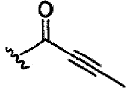 ou 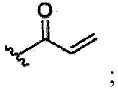
où, dans les Formules 1 et 4,
R₅ est un groupe halogène ou un groupe alcoxy en C₁-C₆;
R₆ et R₇ sont identiques ou différents l'un de l'autre, et chacun représente un groupe alkyle à chaîne ouverte en C₁-C₆;
où, dans les Formules 1 et 5,
R₈ est NR₁₀C(O)R₁₁, OR₁₂, un groupe hétéroaryle en C₅-C₁₀, ou un groupe tétrahydropyranyl-amine;
R₉ est un atome d'hydrogène, un groupe alcoxy en C₁-C₆, ou un groupe hétérocycloalkyle en C₅-C₁₀, où le groupe hétérocycloalkyle est non substitué ou substitué par un groupe alkyle à chaîne ouverte en C₁-C₆;
R₁₀ et R₁₁ sont identiques ou différents l'un de l'autre, et chacun représente un groupe alkyle à chaîne ouverte en C₁-C₆ ou un groupe aryle en C₅-C₁₀, où le groupe alkyle est non substitué ou substitué par un groupe méthylpipérazinyle;
R₁₂ est un groupe aryle en C₅-C₁₀ ou un groupe benzyle en C₅-C₁₀, où le groupe benzyle est non substitué ou substitué par un groupe halogène.

11. Procédé selon la revendication 10, **caractérisé en ce que** le composé représenté par la [Formule 2] est l'un quelconque ou plusieurs choisis dans le groupe consistant en les composés représentés par les formules suivantes: 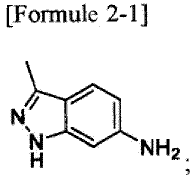 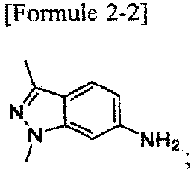 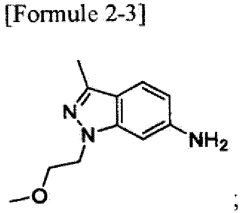 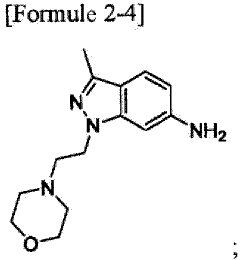 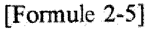 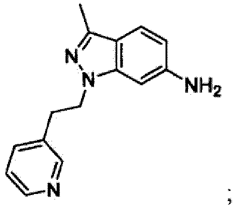 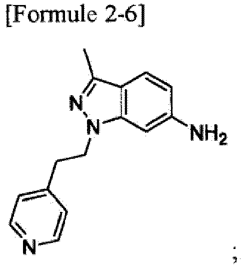 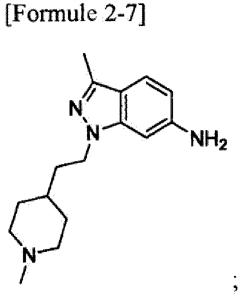 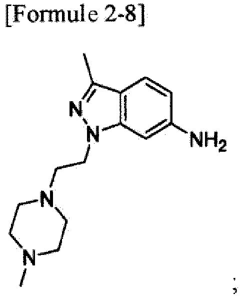 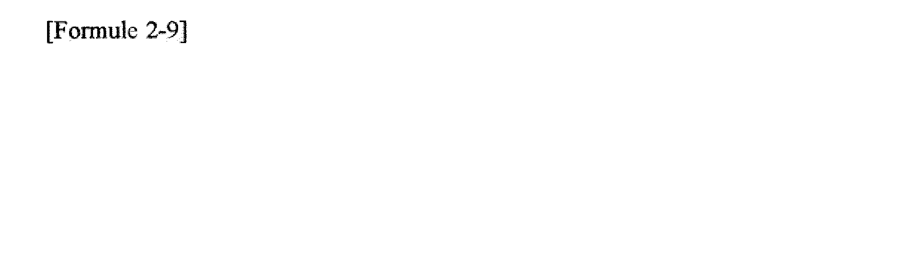 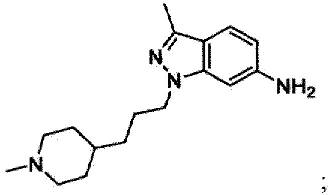 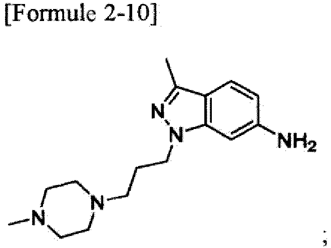 et

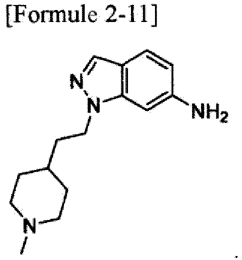

12. Procédé selon la revendication 10, **caractérisé en ce que** le composé représenté par la [Formule 3] est l'un quelconque ou plusieurs choisis dans le groupe consistant en les composés représentés par les formules suivantes: 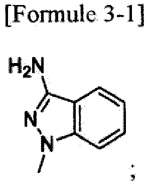 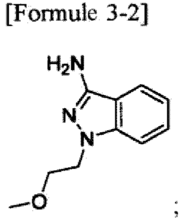 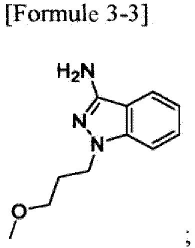 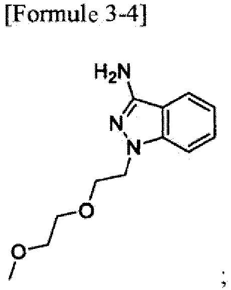 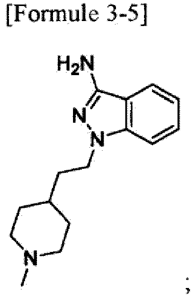 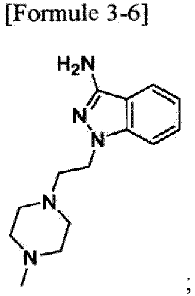 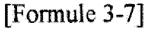 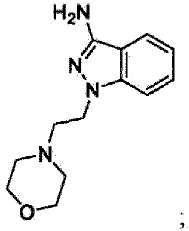 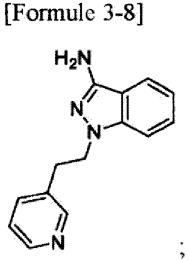 et

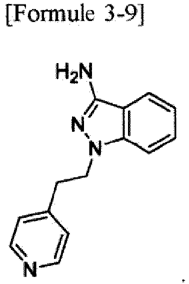

13. Procédé selon la revendication 10, **caractérisé en ce que** le composé représenté par la [Formule 4] est l'un quelconque ou plusieurs choisis dans le groupe consistant en les composés représentés par les formules suivantes: 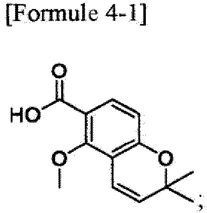 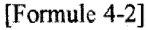 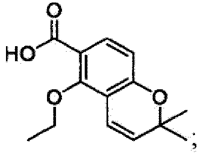 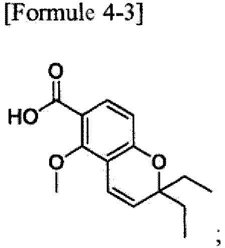 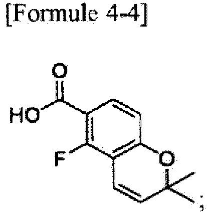 et

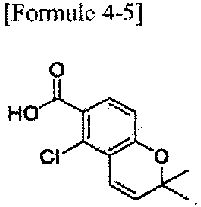

14. Procédé selon la revendication 10, **caractérisé en ce que** le composé représenté par la [Formule 5] est l'un quelconque ou plusieurs choisis dans le groupe consistant en les composés représentés par les formules suivantes: 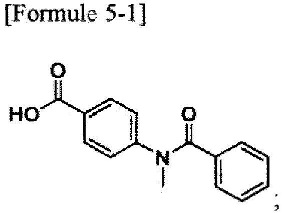 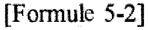 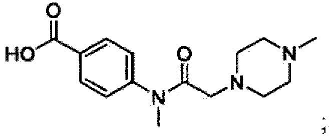 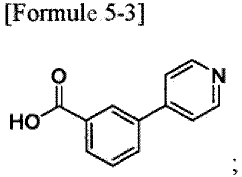 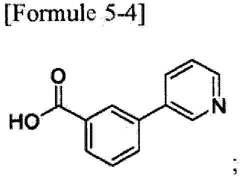 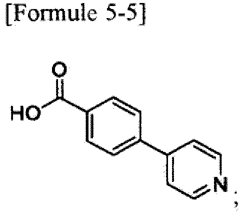 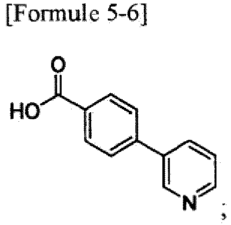 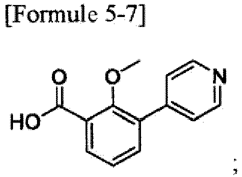 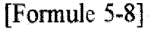 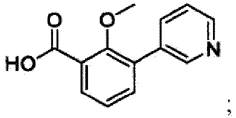 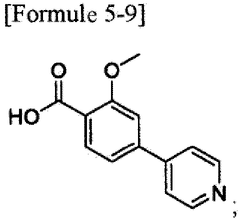 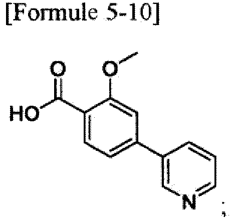 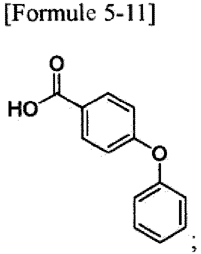 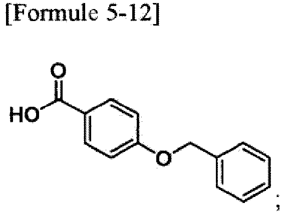 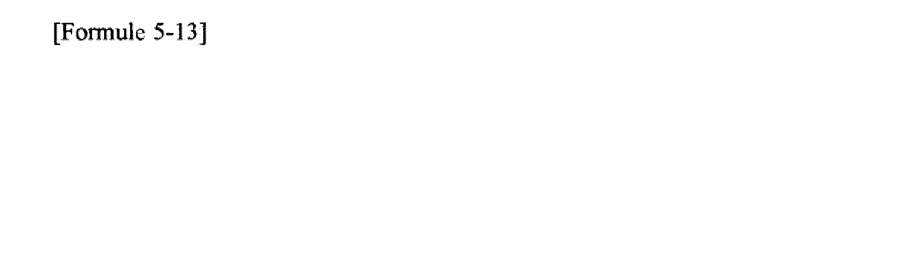 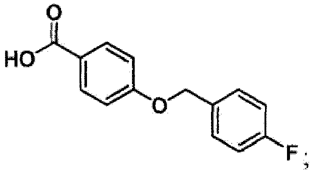 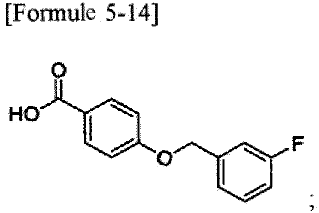 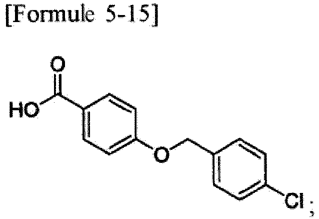 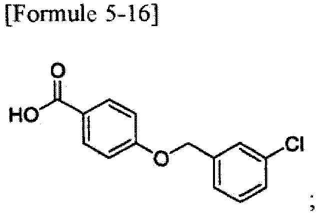 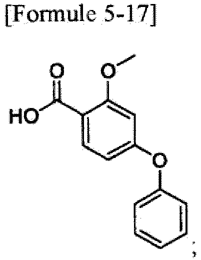 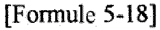 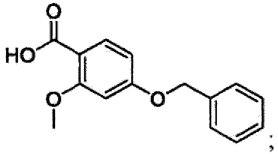 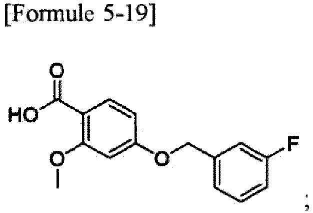 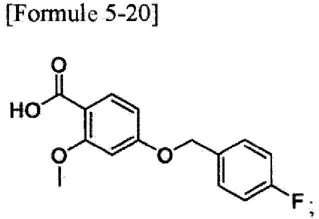 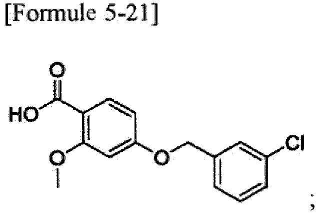 et

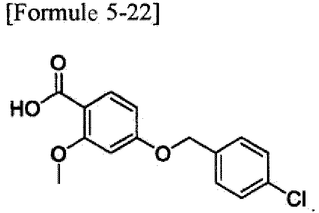

15. Procédé selon la revendication 10, **caractérisé en ce que** le composé représenté par la [Formule 2] ou la [Formule 3] est mis à réagir par addition du composé représenté par la [Formule 2] ou la [Formule 3] à un mélange du composé représenté par la [Formule 4] ou la [Formule 5], d'hydroxybenzotriazole (HOBt), de triéthylamine (Et₃N) et de EDC·HCl.
